(19) Europäisches Patentamt
European Patent Office
Office européen des brevets

(11) Publication number: **0 670 315 A1**

(12) **EUROPEAN PATENT APPLICATION**

(21) Application number: **95101379.6**

(51) Int. Cl.[6]: **C07D 249/08**, A01N 43/653

(22) Date of filing: **01.02.95**

(30) Priority: **04.02.94 JP 33192/94**

(43) Date of publication of application: **06.09.95 Bulletin 95/36**

(84) Designated Contracting States: **AT BE CH DE DK ES FR GB GR IE IT LI LU MC NL PT SE**

(71) Applicant: **MOCHIDA PHARMACEUTICAL CO., LTD.**
**7, Yotsuya 1-chome**
**Shinjuku-ku**
**Tokyo 160 (JP)**

(72) Inventor: **Mochizuki, Hidenori, c/o MOCHIDA Pharmaceutical Co., Ltd,**
**7, Yotsuya 1-chome,**
**Shinjuku-ku**
**Tokyo (JP)**
Inventor: **Tomiguchi, Akira, c/o MOCHIDA Pharmaceutical Co., Ltd,**
**7, Yotsuya 1-chome,**
**Shinjuku-ku**
**Tokyo (JP)**
Inventor: **Takiguchi, Kazuhiko, c/o MOCHIDA Pharmaceutical Co., Ltd,**
**7, Yotsuya 1-chome,**
**Shinjuku-ku**
**Tokyo (JP)**

(74) Representative: **Patentanwälte Grünecker, Kinkeldey, Stockmair & Partner**
**Maximilianstrasse 58**
**D-80538 München (DE)**

(54) **Triazole oxime derivatives having antimycotic acitivity.**

(57) Triazole oxime derivatives represented by the formula (I):

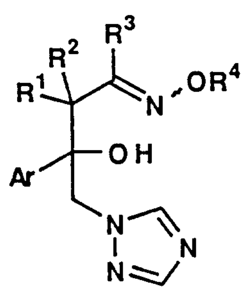

(I)

(where Ar is a phenyl group substituted by 1 or 2 halogen atoms; $R^1$ and $R^2$ are typically such that, when taken together with the adjacent carbon atom, they form a cyclopropylidene group; $R^3$ is a straight-chained, branched or cyclic alkyl group having 1 - 4 carbon atoms; $R^4$ is typically an optionally substituted straight-chained or branched alkyl group having 1 - 4 carbon atoms; and the wavy line represents either an E- or Z-type bond) or salts thereof. The triazole derivatives exhibit a marked therapeutic effects not only in in vitro experiments but also in in vivo experiments using laboratory animal models such as Aspergillus infected mice. The derivatives are also safe to use. Therefore, they are extremely useful as therapeutics for various superficial der-

EP 0 670 315 A1

matomycoses, deep dermatomycoses and deep mycoses (mycoses in internal organs).

FIG. 1

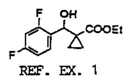
REF. EX. 1

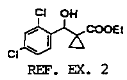
REF. EX. 2

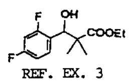
REF. EX. 3

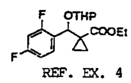
REF. EX. 4

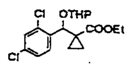
REF. EX. 5

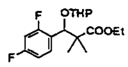
REF. EX. 6

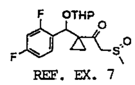
REF. EX. 7

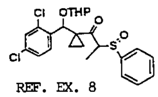
REF. EX. 8

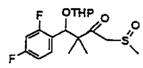
REF. EX. 9

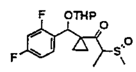
REF. EX. 10

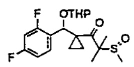
REF. EX. 11

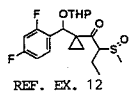
REF. EX. 12

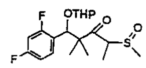
REF. EX. 13

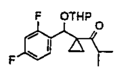
REF. EX. 14

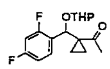
REF. EX. 15

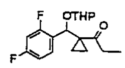
REF. EX. 16

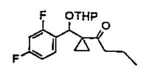
REF. EX. 17

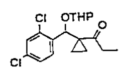
REF. EX. 18

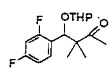
REF. EX. 19

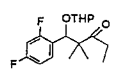
REF. EX. 20

## BACKGROUND OF THE INVENTION

### Field of the Invention

This invention relates to novel triazole derivatives or salts thereof, processes for the production of said derivatives, antimycotics containing said derivatives as active ingredients, and compounds useful as intermediates for the synthesis of said derivatives.

### Prior Art

Mycoses are diseases caused by infections with molds and yeasts and their incidence is growing from year to year. Particularly with respect to deep mycoses, the infection is prone to occur in the state of immunodeficiency, so the number of patients with mycoses is ever increasing as patients with terminal cancers and AIDS are growing in number.

The state of immunodeficiency is also induced by immunosuppressants used in organ transplantation and, hence, mycoses are also becoming a problem in surgery.

Compounds presently available on the market as therapeutics of various deep mycoses include amphotericin B, flucytosine and azol antimycotics such as miconazole, fluconazole and itraconazole and these drugs have been reported to be effective in the therapy of mycoses.

However, these existing mycosis therapeutics have the following various problems in clinical applications. For example, amphotericin B is not absorbed when administered orally and yet it presents considerable toxicity. Flucytosine is not highly toxic but its antimycotic activity is weak and high-dose administration is necessary to attain satisfactory effects. Another problem is the high incidence of drug-resistant fungi.

The azol antimycotics presently available on the market also have various problems. For example, none of them are completely satisfactory as medicines from the viewpoints of pharmacokinetics, toxicity and solubility as well as the spectrum and strength of antimycotic activity. In particular, the effects against Aspergillus are quite unsatisfactory.

In addition, several cases of attenuated antimycotic activity have been reported in connection with immunodeficiency which is a serious problem common to terminal cancers, AIDS, organ transplantation, etc. Itraconazole recently put on the market has been reported to be effective against Aspergillus in clinical tests in Japan; on the other hand, it has been reported that itraconazole is ineffective against candidosis in immunocompromised patients and aspergillosis in bone-marrow transplanted patients.

Under the circumstances, the unavailability of antimycotics that are effective against many fungi including Aspergillus or which exhibit satisfactory effectiveness in the treatment of fungal infections in immunocompromised patients has become a serious social problem. The existing therapeutics of mycoses are not yet to be completely satisfactory as medicines from the viewpoints of dosage form, the efficiency of absorption, etc.

With a view to solving these problems, various derivatives of azol antimycotics have been synthesized. Included in the efforts made from this approach are the recent studies on triazol antimycotics having an aldoxime group (an oxime group derived from aldehyde), among which the following are known.

Unexamined Published Japanese Patent Application (kokai) Sho 61-91175 teaches that triazole derivatives having an aldoxime group are effective in the treatment of Candida infected mice. However, it has no teaching that these triazole derivatives are effective in the treatment of diseases caused by infections with Aspergillus and mycoses (candidosis) with immunocompromised states.

Unexamined Published Japanese Patent Application (kokai) Hei 2-9864 also teaches that triazole derivatives having an aldoxime group are effective in the treatment of Candida infected mice. However, it has no teaching that those triazole derivatives are effective in the treatment of diseases caused by infections with Aspergillus and mycoses (candidosis) with immunocompromised states.

Unexamined Published Japanese Patent Application (kokai) Hei 1-50869 also teaches aldoxime group containing triazole derivatives that have an antimycotic activity. However, this patent teaches only the antimycotic activity against fungi that infect plants such as wheat and rice and it has no teaching about the antimycotic activity against fungi that infect animals.

Unexamined Published Japanese Patent Application (kokai) Hei 1-151564 also teaches triazole derivatives that have an aldoxime group and which are useful as starting materials for the synthesis of nitrile derivatives having antimycotic activity. However, it teaches nothing about the antimycotic activity of such triazole derivatives per se.

The compound prepared in Example 37 of the invention disclosed in Unexamined Published Japanese Patent Application (kokai) Sho 61-91175, supra, is the most advanced in the development of medicines in the relevant prior art and has been reported as Bay R 3783 [(±)-3-(4-chlorophenyl)-2,2-dimethyl-3-hydroxy-4-(1,2,4-triazole-1-yl)butanol-oxime O-methylether] which is represented by the following formula (P):

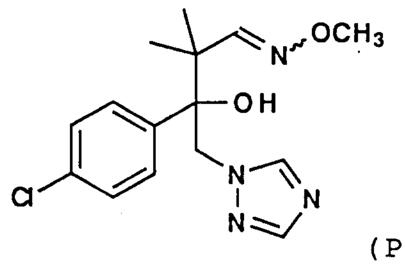

(P)

This compound has been reported to have activity against Aspergillus in Antimicrobial Agents & Chemotherapy, vol. 34, p. 448, 1990. Bay R 3783 is comparable to itraconazole in effectiveness against Aspergillus but there is no teaching of its effectiveness against mycoses (candidosis) with immunocompromised states with Candida. It has also been reported that Bay R 3783 has hepatotoxicity as a side effect and that this has forced researchers to give up their development efforts. The side effect of Bay R 3783 is believed to originate from the nitrile derivative which is a metabolites of aldoxime.

All prior art compounds under consideration including Bay R 3783 are aldoxime derivatives and suspected of exhibiting hepatotoxicity as a side effect since they are anticipated to produce similar metabolites to Bay R 3783.

There is no prior art on analogous compounds having ketoxime group (an oxime group derived from ketone) in its molecular structure.

Under the circumstances, there is a strong need for antimycotics that have a broad spectrum of antimycotic activity to exhibit effectiveness in the treatment of not only infections with Aspergillus but also mycoses (candidosis) with immunocompromised states and which yet are safe to use. However, as of today, no compounds have been available that satisfy these requirements.

## SUMMARY OF THE INVENTION

An object, therefore, of the present invention is to provide novel antimycotics that solve at least one of the problems with the prior art by meeting at least one of the following requirements; a sufficiently broad spectrum of antimycotic activity to exhibit high effectiveness not only against aspergillosis and other diseases that have not been fully cured by conventional drugs but also in the treatment of mycoses occured in patients with terminal cancers or AIDS and organ transplanted patients who have become immunocompromised as a result of administration of immunosuppressants; good enough solubility to permit use as an injection; and extremely high safety.

With a view to attaining this object, the present inventors have conducted intensive studies and found that specified triazole derivatives had at least one of the features set forth above including excellent antimycotic activity, particularly against Aspergillus and in the treatment of mycoses (candidosis) with immunocompromised states. The present invention has been accomplished on the basis of this finding.

## BRIEF DESCRIPTION OF THE DRAWINGS

Fig. 1 shows the structures of the compounds synthesized in Reference Examples 1 - 20;
Fig. 2 shows the structures of the compounds synthesized in Reference Examples 21 - 31;
Fig. 3 shows the structures of the compounds synthesized in Reference Examples 32 - 46;
Fig. 4 shows the structures of the compounds synthesized in Reference Examples 47 - 61;
Fig. 5 shows the structures of the compounds synthesized in Reference Examples 62 - 79;
Fig. 6 shows the structures of the compounds synthesized in Reference Examples 80 - 94;
Fig. 7 shows the structures of the compounds synthesized in Reference Examples 95 - 101;
Fig. 8 shows the structures of the compounds synthesized in Examples 1 - 12;
Fig. 9 shows the structures of the compounds synthesized in Examples 13 - 24;
Fig. 10 shows the structures of the compounds synthesized in Examples 25 - 34;

Fig. 11 shows the structures of the compounds synthesized in Examples 35 - 44;
Fig. 12 shows the structures of the compounds synthesized in Examples 45 - 56;
Fig. 13 shows the structures of the compounds synthesized in Examples 57 - 66; and
Fig. 14 shows the structures of the compounds synthesized in Examples 67 - 76.

DETAILED DESCRIPTION OF THE INVENTION

According to the first aspect of the present invention, there are provided novel triazole derivatives represented by the following formula (I):

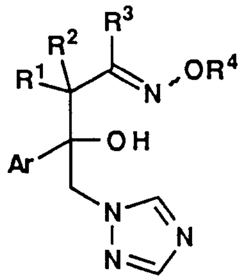
(I)

(where Ar is a phenyl group substituted by one or two halogen atoms; $R^1$ and $R^2$ are each a methyl group or, when taken together with the adjacent carbon atom, represent a cyclopropylidene group; $R^3$ is a straight-chained, branched or cyclic alkyl group having 1 - 4 carbon atoms; $R^4$ is a hydrogen atom, a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group; and the wavy line represents an E- or Z-type bond) or salts thereof.

In the compounds of formula (I), $R^3$ is preferably an n-propyl or an isopropyl group.

Preferred combinations of substituents in the compounds of formula (I) include but are by no means limited to the following. Preferred embodiments of each substituent will be discussed in separate entries.

In the combination of Ar, $R^1$, $R^2$ and $R^3$, Ar is preferably a 2,4-difluorophenyl group and $R^1$ and $R^2$ preferably form a cyclopropylidene group as taken together with the adjacent carbon atom and $R^3$ is preferably an n-propyl or an isopropyl group.

This combination preferably includes the case where $R^4$ is a methyl group, a methoxymethyl group or a 1H-(1,2,4-triazol-1-yl) methyl group.

The bond represented by the wavy line is preferably of the E-type bond.

According to the second aspect of the present invention, there are provided three processes A - C for producing the derivatives (I).

(Process A)

A process for producing compounds of the formula (Ia):

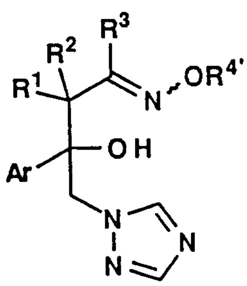
(Ia)

(where Ar, $R^1$, $R^2$, $R^3$ and the wavy line have the same meanings as defined in the formula (I), and $R^{4'}$ is a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group) or salts thereof by reacting oxime derivatives of the formula (II):

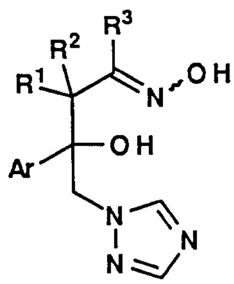

(II)

[where Ar, $R^1$, $R^2$, $R^3$ and the wavy line have the same meanings as defined in the formula (I)] with compounds of the following formula (III):

$R^{4'}$ - X (III)

(where $R^{4'}$ has the same meaning as defined in the formula (Ia); and X is a leaving group) in the presence of a base.

(Process B)

A process for producing compounds of the formula (I) or salts thereof by reacting oxirane derivatives of the formula (IV):

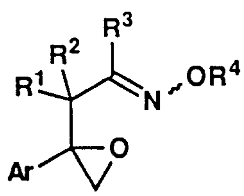

(IV)

[where Ar, $R^1$, $R^2$, $R^3$, $R^4$ and the wavy line have the same meanings as defined in the formula (I)] with triazole derivatives of the formula (V):

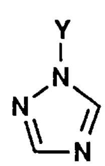

(V)

(where Y is a hydrogen atom or a trialkylsilyl group) in the presence of a base and optionally hydrolyzing the reaction product.

(Process C)

A process for producing compounds of the formula (I) or salts thereof by reacting ketone derivatives of the formula (VI):

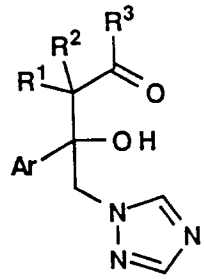

(VI)

[where Ar, $R^1$, $R^2$ and $R^3$ have the same meanings as defined in the formula (I)] with hydroxylamine derivatives of the formula (VII):

$R^4$ - $ONH_2$ (VII)

[where $R^4$ has the same meaning as defined in the formula (I)].

According to the third aspect of the present invention, there are provided novel antimycotics that contain at least one of the compounds of the formula (I) or salts thereof as an active ingredient.

In the combination of Ar, $R^1$, $R^2$ and $R^3$, Ar is preferably a 2,4-difluorophenyl group and $R^1$ and $R^2$ preferably form a cyclopropylidene group as taken together with the adjacent carbon atom and $R^3$ is preferably an n-propyl or isopropyl group.

This combination preferably includes the case where $R^4$ is a methyl group, a methoxymethyl group or a 1H-(1,2,4-triazol-1-yl)-methyl group.

The present invention is characterized in that Ar is a 2,4-di-fluorophenyl group. The invention is also characterized in that $R^4$ is a methyl group, a methoxymethyl group or a 1H-(1,2,4-triazol-1-yl)-methyl group.

According to the fourth aspect of the present invention, there are provided the following compounds or salts thereof which are useful in the synthesis of the compounds of the formula (I) or salts thereof:

i) compounds of the formula (IV):

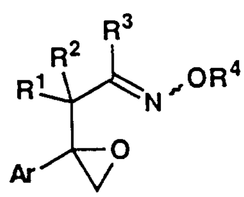

(IV)

[where Ar, $R^1$, $R^2$, $R^3$, $R^4$ and the wavy line have the same meanings as defined in the formula (I)] or salts thereof; and

ii) compounds of the formula (VIII):

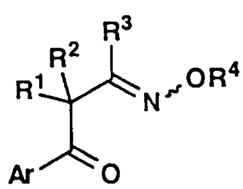

(VIII)

[where Ar, $R^1$, $R^2$, $R^3$, $R^4$ and the wavy line have the same meanings as defined in the formula (I)] or salts thereof.

In the formula (I) and others which have the wavy line, the N-O bond in the alkoxyimino or hydroxyimino group can occur either as a Z isomer (syn-isomer) or an E isomer (anti-isomer) or a mixture thereof and it should be understood that the invention shall include both types of isomers and the mixture thereof in the scope of claims.

The carbon in the tertiary alcohol in the formula (I) is asymmetric and, hence, the compounds of the formula (I) can occur as either an optical isomer having the steric configuration (R), or an optical isomer having the configuration (S), or racemic forms or mixtures thereof. Asymmetric carbon atoms can occasionally occur in substituents $R^3$ and $R^4$ and in each of these cases, optical isomers, racemic forms or mixtures thereof will exist.

Consider, for example, the case where an asymmetric carbon atom exists in $R^3$ or $R^4$ in the formula (I) to give prural asymmetric carbon atom in the molecule. In a case like this, both enantiomers and diastereomers occur and they may or may not have optical activity. The invention shall include all of these isomers and mixtures thereof in the scope of claims.

Asymmetric carbon atoms also exist in other compounds of the invention, such as the ones represented by the formulas (Ia) and (II), as well as compounds such as the ones represented by the formulas (IV) and (VI) which are useful in the synthesis of the compounds of the formula (I) and this gives rise to optical isomers having the steric configuration (R), optical isomers having the configuration (S), racemic forms, as well as mixtures thereof. The invention shall include either of these isomers and mixtures thereof in the scope of claims.

The specific rotation of the compounds of the invention may assume either the minus or plus sign or, alternatively, intramolecular or intermolecular cancellation may yield zero specific rotation.

Specific embodiments of the substituents in the compounds of the invention will now be described in detail.

Substituent Ar is a phenyl group substituted by 1 or 2 halogen atoms. If Ar is a phenyl group substituted by one halogen atom, it is preferably a 4-chlorophenyl group. If Ar is a phenyl group substituted by two halogen atoms, each halogen atom is preferably chlorine or fluorine, with fluorine being more preferred. Substitution by halogen atoms is preferably in 2- or 4-position. Preferred specific examples of Ar include but are not limited to a 2,4-dichlorophenyl group, a 2,4-difluorophenyl group, a 2-chloro-4-fluorophenyl group and a 4-chloro-2-fluorophenyl group. In a particularly preferred embodiment, Ar is a 2,4-difluorophenyl group.

Substituents $R^1$ and $R^2$ are each a methyl group or, when taken together with the adjacent carbon atom, represent a cyclopropylidene group.

Substituent $R^3$ is a straight-chained, branched or cyclic alkyl group having 1 - 4 carbon atoms, as exemplified by a methyl group, an ethyl group, an n-propyl group, an isopropyl group, a cyclopropyl group, an n-butyl group, an isobutyl group, a sec-butyl group, a tert-butyl group, a methyl-substituted cyclopropyl group, a cyclopropylmethyl group and a cyclobutyl group. Preferred examples include a methyl group, an ethyl group, an n-propyl group, an isopropyl group, a cyclopropyl group, an isobutyl group and a tert-butyl group, with an n-propyl group and an isopropyl group being more preferred.

Substituent $R^4$ is a hydrogen atom, a dimethoxymethyl group, a dimethoxyethyl group or a straight-chained or branched alkyl group having 1 - 4 carbon atoms which may have at least one substituent. Exemplary alkyl groups as $R^4$ include a methyl group, an ethyl group, an n-propyl group, an isopropyl group, an n-butyl group, an isobutyl group, a sec-butyl group, a tert-butyl group, etc. Preferred examples include a methyl group, an ethyl group, an n-propyl group, an isopropyl group and an n-butyl group, with a methyl group being particularly preferred.

The substituent on the alkyl group represented by $R^4$ is selected from the group consisting of a 5- or 6-membered monocyclic hetero group having 1, 2 or 3 nitrogen atoms that may be substituted by 1 or 2 methyl group, a methylthio group, a methoxy group, an ethoxy group, a methoxyethoxy group, a cyano group, a halogen atom, an ethynyl group and an epoxy group. The monocyclic hetero group is preferably a 5-membered monocyclic hetero group having 2 or 3 nitrogen atoms or a 6-membered monocyclic hetero group having 1 or 2 nitrogen atoms. Examples of the former case include an imidazolyl group and a triazolyl group, with a triazolyl group being particularly preferred. Examples of the latter case include a pyridyl group and a piperazinyl group.

Specifically, $R^4$ is preferably selected from among a hydrogen atom, a dimethoxymethyl group, a dimethoxyethyl group, a methyl group, an ethyl group, an n-propyl group, an n-butyl group, a triazolylmethyl group, a methyl-substituted triazolylmethyl group, a triazolylethyl group, a methyl-substituted triazolylethyl group, a pyridylmethyl group, a methylthiomethyl group, a methoxymethyl group, a methoxypropyl group, an ethoxymethyl group, a methoxyethoxymethyl group, a cyanomethyl group, a bromoethyl group and a propynyl group, with a methyl group, a methoxymethyl group and a triazolylmethyl group being particularly preferred. As for the triazolylmethyl group, a 1H-(1,2,4-triazol-1-yl)methyl group being more preferred.

Speaking of the combination of substituents $R^3$ and $R^4$, an n-propyl or isopropyl group as $R^3$ is desirably combined with a methyl, methoxymethyl or triazolylmethyl group as $R^4$. Substituent X represents

a leaving group such as a halogen atom, a tosyloxy group, a mesyloxy group, etc. Substituent Y represents a hydrogen atom or a trialkylsilyl group.

The wavy line represents preferably an E-type bond.

The preferred embodiments of substituents which are set forth above are applied with particular advantage to the compounds of the formulas (I), (IV) and (VIII).

As for compounds of the formula (I) where $R^4$ is a methyl or methoxymethyl group, optical isomers that exhibit a (-) specific rotatory power when measured in a methanol solution at 27°C with the sodium D rays are particularly preferred.

The most preferred combinations of the substituents are shown below. When Ar is a 4-chlorophenyl group, $R^1$ and $R^2$ preferably form a cyclopropylidene group as taken together with the adjacent carbon atom, and $R^3$ is preferably an n-propyl or isopropyl group. As for $R^4$, a methyl group, a methoxymethyl group or a 1H-(1,2,4-triazol-1-yl)methyl group is preferred. The wavy line represents preferably an E-type bond.

Preferred examples of the compound of the formula (I) where Ar is a 4-chlorophenyl group include specifically the following:

(±)-(E)-1-[1-[1-(4-chlorophenyl)-1-hydroxy-2-(1H-1,2,4-triazol-1-yl)ethyl]cyclopropyl]-1-butanone O-methyloxime;

(±)-(E)-1-[1-[1-(4-chlorophenyl)-1-hydroxy-2-(1H-1,2,4-triazol-1-yl)ethyl]cyclopropyl]-1-butanone O-(methoxymethyl)oxime;

(±)-(E)-1-[1-[1-(4-chlorophenyl)-1-hydroxy-2-(1H-1,2,4-triazol-1-yl)ethyl]cyclopropyl]-2-methyl-1-propanone O-methyloxime; and

(±)-(E)-1-[1-[1-(4-chlorophenyl)-1-hydroxy-2-(1H-1,2,4-triazol-1-yl)ethyl]cyclopropyl]-2-methyl-1-propanone O-(methoxymethyl)oxime.

When Ar is a 2,4-difluorophenyl group, $R^1$ and $R^2$ preferably form a cyclopropylidene group as taken together with the adjacent carbon atom, and $R^3$ is preferably an n-propyl or isopropyl group. As for $R^4$, a methyl group, a methoxymethyl group or a 1H-(1,2,4-triazol-1-yl)methyl group is preferred. The wavy line represents preferably an E-type bond.

Preferred examples of the compound of the formula (I) where Ar is a 2,4-difluorophenyl group include specifically the following:

(±)-(E)-1-[1-[1-(2,4-difluorophenyl)-1-hydroxy-2-(1H-1,2,4-triazol-1-yl)-ethyl]cyclopropyl]-1-butanone O-methyloxime;

(±)-(E)-1-[1-[1-(2,4-difluorophenyl)-1-hydroxy-2-(1H-1,2,4-triazol-1-yl)-ethyl]cyclopropyl]-1-butanone O-(methoxymethyl)oxime;

(±)-(E)-1-[1-[1-(2,4-difluorophenyl)-1-hydroxy-2-(1H-1,2,4-triazol-1-yl)-ethyl]cyclopropyl]-1-butanone O-(1H-1,2,4-triazol-1-yl)-methyloxime;

(±)-(E)-1-[1-[1-(2,4-difluorophenyl)-1-hydroxy-2-(1H-1,2,4-triazol-1-yl)-ethyl]cyclopropyl]-2-methyl-1-propanone O-methyloxime;

(-)-(E)-1-[1-[1-(2,4-difluorophenyl)-1-hydroxy-2-(1H-1,2,4-triazol-1-yl)ethyl]cyclopropyl]-2-methyl-1-propanone O-methyloxime;

(±)-(E)-1-[1-[1-(2,4-difluorophenyl)-1-hydroxy-2-(1H-1,2,4-triazol-1-yl)-ethyl]cyclopropyl]-2-methyl-1-propanone O-(1H-1,2,4-triazol-1-yl)-methyloxime;

(±)-(E)-1-[1-[1-(2,4-difluorophenyl)-1-hydroxy-2-(1H-1,2,4-triazol-1-yl)-ethyl]cyclopropyl]-2-methyl-1-propanone O-(methoxymethyl)oxime; and

(-)-(E)-1-[1-[1-(2,4-difluorophenyl)-1-hydroxy-2-(1H-1,2,4-triazol-1-yl)ethyl]cyclopropyl]-2-methyl-1-propanone O-(methoxymethyl)oxime.

Particularly preferred as the following two compounds:

(-)-(E)-1-[1-[1-(2,4-difluorophenyl)-1-hydroxy-2-(1H-1,2,4-triazol-1-yl)ethyl]cyclopropyl]-2-methyl-1-propanone O-methyloxime; and

(-)-(E)-1-[1-[1-(2,4-difluorophenyl)-1-hydroxy-2-(1H-1,2,4-triazol-1-yl)ethyl]cyclopropyl)-2-methyl-1-propanone O-methoxymethyloxime.

The triazole derivatives of the invention which are represented by the formula (I) can be manufactured by the routes according to the following reaction schemes 1 - 7. While details of the production processes are given later in this specification, it should be understood that the invention is by no means limited to the specific processes described herein and that they may be modified as appropriate.

It should also be noted that the invention includes optical isomers and mixtures in the scope of claims and that the optical isomers of the compounds of formula (I) can be obtained by any separation and isolation techniques that are commonly employed in the art. For example, the desired isomers can be obtained selectively by employing optical resolution procedures through column chromatography, asymmetric synthesis procedures, etc. with reference being made to standard textbooks such as "Fuseigosei to kogakubunkatsu no shimpo (Advances in Asymmetric Synthesis and Optical Resolution)", ed. by Ohtsuka and Mukaiyama, 1982, Extra Issue No. 97 of Kagaku (Chemistry), published by Kagaku Dojin Shuppan, and "Kosentakuteki hanno (High-Selectivity Reactions)", ed. by Nozaki, Mukaiyama and Noyori, 1981, Extra Issue No. 91 of Kagaku (Chemistry), published by Kagaku Dojin Shuppan. Measuring the antimycotic activity of the optical isomers produced is a common practice in the art and this may be accomplished by any techniques commonly employed in the art.

Reaction scheme 1

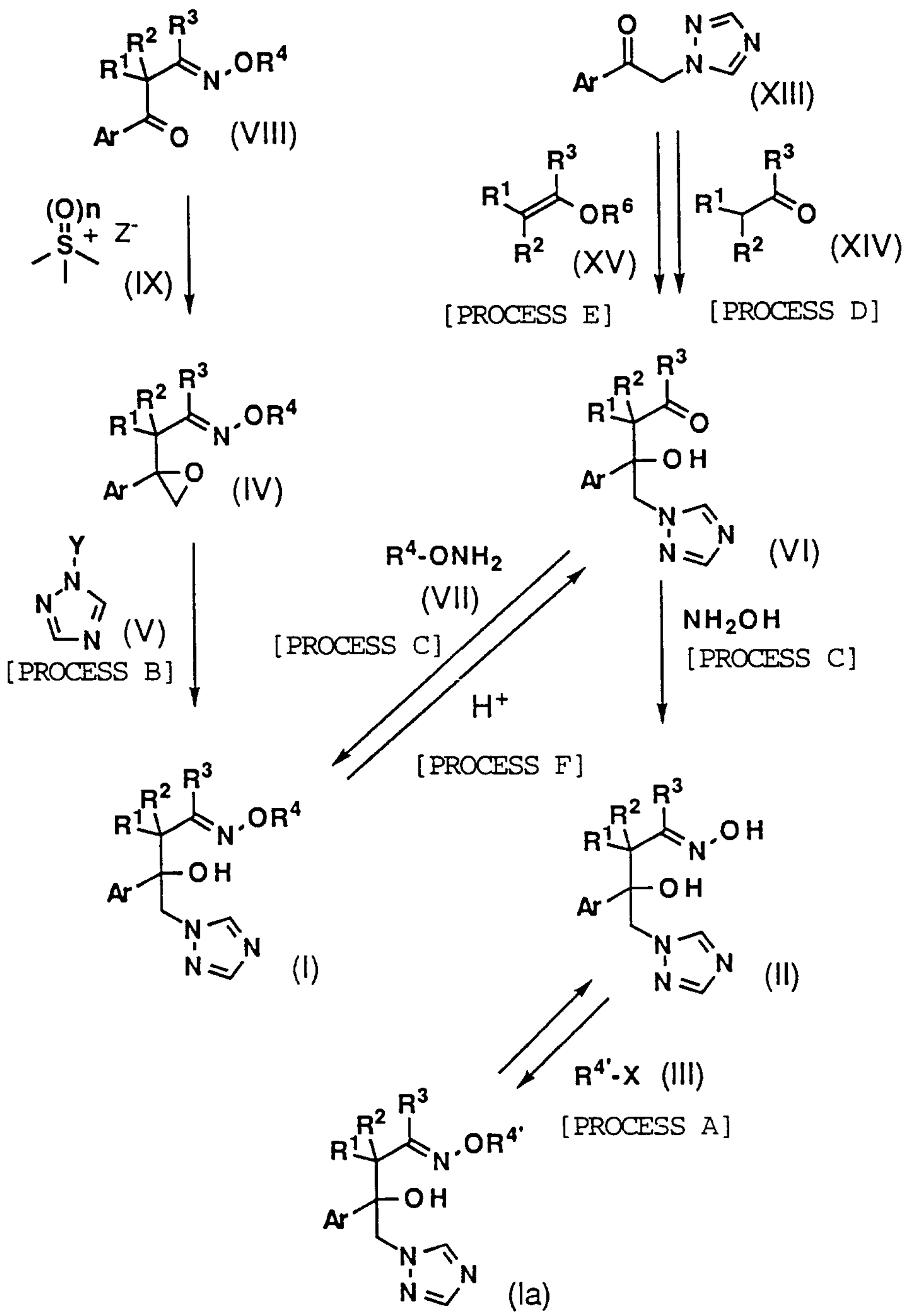

Reaction scheme 2

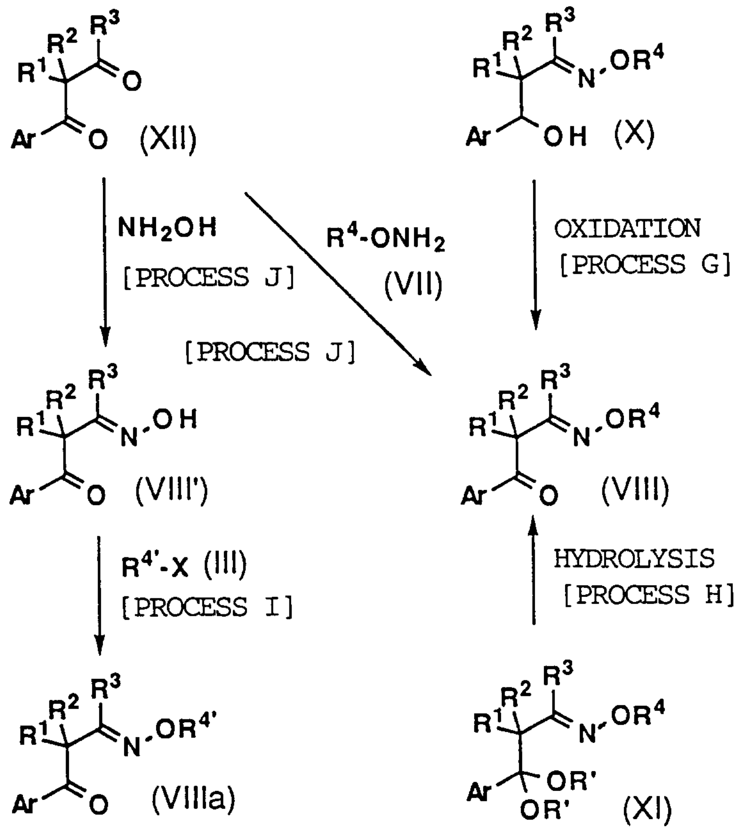

Reaction scheme 3

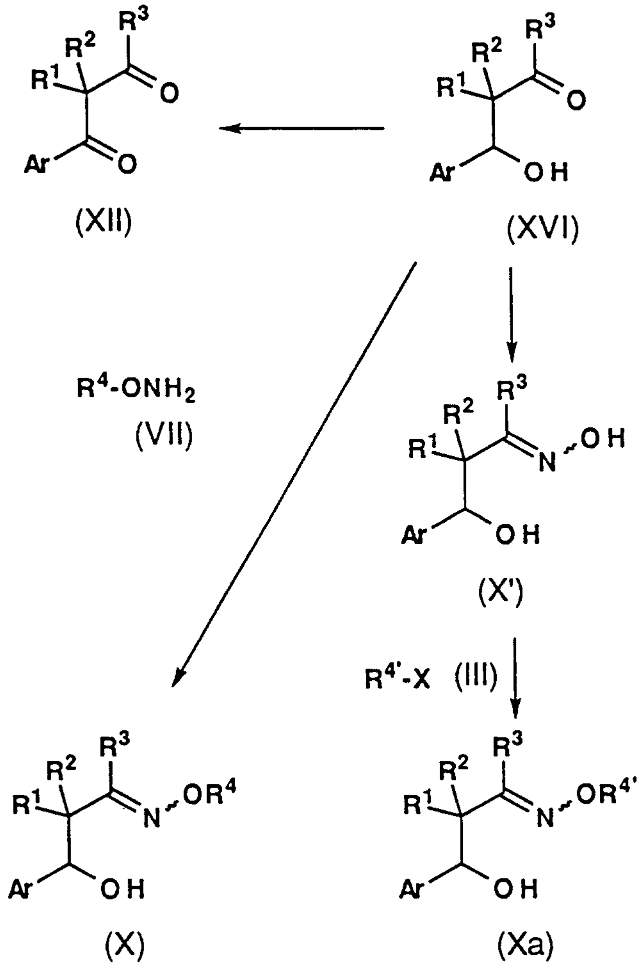


(XII)

(XVI)

(X')

(X)

(Xa)

Reaction scheme 4

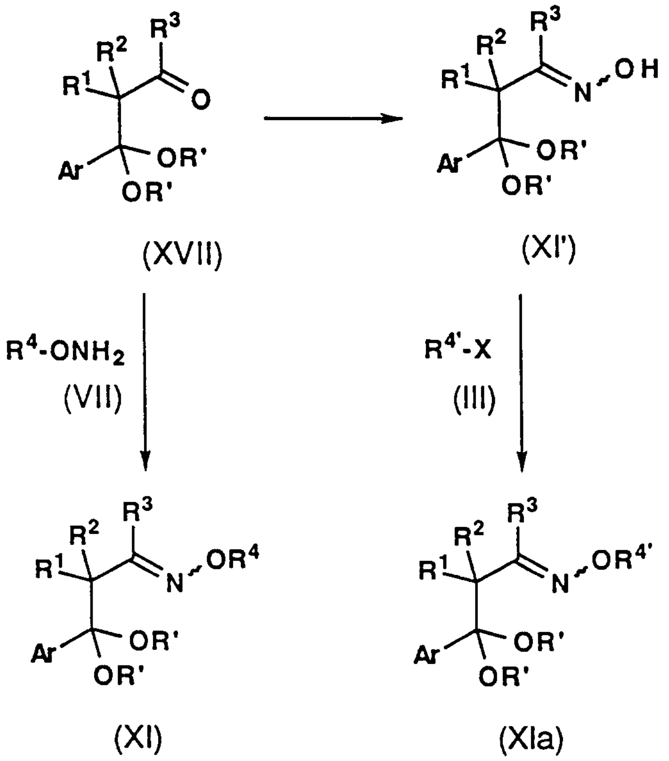

Reaction scheme 5

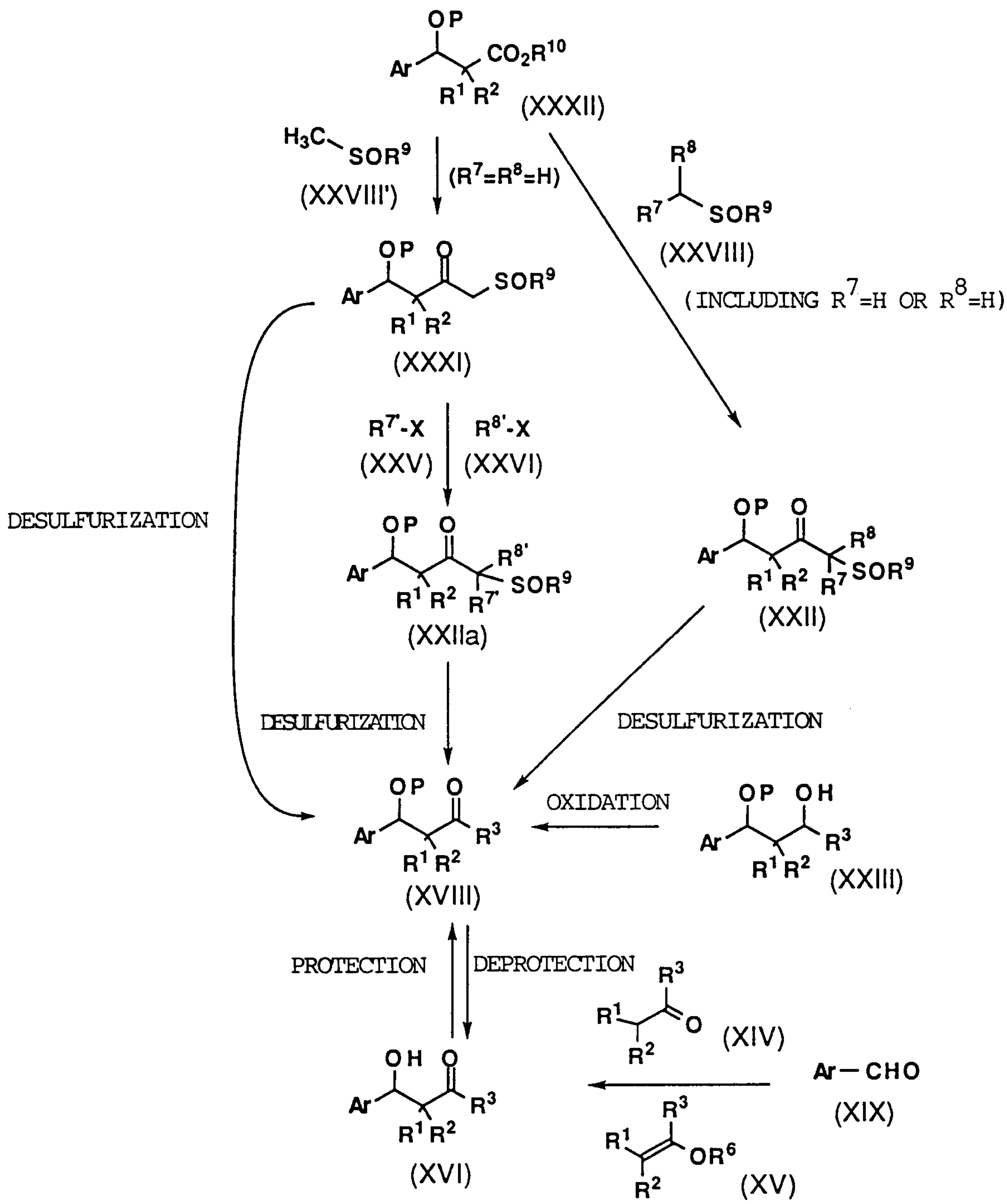

Reaction scheme 6

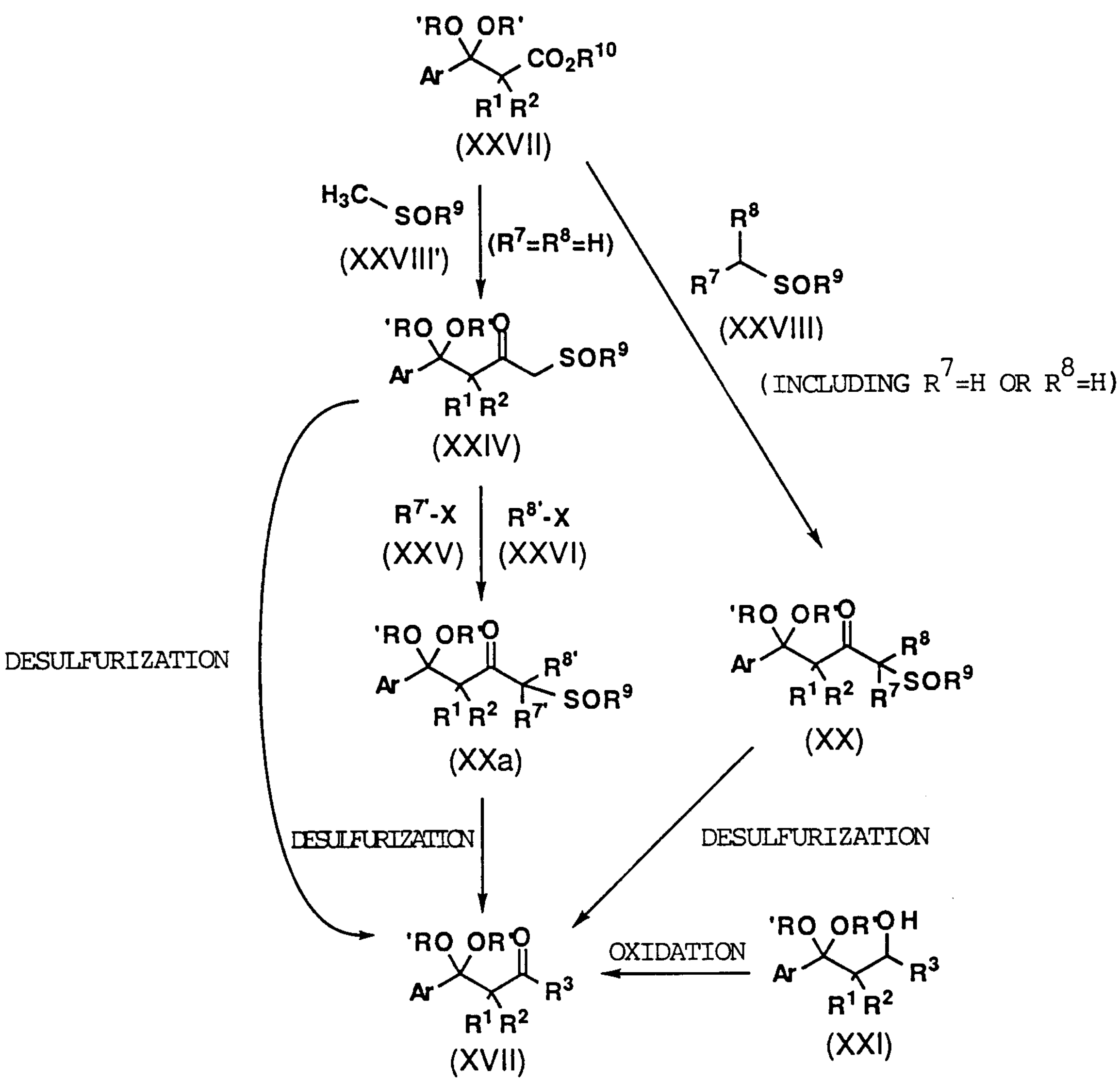

Reaction scheme 7

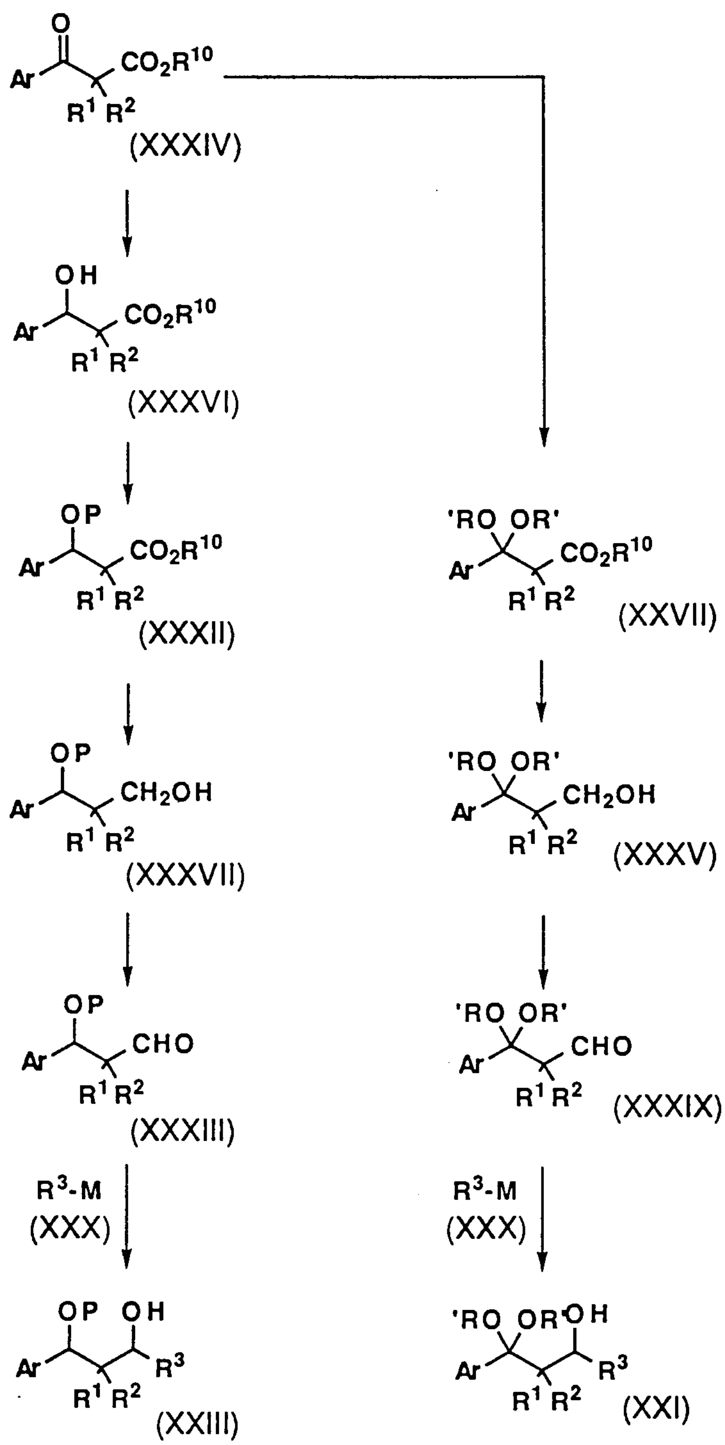


(XXXIV) (XXXVI) (XXXII) (XXXVII) (XXXIII) (XXIII) (XXVII) (XXXV) (XXXIX) (XXI)

Unless otherwise noted, the substituents appearing in the reaction schemes 1 - 7 and those used in the formulae set forth below have the following respective meanings:

Ar is a phenyl group substituted by 1 or 2 halogen atoms;

$R^1$ and $R^2$ are each a methyl group or, when taken together with the adjacent carbon atom, represent a cyclopropylidene group;

$R^3$ is a straight-chained, branched or cyclic alkyl group having 1 - 4 carbon atoms;

$R^4$ is a hydrogen atom, a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group;

$R^{4'}$ is a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group and an epoxy group;

$R^6$ is a trialkylsilyl group or a trialkylstanyl group;

$R^7$ and $R^8$ are each a hydrogen atom, or a straight-chained or branched alkyl group having 1 - 3 carbon atoms;

$R^{7'}$ and $R^{8'}$ are each a straight-chained or branched alkyl group having 1 - 3 carbon atoms;

$R^9$ is a methyl group, an ethyl group or a phenyl group;

$R^{10}$ is a methyl group or an ethyl group;

R' is a ketone protective group, such as a methyl or ethyl group that are individually represented by R' or a methylene group that is represented by two R's taken together;

P is a hydroxyl protective group such as a tetrahydropyranyl (THP) group, a methoxymethyl (MOM) group or a methoxyethoxymethyl (MEM) group;

X is a leaving group such as a halogen atom, a tosyloxy group or a mesyloxy group;

Y is a hydrogen atom or a trialkylsilyl group;

Z is a halogen atom; and

the wavy line represents either an E- or Z-type bond.

Specific substituents such as a hydroxyl group and a ketone may be protected and deprotected by existing procedures (with any necessary modifications) such as the ones described in T.W. Greene et al., "Protective Groups in Organic Synthesis", Chapters 2 - 4, 1991, Wiley Company.

The respective reaction processes will now be described.

(Reaction Scheme 1)

[Processes for the preparation of compounds (I)]

(Process A)

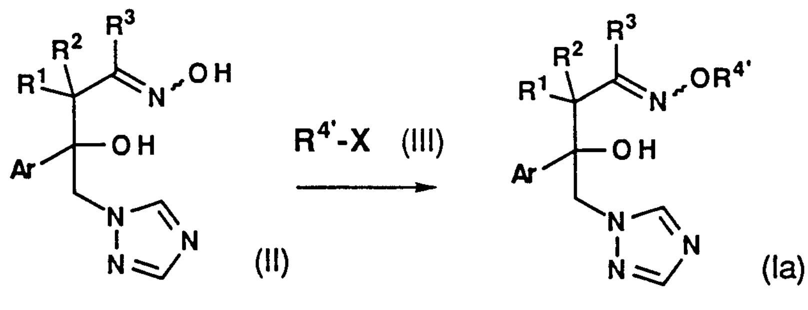


(II) (Ia)

(Process B)

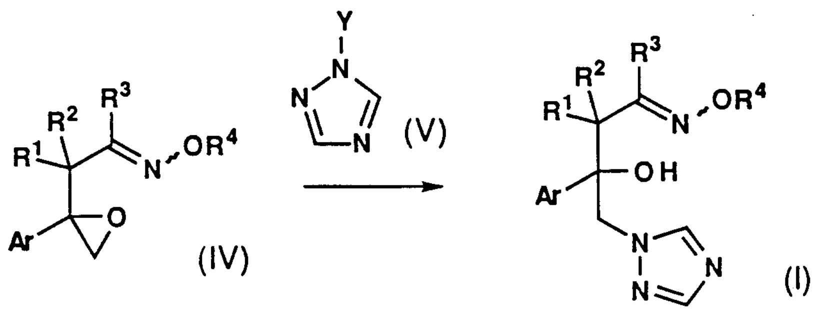


(IV) (I)

(Process C)

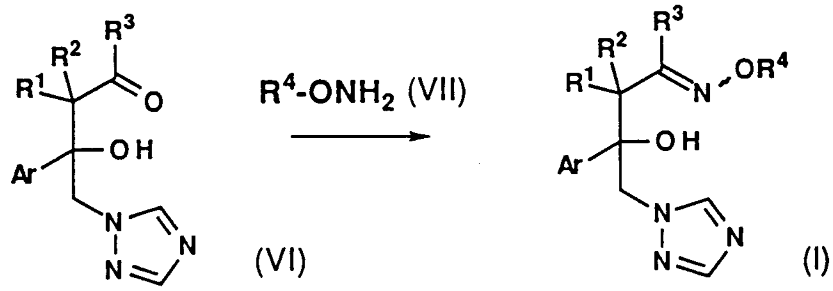


(VI) (I)

Process A

Oxime derivatives of the formula (II):

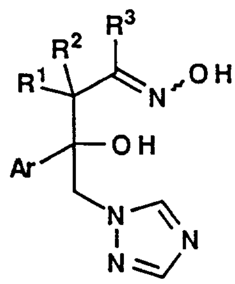

(II)

[where Ar, $R^1$, $R^2$, $R^3$ and the wavy line have the same meanings as defined in the formula (I)] that are embraced by the formula (I) and that are prepared by processes to be described hereinafter are reacted with compounds of the formula (III):

$R^{4'}$ - X (III)

(where $R^{4'}$ has the same meaning as already defined) in an aromatic hydrocarbon solvent, an inert halogen-containing organic solvent (e.g. dichloromethane or 1,2-dichloroethane), an ether solvent (e.g. diethylether, tetrahydrofuran or dioxane), an amide solvent (e.g. dimethylformamide, dimethylacetamide or dimethylimidazolidone) or a sulfoxide solvent (e.g. dimethyl sulfoxide) or mixed solvents thereof in the presence of a base such as an alkali metal amide (e.g. sodium amide or potassium amide), an alkali metal hydride (e.g. sodium hydride or potassium hydride), an alkali metal alcoxide (e.g. potassium t-butoxide or sodium methoxide), a hydroxide (e.g. sodium hydroxide or potassium hydroxide), a carbonate (e.g. potassium carbonate or sodium carbonate) or a quaternary ammonium salt of hydroxide (e.g. tetrabutyl ammonium hydroxide or benzyl trimethyl ammonium hydroxide) at a temperature ranging from -70 °C to the boiling point of the solvent used, preferably at -20 to 130 °C.

When performing a two-phase reaction, such as the one involving a solid powder of sodium hydroxide or potassium hydroxide optionally mixed in an aqueous solution, organic solvents that do not dissolve the alkali metal hydroxide are used, as exemplified by inert solvents including aromatic hydrocarbon solvents, halogen containing organic solvents, ether solvents and amide solvents. In the case of a two-phase reaction involving an aqueous solution, inert solvents such as those aromatic hydrocarbon solvents, halogen containing organic solvents and other solvents which separate from water, or mixtures thereof are used. In these cases, compounds of the formula (II) are reacted with compounds of the formula (III) in the presence of a catalytic or equivalent amount of a phase-transfer catalyst such as tetrabutyl ammonium hydrogensulfate, tetrabutyl ammonium bromide or benzyl trimethyl ammonium chloride, thereby yielding compounds of the formula (Ia):

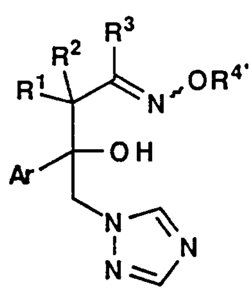

(Ia)

[where Ar, $R^1$, $R^2$, $R^3$ and the wavy line have the same meanings as defined in the formula (I) and $R^{4'}$ has the same meaning as defined in the formula (III)].

Alternatively, oxime derivatives of the formula (II) may be reacted with ortho-ester derivatives or vinyl ether derivatives under acidic conditions to yield compounds of the formula (Ia).

If one wants to produce compounds of the formula (Ia) where $R^{4'}$ contains an alkylene group as a crosslinking group, a dihalogenoalkane may be first reacted with compounds of the formula (II) and the resulting halogenoalkyloxime group may be subjected to a nucleophilic substitution reaction with the remaining group (e.g. a 5- or 6-membered monocyclic hetero group having 1, 2 or 3 nitrogen atoms that may be substituted by 1 or 2 methyl groups).

Process B

β-Ketoxime derivatives of the formula (IV):

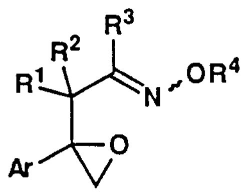

(IV)

[where Ar, $R^1$, $R^2$, $R^3$, $R^4$ and the wavy line have the same meanings as defined in the formula (I)] that are prepared by a process to be described hereinafter are reacted with compounds of the formula (V):

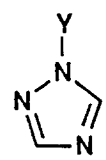

(V)

(where Y is a hydrogen atom or a trialkylsilyl group) in an inert aromatic hydrocarbon solvent, an alcoholic solvent (e.g. ethanol, propanol or methoxyethanol), an ether solvent (e.g. tetrahydrofuran or dioxane), a ketone solvent (e.g. diethyl ketone or ethyl methyl ketone), a nitrile solvent (e.g. acetonitrile or propionitrile), an ester solvent (e.g. ethyl acetate or ethyl propionate), an amide solvent (e.g. dimethylformamide or dimethylacetamide) or mixed solvents thereof in the presence of an amide such as an alkali metal carbonate (e.g. sodium carbonate or potassium carbonate), an alkali hydroxide (e.g. sodium hydroxide or potassium hydroxide), an alkali metal hydride (e.g. sodium hydride or potassium hydride), an alkali metal alcoxide (e.g. potassium t-butoxide or sodium methoxide) or a tertiary amine [e.g. triethylamine or diazabicyclo[5.4.0]undec-7-ene (DBU)] at a temperature ranging from 0 °C to the boiling point of the solvent used, preferably in the range from room temperature to 150 °C, and the remaining trialkylsilyl group may optionally be deprotected by an existing procedure, with any necessary modifications made, thereby yielding compounds of the formula (I).

Process C

Ketone derivatives of the formula (VI):

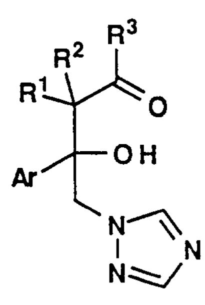

(VI)

[where Ar, $R^1$, $R^2$ and $R^3$ have the same meanings as defined in the formula (I)] are reacted with compounds of the formula (VII):

$R^4$ - $ONH_2$ (VII)

[where $R^4$ has the same meaning as defined in the formula (I)] in an alcoholic solvent (e.g. methanol or ethanol), water, a pyridine solvent (e.g. pyridine or 2,6-lutidine), an amide solvent (e.g. dimethylformamide or dimethylacetamide) or mixed solvents thereof in the presence of an organic base (e.g. triethylamine, pyridine or DBU), an alkali metal alcoxide (e.g. potassium t-butoxide or sodium methoxide), an alkali hydroxide (e.g. sodium hydroxide or potassium hydroxide), an alkali metal carbonate (e.g. sodium carbonate or potassium carbonate) or an alkali metal salt of organic acid (e.g. potassium acetate or sodium acetate) at a temperature ranging from -20 °C to the boiling point of the solvent used, preferably in the range from room temperature to 130 °C, thereby yielding compounds of the formula (I).

Process (C) is adapted for the preparation of compounds of the formula (II) if $R^4$ in the formula (I) is hydrogen. If $R^4$ in the formula (I) is a protective group, it may be deprotected by common procedures for the deprotection of a hydroxyl group, with any necessary modifications made, so as to yield compounds of the formula (II).

[Process for the preparation of compounds (IV)]

Intermediate oxirane derivatives of the formula (IV) are prepared by reacting ketone derivatives of the formula (VIII):

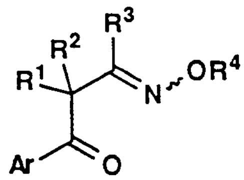

(VIII)

[where Ar, $R^1$, $R^2$, $R^3$, $R^4$ and the wavy line have the same meanings as defined in the formula (I)] with sulfonium or sulfoxonium salts of the formula (IX):

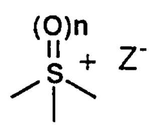

(IX)

(where n is an integer of 0 or 1, and Z is a halogen atom) in accordance with methods known per se, namely, in a solvent such as dimethyl sulfoxide or tetrahydrofuran in the presence of a base such as sodium hydride or sodium ethoxide at a temperature of -20 to 100 °C, preferably at -10 to 60 °C.

It should be mentioned here that the reaction with sulfonium salts can be performed with reference made to Journal of the American Chemical Society, 109, p. 3353 ff., 1987, particularly in accordance with the method of synthesis of compound 11 which is described on page 3355 whereas the reaction with sulfoxonium salts can be performed with reference made to Journal of the American Chemical Society, 109, supra, p. 1269 ff, 1987, particularly in accordance with the method of synthesis of compound 4 which is set forth in Scheme II on page 1271.

[Processes for the preparation of compounds (VI)]

Process D

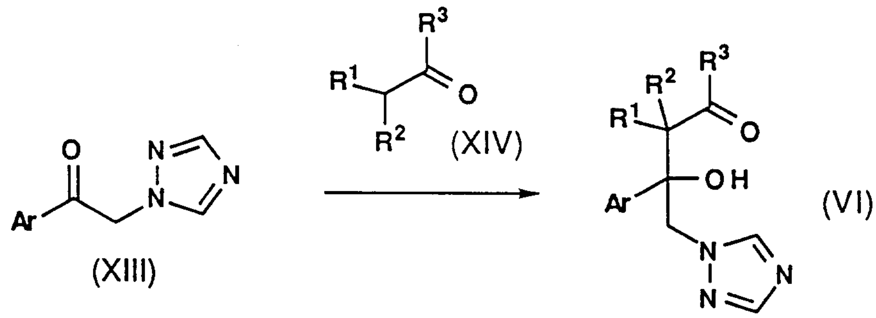

(XIII) (XIV) (VI)

Process E

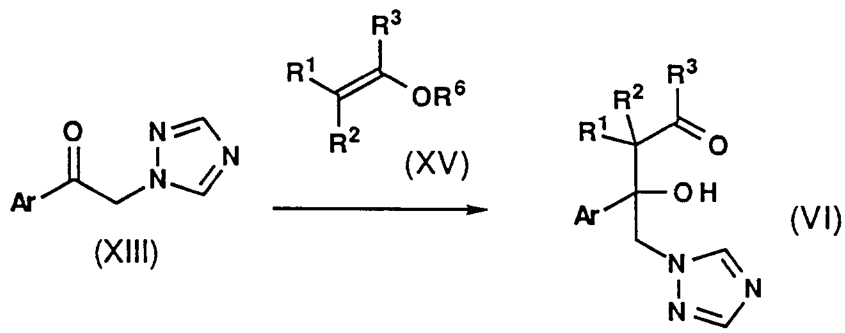

(XIII) (XV) (VI)

Process F

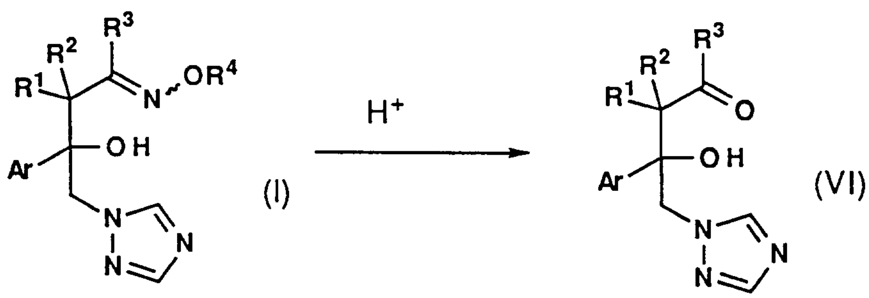


(I) (VI)

Process D

Ketone derivatives of the formula (VI) can be prepared from ketone derivatives of the formula (XIII):

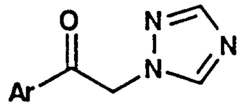

(XIII)

[where Ar has the same meaning as defined in the formula (I)] and ketone derivatives of the formula (XIV):

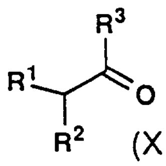

(XIV)

[where $R^1$, $R^2$ and $R^3$ have the same meanings as defined in the formula (I)] in accordance with a procedure known per se, for example, the aldol reaction which is described in Unexamined Published Japanese Patent Application (kokai) Sho 58-134079.

Process E

Compounds of the formula (XIII) and enol derivatives of the formula (XV):

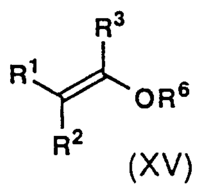

(XV)

[where $R^1$, $R^2$ and $R^3$ have the same meanings as defined in the formula (I) and $R^6$ is a trialkylsilyl group or a trialkylstanyl group) are prepared to a reaction known per se, such as by the method described in Journal of the American Chemical Society, 96, p. 7503 ff., 1974, particularly in accordance with the method of synthesis of compound 9 which is set forth on page 7504 or the method described in Chemistry Letters, p. 851 ff., 1983, particularly in accordance with the method of synthesis of compounds 3 and 4 which is set forth on page 851.

Process F

Oxime derivatives of the formula (I) are hydrolyzed in organic solvents or mixtures thereof with water, preferably using catalytic, equivalent or excess amounts of mineral acids (e.g. hydrochloric acid or sulfuric acid) or organic acids (e.g. trifluoroacetic acid or trifluoromethanesulfonic acid) at a temperature of -20 to 150 °C, preferably at 0 - 100 °C.

(Reaction Scheme 2)

[Processes for the preparation of compounds (VIII)]

(Process G)

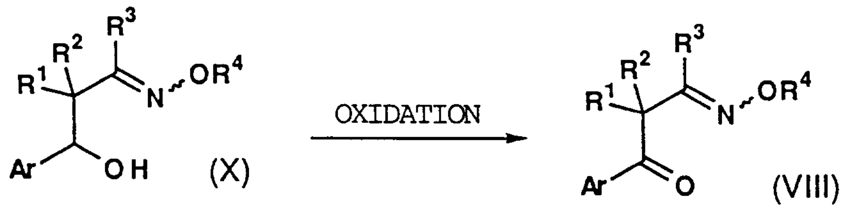


(Process H)

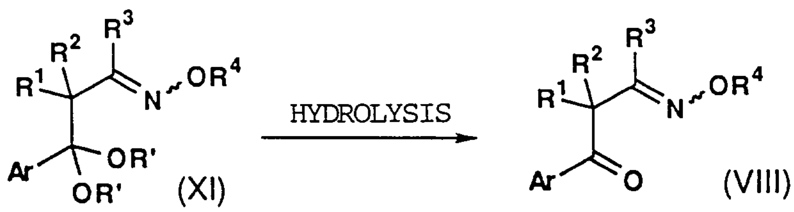


(Process I)

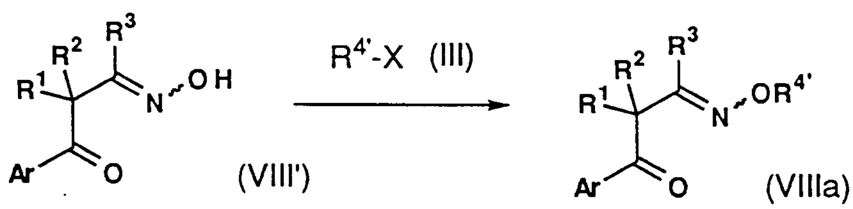


(Process J)

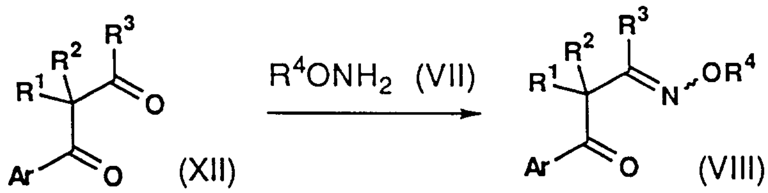

Process G

Hydroxyoxime derivatives of the formula (X):

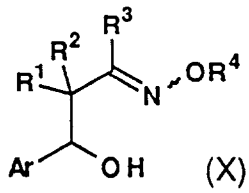

(X)

[where Ar, $R^1$, $R^2$, $R^3$, $R^4$ and the wavy line have the same meanings as defined in the formula (I)] which are prepared by a method to be described hereinafter are subjected to a treatment using a chromic acid derivative such as pyridinium chlorochromate (PCC) or pyridinium dichromate (PDC), for example, in accordance with the synthetic method of compound 4 which is described in "Synthesis", p. 567, 1981 or with the method described in Chapter 2 "Activated Dimethyl Sulfoxide Reagents", page 165 of the review of "Synthesis", 1981.

Process H

Oxime derivatives of the formula (XI):

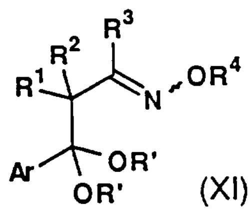

(XI)

(where Ar, $R^1$, $R^2$, $R^3$, $R^4$ and the wavy line have the same meanings as defined in the formula (I), and R' is a commonly employed ketone protective group) are hydrolyzed in organic solvents or mixtures thereof with water, preferably using catalytic, equivalent or excess amounts of mineral acids (e.g. hydrochloric acid or sulfuric acid) or organic acids (e.g. trifluoroacetic acid, p-toluenesulfonic acid or trifluoromethanesulfonic acid) at a temperature of -20 to 200 °C, preferably at 0 - 100 °C.

Process I

Oxime derivatives of the formula (VIII'):

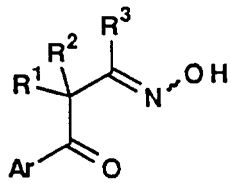

(VIII')

[where Ar, $R^1$, $R^2$, $R^3$ and the wavy line have the same meanings as defined in the formula (I)] which are embraced by the formula (VIII) are reacted with compounds of the formula (III) in a way similar to Process A, thereby obtaining compounds of the formula (VIIIa):

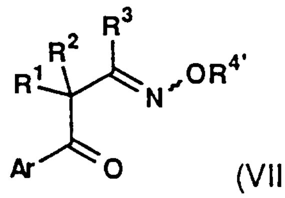

(VIIIa)

[where Ar, $R^1$, $R^2$, $R^3$ and the wavy line have the same meanings as defined in the formula (L), and $R^{4'}$ has the same meaning as defined in the formula (Ia)].

Process J

Diketone derivatives of the formula (XII):

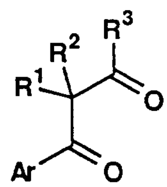

(XII)

[where Ar, $R^1$, $R^2$ and $R^3$ have the same meanings as defined in the formula (I)] are reacted with compounds of the formula (VII) in a way similar to Process C.

(Reaction Schemes 3 and 4)

(Processes for the preparation of compounds of (X) and (XI)]

To prepare compounds of the formula (X), compounds of the formula (XVI):

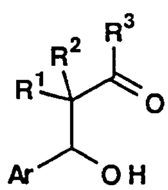

(XVI)

[where Ar, $R^1$, $R^2$ and $R^3$ have the same meanings as defined in the formula (I)] which are prepared by a method that is to be described hereinafter are reacted with compounds of the formula (VII) in a way similar to Process C. To prepare compounds of the formula (XI), compounds of the formula (XVII):

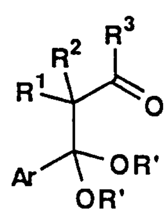

(XVII)

[where Ar, $R^1$, $R^2$ and $R^3$ have the same meanings as defined in the formula (I), and R' has the same meaning as defined in the formula (XI)] which are prepared by methods that are to be described hereinafter are reacted with compounds of the formula (VII) in a way similar to Process C.

Alternatively, if compounds of the formula (X'):

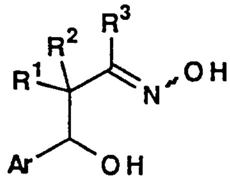

(X')

[where Ar, $R^1$, $R^2$, $R^3$ and the wavy line have the same meanings as defined in the formula (I)] are reacted with compounds of the formula (III) in a way similar to Process A, compounds of the formula (X) can be obtained as compounds of the formula (Xa):

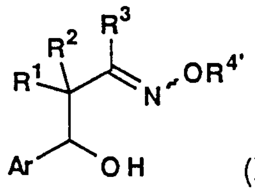

(Xa)

[wherein Ar, $R^1$, $R^2$, $R^3$ and the wavy line have the same meanings as defined in the formula (I), and $R^{4'}$ has the same meaning as defined in the formula (Ia)]. If compounds of the formula (XI'):

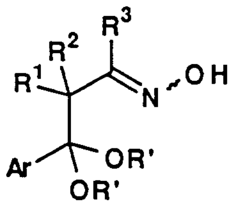

(XI')

[where Ar, $R^1$, $R^2$, $R^3$ and the wavy line have the same meanings as defined in the formula (I), and R' has the same meaning as defined in the formula (XI)] are reacted with compounds of the formula (III) in a way similar to Process A, compounds of the formula (XI) can be obtained as compounds of the formula (XIa):

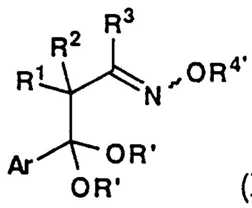

(XIa)

[where Ar, $R^1$, $R^2$, $R^3$ and the wavy line have the same meanings as defined in the formula (I). $R^{4'}$ has the same meaning as defined in the formula (Ia), and R' has the same meaning as defined in the formula (XI)].

[Process for the preparation of compounds (XII)]

To prepare compounds of the formula (XII), compounds of the formula (XVI) which are prepared by a method that is to be described hereinafter are subjected to the same treatment as in Process G.

(Reaction Schemes 5 and 6)

[Process for the preparation of compounds (XV)]

Compounds of the formula (XV) are typically obtained by performing the methods described in "Tetrahedron", 43, p. 2075 ff., 1987, particularly those described under the Compound Synthesis Method in Table 1 (p. 2077) and Table 2 (p. 2088), or the methods described in "Synthetic Communications", 18, p. 1679 ff., 1988, particularly those described under the Compound Synthesis Method in the table on page 1680.

[Processes for the preparation of compounds (XVI)]

To prepare compounds of the formula (XVI), compounds of the formula (XVIII):

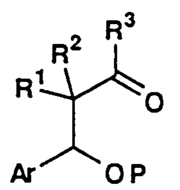

(XVIII)

(where Ar, $R^1$, $R^2$ and $R^3$ have the same meanings as defined in the formula (I), and P is a hydroxyl protective group) are deprotected by a common procedure of deprotecting hydroxyl groups, such as by referring to "Protective Groups in Organic Synthesis", supra. Alternatively, compounds of the formula (XIX):

Ar - CHO (XIX)

[where Ar has the same meaning as defined in the formula (I)] and compounds of the formula (XIV) or (XV) are subjected to the same treatment as in Process D or E. [Processes for the preparation of compounds (XVII) and (XVIII)]

Preparation of compounds of the formula (XVII) starts with ketosulfoxide derivatives of either the formula (XX):

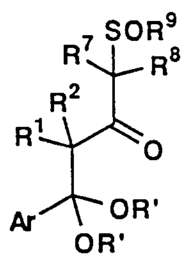

(XX)

(where Ar, $R^1$ and $R^2$ have the same meanings as defined in the formula (I), R' has the same meaning as defined in the formula (XI), $R^7$ and $R^8$ which may be the same or different are each a hydrogen atom or a straight-chained or branched alkyl group having 1 - 3 carbon atoms, and $R^9$ is a methyl, ethyl or phenyl group) or the formula (XXa):

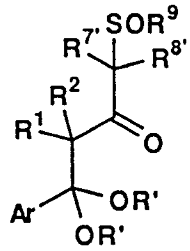

(XXa)

(where Ar, $R^1$ and $R^2$ have the same meanings as defined in the formula (I), R' has the same meaning as defined in the formula (XI), $R^{7'}$ and $R^{8'}$ which may be the same or different are each a straight-chained or branched alkyl group having 1 - 3 carbon atoms, and $R^9$ is a methyl, ethyl or phenyl group). Preparation of compounds of the formula (XVIII) starts with ketosulfoxide derivatives of either the formula (XXII):

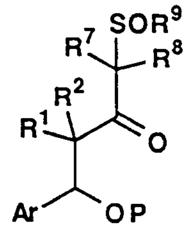

(XXII)

[where Ar, $R^1$ and $R^2$ have the same meanings as defined in the formula (I), $R^7$, $R^8$ and $R^9$ have the same meanings as defined in the formula (XX), and P has the same meaning as defined in the formula (XVIII)] or the formula (XXIIa):

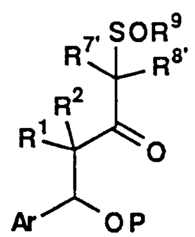

(XXIIa)

[where Ar, $R^1$ and $R^2$ have the same meanings as defined in the formula (I), R' has the same meaning as defined in the formula (XI), $R^{7'}$ and $R^{8'}$ and $R^9$ have the same meanings as defined in the formula (XXa)]. These starting materials are treated by known methods using a metal such as aluminum amalgam or zinc or a metal salt such as samarium iodide in accordance with the methods described in "Journal of the American Chemical Society", 104, p. 4180 ff., 1982 (particularly the method for the synthesis of compound 2 which is described on page 4181), "Journal of Organic Chemistry", 52, p. 2317 ff., 1987 (particularly the method for the synthesis of compound 1 which is described on page 2318), and "Journal of Organic Chemistry", supra, 51, p. 1135 ff., 1986 (particularly the method for the synthesis of cyclohexanone which is described in Table II on page 1136).

Alternatively, compounds of the formula (XXI):

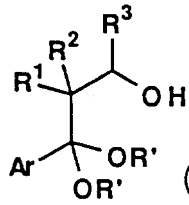

(XXI)

(where Ar, $R^1$, $R^2$ and $R^3$ have the same meanings as defined in the formula (I), and R' has the same meaning as defined in the formula (XI)] or compounds of the formula (XXIII):

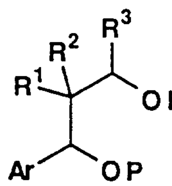

(XXIII)

[where Ar, $R^1$, $R^2$ and $R^3$ have the same meanings as defined in the formula (I), and P has the same meaning as defined in the formula (XVIII)] may be treated by the same method as in Process G.

[Processes for the preparation of compounds (XX) and (XXII)]

Preparation of compounds of the formula (XX) starts with compounds of the formula (XXIV):

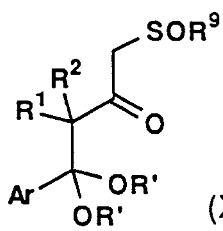

(XXIV)

[where Ar, $R^1$ and $R^2$ have the same meanings as defined in the formula (I), $R^9$ has the same meanings as defined in the formula (XX), and R' has the same meaning as defined in the formula (XI)] and compounds of the formula (XXV):

$R^{7'}$ - X (XXV)

[where $R^{7'}$ has the same meaning as defined in the formula (XXa), and X has the same meaning as defined in the formula (III)] and compounds of the formula (XXVI):

$R^{8'}$ - X (XXVI)

[where $R^{8'}$ has the same meaning as defined in the formula (XXa), and X has the same meaning as defined in the formula (III)].

Preparation of compounds of the formula (XXII) starts with the same compounds except that the compounds of the formula (XXIV) are replaced by those of the formula (XXXI):

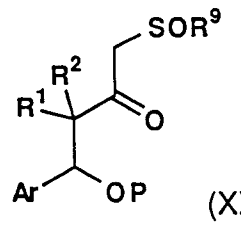

(XXXI)

[where Ar, $R^1$ and $R^2$ have the same meanings as defined in the formula (I), $R^9$ has the same meanings as defined in the formula (XX), and P has the same meaning as defined in the formula (XVIII)].

These starting materials are treated by procedures known per se such as, for example, the method described in "Journal of Organic Chemistry", 31, p. 2355 ff., 1966 (particularly the method of alkylation described in Table 1) and the method described in "Journal of Organic Chemistry", supra, 39, p. 732 ff., 1974 (particularly the method for the synthesis of compounds 4 and 6), whereby compounds of the formula (XX) or (XXII) are obtained as compounds of the formula (XXa) or (XXIIa), respectively.

Alternatively, compounds of the formula (XXVII):

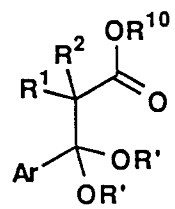

(XXVII)

(where Ar, $R^1$ and $R^2$ have the same meanings as defined in the formula (I), R' has the same meaning as defined in the formula (XI), and $R^{10}$ is a methyl or ethyl group) or compounds of the formula (XXXII):

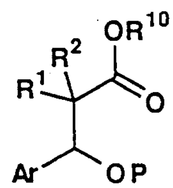

(XXXII)

[where Ar, $R^1$ and $R^2$ have the same meanings as defined in the formula (I), $R^{10}$ has the same meaning as defined in the formula (XXVII), and P has the same meaning as defined in the formula (XVIII)] are reacted with compounds of the formula (XXVIII):

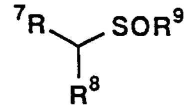

(XXVIII)

[where $R^7$, $R^8$ and $R^9$ have the same meanings as defined in the formula (XX)] by known procedures such as by referring to the method described in "Journal of the American Chemical Society", 87, p. 1345 ff., 1965 [particularly the method described on page 1346 for the synthesis of $\beta$-ketosulfoxide within the scope of formula (I)] or the method described in "Synthesis", p. 640 ff., 1980 (particularly the method of generating the carbanion of methylphenyl sulfoxide). If $R^7$ and $R^8$ are both a hydrogen atom, namely, in the case where the formula (XXVIII) is a more specific formula (XXVIII'):

$H_3C - SOR^9$

[where $R^9$ has the same meaning as defined in the formula (XX)], compounds of the formula (XXIV) or (XXXI) can respectively be obtained.

(Reaction Scheme 7)

[Processes for the preparation of compounds (XXI) and (XXIII)]

Compounds of the formula (XXIX):

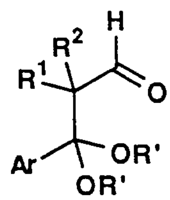

(XXIX)

[where Ar, $R^1$ and $R^2$ have the same meanings as defined in the formula (I), and R' has the same meaning as defined in the formula (XI)] or compounds of the formula (XXXIII):

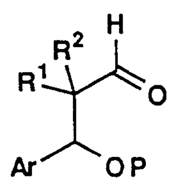

(XXXIII)

[where Ar, $R^1$ and $R^2$ have the same meanings as defined in the formula (I), and P has the same meaning as defined in the formula (XVIII)] are reacted with compounds of the formula (XXX):

$R^3$ - M (XXX)

(where $R^3$ has the same meaning as defined in the formula (I) and M is a metal such as lithium, sodium, a magnesium derivative or an aluminum derivative or a trialkylsilyl group) by known procedures such as in accordance with the method described in "Journal of Chemical Society Parkin Transaction II), p. 372, 1975 (particularly the method of synthesis of compound 20) or the method described in Unexamined Published Japanese Patent Application (kokai) Hei 2-256627 (particularly the one described in Example 1 under the Synthetic Method).

[Process for the preparation of compounds (XXVII)]

Compounds of the formula (XXXIV):

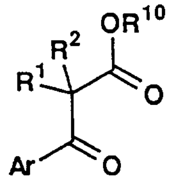

(XXXIV)

[where Ar, $R^1$ and $R^2$ have the same meanings as defined in the formula (I), and $R^{10}$ has the same meaning as defined in the formula (XXVII)] are treated by a common procedure for ketone protection.

[Processes for the preparation of compounds (XXIX) and (XXXIII)]

To prepare compounds of the formula (XXIX):

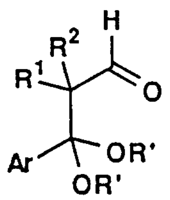

(XXIX)

[where Ar, $R^1$ and $R^2$ have the same meanings as defined in the formula (I), and R' has the same meaning as defined in the formula (XI)] or compounds of the formula (XXXIII), compounds of the formula (XXVII) or (XXXII), respectively, are reduced and subsequently treated by a known procedure such as in accordance with the method described in "Journal of Organic Chemistry", 49, p. 3784 (particularly the one described under the Synthetic method of compound 12).

Alternatively, reduction is made to compounds of the formula (XXXV):

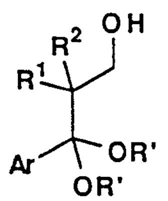

(XXXV)

[where Ar, $R^1$ and $R^2$ have the same meanings as defined in the formula (I), and R' has the same meaning as defined in the formula (XI)] or compounds of the formula (XXXVII):

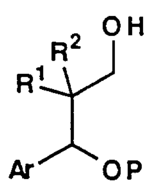

(XXXVII)

[where Ar, $R^1$ and $R^2$ have the same meanings as defined in the formula (I), and P has the same meaning as defined in the formula (XVIII)] and these compounds are subsequently treated by a known procedure such as in accordance with the method described in "Journal of Organic Chemistry", 49, p. 3784 (particularly the one described under the Synthetic method of compound 10) and thereafter oxidation is performed in the same manner as in Process G.

[Process for the preparation of compounds (XXXII)]

Compounds of the formula (XXXII):

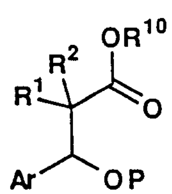

(XXXII)

[where Ar, $R^1$ and $R^2$ have the same meanings as defined in the formula (I), $R^{10}$ has the same meaning as defined in the formula (XXVII), and P has the same meaning as defined in the formula (XVIII)] can be prepared by protecting the hydroxyl group in compounds of the formula (XXXVI):

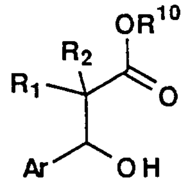

(XXXVI)

[where Ar, $R^1$ and $R^2$ have the same meanings as defined in the formula (I), and $R^{10}$ has the same meaning as defined in the formula (XXVII)] in accordance with an existing procedure.

The thus produced compounds or salts thereof may, after the end of the respective reactions, be treated in the usual manner or by specially adapted methods, or subjected to isolation and purification procedures such as column chromatography, recrystallization and extraction.

For medical use, the compounds of the present invention may be formulated in various dosage forms by mixing them with pharmaceutically acceptable excipients, binders, lubricants, colorants, flavoring agents, disintegrators, antiseptics, isotonizers, stabilizers, dispersants, antioxidants, buffering agents, preservatives, aromatics, suspending agents or emulsifiers, commonly employed suitable carriers or solvents which may optionally be sterilized water, vegetable oils or even physiologically acceptable solvents or solubilizers, which may be used in appropriate combinations.

Exemplary dosage forms include tablets, capsules, granules, powders, rectal suppositories, vaginal suppositories, syrups, inhalants, ointments, emulsions, suspensions, gels, jellies, creams, solutions, aerosols, solutions for eye drops, aqueous or nonaqueous injections, emulsifiable or suspensable injections, as well as solid injections that are solubilized, emulsified or suspended just prior to use. These dosage forms may be administered to patients by either oral or parenteral route (e.g. intravenously, intramuscularly, subcutaneously, intrarectally, intravaginally, percutaneous adsorption or transmucosal absorption) or, alternatively, they may be applied to diaphragm pessaries or the like.

The compounds of the invention exhibit strong antimycotic action and prove effective against both superficial and deep mycoses in humans and animals.

Stated specifically, the compounds can effectively be used in the treatment of various superficial dermatomycoses, deep dermatomycoses, and deep mycoses (mycoses in internal organs) caused by dermatophytes (Trichophyton, Microsporum and Epidermophyton), Candida, Malassezia, Aspergillus, Cryptococcus, Sporothrix and Fonsecaea), as exemplified by favus such as tinea corporis, tinea cruris, tinea manus, tinea pedis, tinea capitis, Celsus' kerion and tinea sycosis, candidosis such as buccal candidosis, dermal candidosis, candida sycosis and chronic mucocutaneous candidosis, tinea versicolor, Malassezia folliculitis, sporotrichosis, chromomycosis, fungemia, mycoses in respiratory organs, mycoses in digestive tracts, mycoses in urinary tracts and fungal meningitis.

The compounds of the invention are to be administered in doses that depend on the severity of the disease to be treated. Normally, the dose for systemic application (by either oral or parenteral route such as intravenous injection) is typically in the range from about 0.1 to about 1,000 mg, more preferably from about 1 to about 600 mg, more preferably from about 10 to about 400 mg, to an adult per day. Needless to say, the dose can be adjusted as appropriate for such factors as the condition of the patient.

If desired, the total dosage may be administered orally or parenterally either at a time or in 2 - 6 divided portions; intravenous drip infusion is of course possible.

The dose for topical application also varies with the severity of the disease to be treated; to take ointments as an example, the compounds of the invention may be formulated to be contained in amounts of 0.1 - 150 mg per gram of a base.

Formulations for topical use may be applied or to the affected area otherwise administered continuously either once or two or more times a day.

The compounds of the invention may form salts with pharmaceutically acceptable inorganic or organic acids. Exemplary salts that can be formed include inorganic acid salts such as hydrochlorides, sulfates and nitrates, organic acid salts such as acetates, p-toluenesulfonates and methanesulfonates, and amino acid salts such as glutamates and aspartates. These salts may be prepared in the usual manner, such as by mixing an equivalent amount of a compound of the invention in the free state with a solution containing a

desired acid and the resulting desired salt is recovered either by filtration or by removing the solvent. It should also be noted that the compounds of the invention may form solvates with various pharmaceutically acceptable solvents such as water, ethanol, glycerin, acetic acid, etc.

The pharmacological actions of representative compounds of the invention will now be described. The numbers assigned to the compounds used in the following tests are keyed to the example numbers.

Experiment 1.

Minimum Inhibitory Concentration (MIC)

MIC measurements were conducted by an agar plate dilution method. Stated specifically, Aspergillus fumigatus strains IFM0063 and IFM4942 were cultured on a potato dextrose agar medium at 27°C for 10 days, and the growing conidia were suspended in a sterile physiological saline containing 0.05% Tween 80 to give a concentration of $10^8$ - $10^9$ conidia per ml. The suspension was conditioned in a Sabouraud's dextrose broth medium at a concentration of $10^5$ spores per ml and a 5-µl aliquot of the culture was inoculated into a drug-containing Sabouraud's dextrose agar medium and cultured at 30°C for 2 days. The growth of fungi was observed visually and the minimum concentration of the test drug that could inhibit the fungal growth was designated as its MIC.

The results are shown in Table 1. The control drug was fluconazole.

Table 1

| | MIC (µg/ml) | |
|---|---|---|
| | Strain IFM0063 | Strain IFM4942 |
| Ex. No. 1 | 25 | 25 |
| 2 | 3.13 | 6.25 |
| 4 | 12.5 | 12.5 |
| 6 | 3.13 | 3.13 |
| 8 | 12.5 | 25 |
| 9 | 6.25 | 6.25 |
| 10 | 6.25 | 12.5 |
| 11 | 3.13 | 3.13 |
| 15 | 12.5 | 12.5 |
| 21 | 3.13 | 3.13 |
| 22 | 1.56 | 3.13 |
| 29 | 6.25 | 12.5 |
| 38 | 12.5 | 12.5 |
| 40 | 12.5 | 12.5 |
| 41 | 12.5 | 12.5 |
| 42 | 25 | 25 |
| 44 | 12.5 | 12.5 |
| 51 | 3.13 | 3.13 |
| 53 | 1.56 | 3.13 |
| 55 | 3.13 | 3.13 |
| 56 | 6.25 | 6.25 |
| 59 | 6.25 | 6.25 |
| Fluconazole | > 100 | > 100 |

As shown in Table 1, the selected compounds of the invention exhibited a strong antimycotic activity against Aspergillus fumigatus in the in vitro experiment. On the other hand, the control fluconazole had MIC values in excess of 100 µg/ml.

Experiment 2.

Protocol 1 for Experiment on the Treatment of Infection

Mice infected with Aspergillus fumigatus were administered selected compounds of the invention orally and their therapeutic effect was measured.

Stated specifically, ICR male mice (4-wk old) in groups each consisting of 10 animals were injected with Aspergillus fumigatus strain IFM41944 by injecting it into the tail vein at a concentration of $1 \times 10^7$ conidia per animal.

Each test compound was administered orally to the mice one hour after the infection and for the following four continuous days on a once-a-day basis. The number of mice that survived at the 7th day of the infection was counted (the day of infection was day "zero") and the $ED_{50}$ of each test compound was calculated by the Litchfield-Wilcoxon method to estimate its therapeutic effect.

The results are shown in Table 2. The control drugs were fluconazole and itraconazole.

Table 2

| | $ED_{50}$ (mg/kg) |
|---|---|
| Ex. No. 1 | 30.3 |
| 2 | 41.7 |
| 8 | 27.7 |
| 9 | 20.2 |
| 30 | 43.4 |
| Fluconazole | > 100 |
| Itraconazole | > 100 |

As shown in Table 2, the test compounds of the invention exhibited a satisfactory antimycotic activity in the Aspergillus fumigatus infected laboratory animal models. In contrast, the control fluconazole and itraconazole did not show any antimycotic action in the experimental models even when they were administered at doses up to 100 mg/kg.

Experiment 3.

Protocol 2 for Experiment on the Treatment of Infection

Mice infected with Candida albicans were administered selected compounds of the invention orally and their therapeutic effect was measured.

Stated specifically, ICR male mice (4-wk old) in groups each consisting of 10 animals were injected with Candida albicans strain IFM40009 by injecting it into the tail vein at a concentration of $1 \times 10^6$ cells per animal. Each test compound was administered orally to the mice once after an hour of infection.

The number of mice that survived at the 7th day of the infection was counted (the day of infection was day "zero") and the $ED_{50}$ of each test compound was calculated by the Litchfield-Wilcoxon method to estimate its therapeutic effect.

The results are shown in Table 3. The control drug was fluconazole.

Table 3

| | $ED_{50}$ (mg/kg) |
|---|---|
| Ex. No. 1 | 1.0 |
| 2 | 2.6 |
| 4 | 0.8 |
| 6 | 1.4 |
| 7 | 8.2 |
| 8 | 13.2 |
| 9 | 7.5 |
| 10 | 10.0 |
| 11 | 4.1 |
| 21 | 13.0 |
| 22 | 5.5 |
| 29 | 13.3 |
| 30 | 7.3 |
| 34 | 14.6 |
| 35 | 13.8 |
| 38 | 13.4 |
| 40 | 11.2 |
| 41 | 4.4 |
| 42 | 14.6 |
| 43 | 14.7 |

Table 3 (continued)

| | $ED_{50}$ (mg/kg) |
|---|---|
| Ex. No. 44 | 14.6 |
| 46 | 15.7 |
| 51 | 11.3 |
| 53 | 13.5 |
| 55 | 14.7 |
| 56 | 14.7 |
| 58 | 13.2 |
| 59 | 10.7 |
| 72 | 13.5 |
| 73 | 5.8 |
| 74 | 8.9 |
| 75 | 8.6 |
| Fluconazole | 11.7 |

As shown in Table 3, the test compounds of the invention exhibited a comparable or stronger antimycotic activity than fluconazole in the Candida albicans infected laboratory animal models.

Experiment 4.

Protocol 3 for Experiment on the Treatment of Infection

Experimentally prepared immunosuppressed mice were infected with Candida albicans; they were then administered selected compounds of the invention orally and the therapeutic effect of the compounds was measured.

Stated specifically, ICR male mice (4-wk old) in groups each consisting of 10 animals were administered cyclophosphamide intraperitoneally at a dose of 100 mg/kg; four days later, the mice were similarly administered cyclophosphamide intraperitoneally to be rendered experimentally immunosuppressed. Simultaneously with the second shot of cyclophosphamide, Candida albicans strain IFM40009 was administered to the tail vein of each animal at a concentration of $1 \times 10^6$ cells per animal to induce infection. Each test compound was administered orally to the mice once after an hour of infection.

The number of mice that survived at the 7th day of the infection was counted (the day of infection was day "zero") and the $ED_{50}$ of each test compound was calculated by the Litchfield-Wilcoxon method to estimate its therapeutic effect.

The results are shown in Table 4. The control drug was fluconazole.

Table 4

| | $ED_{50}$ (mg/kg) |
|---|---|
| Ex. No. 1 | 4.9 |
| 2 | 4.9 |
| Fluconazole | 54.4 |

As shown in Table 4, the test compounds of the invention were at least ten times as effective as the control fluconazole in the treatment of the mice that were infected with Candida albicans after being rendered immunosuppressed by experimentally reducing neutrophiles.

Experiment 5.

Toxicity Test

The four selected compounds of the invention (those prepared in Examples 1, 2, 9 and 30) were administered orally to 4-wk old mice for 5 consecutive days at a dose of 100 mg/kg. The mice showed no evidence for abnormality.

The results of Experiments 1 - 4 showed that the compounds of the invention exhibited a marked antimycotic action against Aspergillus species both in an in vitro experiment and in in vivo experiments with infected laboratory animal models according to protocols that were so designed that fluconazole, one of the most popular commercial azol antimycotics, would be ineffective.

The commercial itraconazole was also ineffective in an in vivo experiment with infected laboratory animal models even when it was administered at a dose of 100 mg/kg; on the other hand, the compounds of the invention exhibited a marked antimycotic action in the same experiment. Therefore, they are anticipated to be more useful antimycotics than itraconazole. In addition, the results of Experiment 5 allow one to expect that the compounds of the invention will prove a more useful therapeutic for fungal infections than Bay R 3783 which has been reported to be comparable to itraconazole in antimycotic activity against Aspergillus.

The test compounds of the invention also exhibited a comparable antimycotic activity to fluconazole. The inventors further verified experimentally that the compounds also had a marked antimycotic activity against Cryptococcus species.

It is extremely difficult to identify causative microorganisms for mycoses in internal organs and their therapeutics are required to have a broad-spectrum antifungal activity. Considering this point as well as other features, the compounds of the invention are indeed anticipated to work more effectively than any other antimycotics.

The inventors also verified experimentally that compounds of the invention had a marked antimycotic activity against Tricophyton and other dermatophytes. It is certainly preferred from the viewpoint of mode of action that therapeutics prove effective for not only dermatomycoses but also mycoses in internal organs.

The compounds of the invention were also verified to show an outstanding antimycotic action in experimentally prepared immunosuppressed laboratory animals. Many patients with mycoses in internal organs also suffer from deficiency in the immune system owing to syndromic diseases such as cancers or AIDS and therapeutics for this particular kind of mycoses are required to exhibit an antimycotic activity even in immunosuppressed states. Considering this point as well as other features, the compounds of the invention are indeed anticipated to work more effectively than any other antimycotics.

It is interesting to note that the compounds of the invention caused nothing abnormal in laboratory animals even when they were administered the compounds continuously. The compounds of the invention have high specificity for the biosynthesis of ergosterol by fungi and, hence, are anticipated to be low in toxicity. As a further advantage, the compounds of the invention which are ketoxime derivatives will not be readily metabolized to nitrile derivatives which are held to develop hepatotoxicity and, hence, they are anticipated to be therapeutics that are extremely safe to use.

The compounds of the invention including the one prepared in Example 30 show good enough solubility to be useful as injections and they can be rendered even more soluble by being converted to salts.

Thus, the triazole derivatives of the invention have been found to possess the following features: they exhibit an outstanding in vitro antimycotic activity against Aspergillus species; they exhibit a marked therapeutic effect in an animal experiment using Aspergillus infected in vivo laboratory animal models (mice); they exhibit a marked therapeutic effect in an animal experiment using Candida infected in vivo laboratory animal models (mice); they also exhibit a marked therapeutic effect in an animal experiment using immunocompromised and Candida infected in vivo laboratory animal models (mice); they have good enough solubility to be useful as injections; and they are highly safe to use. The triazole derivatives of the invention have been found to excel in at least one of these features.

Therefore, the triazole derivatives of the invention are anticipated to be extremely useful in the treatment of various superficial dermatomycoses (e.g. favus such as tinea corporia, tinea cruris, tinea manus, tinea pedis, tinea capitis, Celsus' kerion and tinea sycosis; candidosis such as buccal candidosis, dermal candidosis, candida sycosis and chronic mucocutaneous candidosis; tinea versicolor and Malassezia

folliculitis), deep dermatomycoses (e.g. sporotrichosis and chromomycosis), and deep mycoses (mycoses in internal organs, e.g. fungemia, mycoses in respiratory organs, mycoses in digestive tracts, mycoses in urinary tracts and fungal meningitis).

The following reference examples and working examples are given for the purpose of further illustrating the present invention but are in no way to be taken as limiting.

The reference examples relate to the synthesis of intermediate compounds and the working examples (or simply "examples") relate to the synthesis of end compounds.

Unless otherwise noted, all of the compounds prepared in the reference examples and examples are racemates. NMR spectroscopy was recorded on JEOL FX90Q FT-NMR, JEOL FX90A FT-NMR (the data taken are preceded by an asterisk; both models are manufactured by JEOL LTD.) or JEOL JNM-270 FT-NMR (product of JEOL LTD.); IR spectroscopy was run with Nicolet 5DX FT-IR (the data taken are preceded by an asterisk; manufactured by Nicolet Co., Ltd.) or HORIBA FT-200 (manufactuerd by Horiba, Ltd.); melting point measurements were conducted with Mettler FP800 (manufactured by Mettler Co., Ltd.); specific rotation was measured with HORIBA SEPA-300 (manufactured by Horiba, Ltd.); and HPLC was run with a TRIROTOR-VI (manufactured by Japan Spectroscopic Co., Ltd.)

If NMR data for two diastereomeric mixtures do not agree in terms of chemical shifts, the following notation shall be used: 7.00 and 7.12 (s, 1H) meaning that two singlets that appear at 7.00 ppm and 7.12 ppm correspond to one hydrogen when combined together.

The data of specific rotation were taken with methanol used as a solvent at 27°C using the sodium D line.

Reference Example 1:

Sodium borohydride (4.78 g) was added to ethanol (350 ml) with ice-cooling and the mixture was stirred under a nitrogen atmosphere at the same temperature for 10 min, followed by stirring at room temperature for 50 min. A solution of an Ethyl 1-(2,4-difluorobenzoyl cyclopropanecarboxylate (57.9 g) in ethanol (180 ml) was added dropwise to the reaction mixture at -40°C over 30 min; the mixture was then stirred at the same temperature for 30 min, at -20°C for 1 h, and finally at -10°C for 2 h.

With the internal temperature held at 0°C, the reaction mixture was rendered acidic with 1 N HCl and concentrated under vacuum. Water (500 ml) was added to the residue and extraction was carried out with ethyl acetate (500 ml × 3). The organic layers were combined, washed with water (300 ml) and brine (300 ml) and subsequently dried over anhydrous sodium sulfate.

The desiccant was filtered off and the solvent was concentrated to dryness under vacuum to give an Ethyl 1-[(2,4-difluorophenyl)hydroxymethyl]cyclopropanecarboxylate (61.1 g; for its structure, see Fig. 1) as a colorless oil.

*NMR ($CDCl_3$) δ [ppm]:

0.52-1.04 (m, 2H), 1.04-1.41 (m, 5H), 3.67 (d, 1H, J=5.6Hz), 3.93-4.32 (m, 2H), 5.34 (d, 1H, J=4.6Hz), 6.56-7.02(m, 2H), 7.32-7.70 (m, 1H)

IR (neat) $cm^{-1}$:

3456, 1707, 1502, 1277, 1142, 1095, 1032, 966

Reference Example 2:

The procedure of Reference Example 1 was repeated using an Ethyl 1-(2,4-dichlorobenzoyl)-cyclopropanecarboxylate, whereby an Ethyl 1-[(2,4-dichlorophenyl)-hydroxymethyl]cyclopropanecarboxylate (see Fig. 1) was obtained as acolorless oil.

NMR ($CDCl_3$) δ [ppm]:

0.38-0.46 (m, 1H), 0.78-0.86 (m, 1H), 1.10-1.17 (m, 1H), 1.20-1.36 (m, 4H), 3.43 (s, 1H), 4.13-4.28 (m, 2H), 5.79 (s, 1H), 7.23-7.27 (m, 1H), 7.35 (d, 1H, J=2.0Hz), 7.50 (d, 1H, J=8.6Hz)

IR (neat) $cm^{-1}$:

3444, 1722, 1705, 1471, 1373, 1147, 1038

Reference Example 3:

The procedure of Reference Example 1 was repeated using an Ethyl 3-(2,4-difluorophenyl)-2,2-dimethyl-3-oxopropionate, whereby an Ethyl 3-(2,4-difluorophenyl)-3-hydroxy-2,2-dimethylpropionate (see Fig. 1) was obtained as acolorless oil.

*NMR ($CDCl_3$) δ [ppm]:

1.15 (s, 6H), 3.38 (d, 2H, J = 4.7Hz), 3.74 (s, 3H), 5.23 (d, 1H, J = 4.7Hz), 6.63-7.02 (m, 2H), 7.26-7.61 (m, 1H)
*IR (neat) $cm^{-1}$:
3480, 1718, 1617, 1506, 1134, 967

Reference Example 4:

p-Toluenesulfonic acid monohydrate (434 mg) was added to a solution of an Ethyl 1-[(2,4-difluorophenyl)hydroxymethyl]-cyclopropanecarboxylate (61.1 g) and dihydropyran (107 ml) in dichloromethane (840 ml) was added with ice-cooling, and the mixture was stirred at the same temperature for 10 min and at room temperature for an additional 2.5 h.

A saturated aqueous solution of sodium hydrogencarbonate (300 ml) was added to the reaction mixture for extraction. Further extraction was carried out with dichloromethane (100 ml). The organic layers were combined, washed with water (200 ml) and brine (200 ml), and dried over anhydrous sodium sulfate.

The desiccant was filtered off and the solvent was concentrated under vacuum and the residue was purified by silica gel column chromatography (eluent: 3% ethyl acetate-hexane). The fractions containing the end product were collected and concentrated under vacuum to give a diastereomeric mixture of an Ethyl 1-[(2,4-difluorophenyl)[(tetrahydro-2H-pyran-2-yl)oxy]methyl]cyclopropanecarboxylate (78.3 g; see Fig. 1) as a colorless oil.
*NMR ($CDCl_3$) δ [ppm]:
0.56-2.16 (m, 13H), 3.08-4.22 (m, 4H), 4.30-4.48 and 4.85-5.06 (m, 1H), 5.60 (s, 1H), 6.59-7.00 (m, 2H), 7.28-7.59 (m. 1H)
IR (neat) $cm^{-1}$:
1726, 1502, 1277, 1140, 1026, 966

Reference Example 5:

The procedure of Reference Example 4 was repeated using the compound prepared in Reference Example 2, whereby an Ethyl 1-[(2,4-dichlorophenyl)[(tetrahydro-2H-pyran-2-yl)oxy]methyl]-cyclopropanecarboxylate (see Fig. 1) was obtained as acolorless oil.
NMR ($CDCl_3$) δ [ppm]:
0.33-0.40 (m, 1H), 0.61-0.67 (m, 1H), 0.82-0.89 (m, 1H), 1.04-1.20 (m, 1H), 1.19 and 1.24 (t, 3H, J = 7.3Hz), 1.40-1.81 (m, 6H), 3.24-3.56 (m, 2H), 3.95-4.97 (m, 1H), 5.87 and 6.00 (s, 1H), 7.20-7.50 (m, 3H)
IR (KBr) $cm^{-1}$:
1724, 1470, 1373, 1282, 1174, 1144, 1026

Reference Example 6:

The procedure of Reference Example 4 was repeated using the compound prepared in Reference Example 3, whereby a Methyl 3-(2,4-difluorophenyl)-3-[(tetrahydro-2H-pyran-2-yl)oxy]-2,2-dimethylpropionate (see Fig. 1) was obtained as acolorless oil.
*NMR ($CDCl_3$) δ [ppm]:
0.93-1.23 (m, 6H), 1.29-1.80 (m, 6H), 3.03-4.05 (m, 2H), 3.70 and 3.72 (s, 3H), 4.29-4.44 and 4.65-4.77 (m, 1H), 5.21 and 5.37 (s, 1H), 6.60-7.02 (m, 2H), 7.17-7.58 (m, 1H)
IR (neat) $cm^{-1}$:
1741, 1618, 1508, 1268, 1134

Reference Example 7:

60% Sodium hydride (13.3 g) was added to a solution of tetrahydrofuran (220 ml) and dimethyl sulfoxide (162 ml) and the mixture was stirred at 60 - 65 °C for 2.5 h.

To the stirred reaction mixture, a solution of Ethyl 1-[(2,4-difluorophenyl)[(tetrahydro-2H-pyran-2-yl)oxy]-methyl]cyclopropanecarboxylate (78.3 g) in tetrahydrofuran (210 ml) was added dropwise with ice-cooling water over 30 min. The reaction mixture was stirred at the same temperature for 1 h, poured into ice water and adjusted to pH 4 with a saturated aqueous solution of citric acid, followed by extraction with ethyl acetate (500 ml × 3). The organic layers were combined, washed with water (400 ml) and brine (400 ml) and dried over anhydrous sodium sulfate.

The desiccant was filtered off and the solvent was concentrated under vacuum and the residue was purified by silica gel column chromatography (eluant: hexane/ethyl acetate = 4/1 to 5% methanol-ethyl acetate). The fractions containing the end product were collected and concentrated under vacuum to give 1-[1-[(2,4-difluorophenyl)[(tetrahydro-2H-pyran-2-yl)oxy]methyl]cyclopropyl]-2-methylsulfinylethanone (70.3 g; see Fig. 1) as an orange oil.

*NMR ($CDCl_3$) $\delta$ [ppm]:

0.52-2.19 (m, 10H), 2.67 and 2.69 (s, 3H), 3.16-3.69 (m, 2H), 3.69-4.14 (m, 2H), 4.14-4.46 and 4.76-4.98 (m, 1H), 5.54-5.92 (m, 1H), 6.65-7.02 (m, 2H), 7.16-7.66 (m, 1H)

IR (neat) $cm^{-1}$:

1684, 1618, 1502, 1273, 1119, 1076, 1026, 966

Reference Example 8:

n-Butyl lithium (3.88 ml; sol. in 1.6 M hexane) was added dropwise to a solution of diisopropylamine (0.67 g) in tetrahydrofuran (2.5 ml) at -78 °C over 3 min, and the mixture was stirred at 0 °C for 15 min. Ethylphenyl sulfoxide (0.92 g) was added dropwise to the reaction mixture at -78 °C over 5 min, followed by stirring at -78 °C for 1 h. To the reaction mixture, a solution of an Ethyl 1-[1-(2,4-dichlorophenyl)[(tetrahydro-2H-pyran-2-yl)oxy]methyl]cyclopropanecarboxylate (2.22 g) in tetrahydrofuran (5 ml) was added dropwise at -78 °C over 10 min. The reaction mixture was then stirred at -78 °C for 80 min.

A saturated aqueous solution of ammonium chloride (20 ml) was added to the reaction mixture at -78 °C. After allowed to warm to room temperature, extraction was carried out with ethyl acetate (20 ml × 3) and the organic layers were combined, washed with water (20 ml) and brine (20 ml) and dried over anhydrous sodium sulfate. After filtering off the desiccant, the solvent was concentrated under vacuum and the residue was purified by silica gel column chromatography (eluant: hexane/acetone = 40/1 - 30/1).

The fractions containing the end product were collected and concentrated under vacuum to give 1-[(1-[-(2,4-dichlorophenyl)[(tetrahydro-2H-pyran-2-yl)oxy]methyl]cyclopropyl]-2-phenylsulfinyl-1-propanone (1.26 g; see Fig. 1) as a pale yellow oil.

NMR ($CDCl_3$) $\delta$ [ppm]:

0.15-1.90 (m, 13H), 3.25-5.00 (m, 3H), 5.80-6.42 (m, 1H), 7.14-7.94 (m, 8H)

IR (KBr) $cm^{-1}$:

1684, 1444, 1378, 1119, 1045, 978

Reference Example 9:

The procedure of Reference Example 7 was repeated using the compound prepared in Reference Example 6, whereby 4-(2,4-difluorophenyl)-4-[(tetrahydro-2H-pyran-2-yl)oxy]-3,3-dimethyl-1-methylsulfinyl-2-butanone (see Fig. 1) was obtained as a yellow oil.

*NMR ($CDCl_3$) $\delta$ [ppm]:

0.90-1.24 (m, 6H), 1.30-1.90 (m, 6H), 2.77 (s, 3H), 3.06-4.78 (m, 5H), 5.11-5.40 (m, 1H), 6.63-7.62 (m, 3H)

*IR (neat) $cm^{-1}$:

1702, 1618, 1503, 1265, 1036

Reference Example 10:

60% Sodium hydride (2.01 g) was added to dimethyl sulfoxide (65 ml) and the mixture was stirred under a nitrogen atmosphere at 60 - 70 °C for 2 h. After cooling to room temperature, a solution of 1-[1-[-(2,4-difluorophenyl)[(tetrahydro-2H-pyran-2-yl)oxy]methyl]cyclopropyl]-2-methylsulfinylethanone (17.0 g) in dimethyl sulfoxide (50 ml) was added dropwise at room temperature over 20 min. The reaction mixture was stirred at room temperature for 20 min; thereafter, methyl iodide (3.19 ml) was added dropwise over 30 min at 20 - 25 °C, followed by stirring at the same temperature for 60 min.

The reaction mixture was poured into ice water and extraction was carried out with ethyl acetate (200 ml × 3); the organic layers were combined, washed with water (200 ml) and brine (200 ml), and dried over anhydrous sodium sulfate.

After filtering off the desiccant, the solvent was concentrated under vacuum and the residue was purified by silica gel column chromatography (eluant: hexane/acetone = 4/1 - 3/1). The fractions containing the end product were collected and concentrated under vacuum to give 1-[1-[(2,4-difluorophenyl)[-(tetrahydro-2H-pyran-2-yl)oxy]methyl]cyclopropyl]-2-methylsulfinyl-1-propanone (15.5 g; see Fig. 1) as a

pale yellow oil.
*NMR ($CDCl_3$) δ [ppm]:
0.54-2.04 (m, 13H), 2.28-2.59 (m, 3H), 3.18-4.98 (m, 4H), 5.58-5.99 (m, 1H), 6.62-7.03 (m, 2H), 7.18-7.68 (m, 1H)
IR (neat) $cm^{-1}$:
1682, 1616, 1502, 1271, 1119, 1026, 966

Reference Example 11:

60% Sodium hydride (6.80 g) was added to a solution of dimethyl sulfoxide (230 ml) and the mixture was stirred under a nitrogen atmosphere at 60 - 70 ° C for 2 h. After cooling to room temperature, a solution of 1-[1-[(2,4-difluorophenyl)[(tetrahydro-2H-pyran-2-yl)oxy]methyl]cyclopropyl]-2-methylsulfinylethanone (60.3 g) in dimethyl sulfoxide (180 ml) was added dropwise at room temperature over 30 min. The reaction mixture was stirred at the same temperature for 30 min and, thereafter, methyl iodide (10.6 ml) was added dropwise over 30 min at 20 - 25 ° C, followed by stirring at the same temperature for 30 min.

To the reaction mixture, a preliminarily prepared dimsyl anion solution (prepared by adding 60% sodium hydride (6.80 g) to a solution of dimethyl sulfoxide (230 ml) and then stirring the mixture under a nitrogen atmosphere at 60 - 70 ° C for 2 h) was added over 30 min. at 20 - 25 ° C. The reaction mixture was stirred at the same temperature for 30 min and, thereafter, methyl iodide (10.6 ml) was added dropwise at 20 - 25 ° C over 30 min, followed by stirring at the same temperature for 30 min.

The reaction mixture was poured into ice water and extraction was carried out with ethyl acetate (500 ml × 3). The organic layers were combined, washed with water (400 ml) and brine (400 ml) and dried over anhydrous sodium sulfate. After filtering off the desiccant, the solvent was concentrated under vacuum and the residue was purified by silica gel column chromatography (eluant: hexane/ethyl acetate = 1/1).

The fractions containing the end product were collected and concentrated under vacuum to give 1-[1-[-(2,4-difluorophenyl)[(tetrahydro-2H-pyran-2-yl)oxy]methyl]cyclopropyl]-2-methyl-2-methylsulfinyl-1-propanone (70.3 g; see Fig. 1) as a yellow oil.
*NMR ($CDCl_3$) δ [ppm]:
0.55-1.94 (m, 16H), 2.10-2.40 (m, 3H), 3.07-3.61 and 3.77-4.03 (m, 2H), 4.17-4.43 and 4.68-4.92 (m, 1H), 5.50-5.82 (m, 1H), 6.63-7.03 (m, 2H), 7.16-7.64 (m, 1H)
IR (KBr) $cm^{-1}$:
1678, 1618, 1502, 1271, 11220, 1024, 964

Reference Example 12:

The procedure of Reference Example 10 was repeated using the compound prepared in Reference Example 7 and replacing methyl iodide with ethyl iodide, whereby 1-[1-[(2,4-difluoromethyl)[(tetrahydro-2H-pyran-2-yl)oxy]methyl]cyclopropyl]-2-methylsulfinyl-1-butanone (see Fig. 1) was obtained as apale yellow oil.
NMR ($CDCl_3$) δ [ppm]:
0.60-2.70 (m, 18H), 3.20-6.10 (m, 5H), 6.70-7.60 (m, 3H)
IR (neat) $cm^{-1}$:
1680, 1502, 1273, 1119, 1026, 966

Reference Example 13:

The procedure of Reference Example 10 was repeated using the compound prepared in Reference Example 9, whereby 1-(2,4-difluorophenyl)-1-[(tetrahydro-2H-pyran-2-yl)oxy]-2,2-dimethyl-4-methylsulfinyl-3-pentanone (see Fig. 1) was obtained as apale yellow oil.
*NMR ($CDCl_3$) δ [ppm]:
0.60-2.10 (m, 17H), 0.22-2.70 (m, 3H), 2.98-5.58 (m, 3H), 6.60-7.62 (m, 3H)
*IR (KBr) $cm^{-1}$:
1692, 1618, 1503, 1120, 1054, 1032, 964

Reference Example 14:

30% aqueous solution of Sodium hydroxide (187 ml) was added dropwise to a suspension of zinc powder (37.0 g) in water (360 ml) at 65 ° C over 10 min and the mixture was stirred at the same temperature

for 20 min. To the stirred reaction mixture, a solution of 1-[1-[(2,4-difluorophenyl)[(tetrahydro-2H-pyran-2-yl)-oxy]methyl]cyclopropyl]-2-methyl-2-methylsulfinyl-1-propanone (39.1 g) in toluene (660 ml) was added at 65 °C over 30 min and the mixture was stirred at the same temperature for 2 h.

The reaction mixture was cooled and filtered to remove the insoluble matter. The filtrate was extracted, followed by further extraction with ether (200 ml × 2). The organic layers were combined, washed with water (200 ml) and brine (200 ml) and dried over anhydrous sodium sulfate. The desiccant was filtered off and the solvent was concentrated to dryness under vacuum, to give 1-[1-[(2,4-difluorophenyl)[(tetrahydro-2H-pyran-2-yl)oxy]methyl]cyclopropyl]-2-methyl-1-propanone (32.2 g; see Fig. 1) as a pale yellow oil.

*NMR ($CDCl_3$) δ [ppm]:

0.60-1.23 (m, 10H), 1.23-1.92 (m, 6H), 2.38-3.09 (m, 1H), 3.09-3.66 and 3.72-4.08 (m, 2H), 4.29-4.47 and 4.71-4.98 (m, 1H), 5.75 and 5.83 (s, 1H), 6.59-6.99 (m,2H), 7.17-7.65 (m,1H)

IR (KBr) $cm^{-1}$:

1691, 1618, 1502, 1273, 1120, 1026, 966

Reference Example 15:

The procedure of Reference Example 14 was repeated using the compound prepared in Reference Example 7, whereby 1-[1-[(2,4-difluorophenyl)[(tetrahydro-2H-pyran-2-yl)oxy]methyl]cyclopropyl]-1-ethanone (see Fig. 1) was obtained as acolorless oil.

*NMR ($CDCl_3$) δ [ppm]:

0.60-2.40 (m, 10H), 1.04 and 2.19 (s, 3H), 3.12-4.11 (m, 2H), 4.26-4.48 and 4.81-5.00 (m, 1H), 5.72 and 5.81 (s, 1H), 6.60-7.08 (m, 2H), 7.11-7.59 (m, 1H) IR (neat) $cm^{-1}$:

1691, 1502, 1138, 1119, 1024, 966

Reference Example 16:

The procedure of Reference Example 14 was repeated using the compound prepared in Reference Example 10, whereby 1-[1-[(2,4-difluorophenyl)[(tetrahydro-2H-pyran-2-yl)oxy]methyl]cyclopropyl]-1-propanone (see Fig. 1) was obtained as acolorless oil.

*NMR ($CDCl_3$) δ [ppm]:

0.50-1.28 (m, 7H), 1.28-2.02 (m, 6H), 2.15-2.69 (m, 2H), 3.12-3.66 and 3.66-4.06 (m, 2H), 4.20-4.46 and 4.74-5.06 (m, 1H), 5.73 and 5.82 (s, 1H), 6.61-6.99 (m, 2H), 7.21-7.64 (m, 1H)

IR (neat) $cm^{-1}$:

1693, 1618, 1502, 1273, 1120, 1026, 966

Reference Example 17:

The procedure of Reference Example 14 was repeated using the compound prepared in Reference Example 12, whereby 1-[1-[(2,4-difluorophenyl)[(tetrahydro-2H-pyran-2-yl)oxy]methyl]cyclopropyl]-1-butanone (see Fig. 1) was obtained as acolorless oil.

NMR ($CDCl_3$) δ [ppm]:

0.62-1.28 (m, 7H), 1.43-1.84 (m, 8H), 2.24 (t, 1H, J = 7.3Hz), 2.33-2.56 (m, 1H), 3.26-3.96 (m, 2H), 4.35-4.89 (m, 1H), 5.75 and 5.83 (s,1H), 6.71-6.89 (m, 2H), 7.31-7.52 (m, 1H)

IR (neat) $cm^{-1}$:

1691, 1502, 1273, 1119, 1024, 966

Reference Example 18:

The procedure of Reference Example 14 was repeated using the compound prepared in Reference Example 8, whereby 1-[1-[(2,4-dichlorophenyl)[(tetrahydro-2H-pyran-2-yl)oxy]methyl]cyclopropyl]-1-propane (see Fig. 1) was obtained as acolorless oil.

*NMR ($CDCl_3$) δ [ppm]:

0.28-2.04 (m, 13H), 2.12-2.85 (m, 2H), 3.08-3.69 and 3.69-4.11 (m, 2H), 4.11-4.40 and 4.75-4.94 (m, 1H), 6.00 and 6.16 (s, 1H), 7.02-7.68 (m, 3H)

IR (neat) $cm^{-1}$:

1693, 1468, 1379, 1120, 1024, 980

Reference Example 19:

The procedure of Reference Example 14 was repeated using the compound prepared in Reference Example 9, whereby 4-(2,4-difluorophenyl)-4-[(tetrahydro-2H-pyran-2-yl)oxy]-3,3-dimethyl-2-butanone (see Fig. 1) was obtained as ayellow oil.

*NMR ($CDCl_3$) $\delta$ [ppm]:

0.87-1.17 (m, 6H), 1.29-1.83 (m, 6H), 2.25 and 2.34 (s, 3H), 3.00-3.96 (m, 2H), 4.23-4.35 and 4.56-4.71 (m, 1H), 5.17 and 5.36 (s, 1H), 6.63-7.62 (m, 3H)

*IR (neat) $cm^{-1}$:

1708, 1618, 1503, 1266, 1120, 1034

Reference Example 20:

The procedure of Reference Example 14 was repeated using the compound prepared in Reference Example 13, whereby 1-(2,4-difluorophenyl)-1-[(tetrahydro-2H-pyran-2-yl)oxy]-2,2-dimethyl-3-pentanone (see Fig. 1) was obtained as ayellow oil.

*NMR ($CDCl_3$) $\delta$ [ppm]:

0.86-1.77 (m, 15H), 2.46-2.88 (m, 2H), 3.06-3.89 (m, 2H), 4.22-4.41 and 4.47-4.74 (m, 1H), 5.15 and 5.34 (s, 1H), 6.61-7.02 (m, 2H), 7.12-7.61 (m, 1H)

*IR (neat) $cm^{-1}$:

1706, 1618, 1503, 1265, 1120, 1031, 965

Reference Example 21:

p-Toluenesulfonic acid monohydrate (1.81 g) was added to a solution of 1-[1-[(2,4-difluorophenyl)[-(tetrahydro-2H-pyran-2-yl)oxy]methyl]cyclopropyl]-2-methyl-1-propanone (32.2 g) in methanol (290 ml) and the mixture was stirred at room temperature for 1.5 h.

The reaction mixture was concentrated under vacuum and the residue was dissolved in ether (600 ml), followed by washing with a saturated aqueous solution of sodium hydrogencarbonate (200 ml), water (200 ml) and brine (200 ml) and drying over anhydrous sodium sulfate.

The desiccant was filtered off and the solvent was concentrated under vacuum to give 1-[1-[(2,4-difluorophenyl)hydroxymethyl]cyclopropyl]-2-methyl-1-propanone (25.0 g; for its structure, see Fig. 2) as a yellow oil.

*NMR ($CDCl_3$) $\delta$ [ppm]:

0.69-1.29 (m, 4H), 0.97 (d, 3H, J = 5.5Hz), 1.04 (d, 3H, J = 6.6Hz), 2.16-2.56 (m, 1H), 3.95 (bs, 1H), 5.33 (bs, 1H), 6.56-7.00 (m, 2H), 7.34-7.66 (m, 1H)

IR (KBr) $cm^{-1}$:

3466, 1682, 1618, 1502, 1271, 1095, 966

Reference Example 22:

The procedure of Reference Example 21 was repeated using the compound prepared in Reference Example 15, whereby 1-[1-[(2,4-difluorophenyl)hydroxymethyl]-cyclopropyl]-1-ethanone (see Fig. 2) was obtained as acolorless oil.

*NMR ($CDCl_3$) $\delta$ [ppm]:

0.66-1.32 (m, 4H), 1.97 (s, 3H), 3.75 (d, 1H, J = 5.3Hz), 5.44 (1H, d, J = 5.3Hz), 6.60-7.08 (m, 2H), 7.38-7.68 (m, 1H)

IR (neat) $cm^{-1}$:

3469, 1682, 1502, 1427, 1140, 1049, 964

Reference Example 23:

The procedure of Example Reference 21 was repeated using the compound prepared in Reference Example 16, whereby 1-[1-[(2,4-difluorophenyl)hydroxymethyl]-cyclopropyl]-1-propane (see Fig. 2) was obtained as acolorless oil.

*NMR ($CDCl_3$) $\delta$ [ppm]:

0.59-1.38 (m, 7H), 1.98-2.37 (m, 2H), 3.82 (bs, 1H), 5.40 (bs, 1H), 6.60-6.99 (m, 2H), 7.33-7.64 (m, 1H)

IR (neat) $cm^{-1}$:

3433, 1682, 1618, 1502, 1271, 1097, 968

Reference Example 24:

The procedure of Reference Example 21 was repeated using the compound prepared in Reference Example 17, whereby 1-[1-[(2,4-difluorophenyl)hydroxymethyl]cyclopropyl]-1-butanone (see Fig. 2) was obtained as acolorless oil.
NMR ($CDCl_3$) δ [ppm]:
0.72-0.78 (m, 1H), 0.85-0.91 (m, 3H), 0.96-1.06 (m, 1H), 1.15-1.28 (m, 2H), 1.51-1.65 (m, 2H), 2.03-2.21 (m, 2H), 5.39 (s, 1H), 6.72-6.80 (m, 1H), 6.84-6.91 (m, 1H), 7.46-7.55 (m, 1H)
IR (neat) $cm^{-1}$:
3453, 1682, 1620, 1504, 1140, 1095, 966

Reference Example 25:

The procedure of Reference Example 21 was repeated using the compound prepared in Reference Example 18, whereby 1-[1-[(2,4-dichlorophenyl)hydroxymethyl]cyclopropyl]-1-propanone (see Fig. 2) was obtained as acolorless crystal.
NMR ($CDCl_3$) δ [ppm]:
0.42-0.50 (m, 1H), 0.86-0.94 (m, 1H), 1.08 (t, 3H, J=7.3Hz), 1.05-1.16 (m, 1H), 1.23-1.31 (m, 1H), 2.17-2.37 (m, 2H), 3.42 (bs, 1H), 5.87 (s, 1H), 7.24-7.28 (m, 1H), 7.35 (d, 1H, J=2,3Hz), 7.47 (d, 1H, J=8.3Hz)
IR (KBr) $cm^{-1}$:
3439, 1670, 1460, 1369, 1066, 1038, 970 m.p.: 83.3-88.8 °C

Reference Example 26:

The procedure of Reference Example 21 was repeated using the compound prepared in Reference Example 19, whereby 4-(2,4-difluorophenyl)-4-hydroxy-3,3-dimethyl-2-butanone (see Fig. 2) was obtained as acolorless crystal.
*NMR ($CDCl_3$) δ [ppm]:
1.06 (s, 3H), 1.14 (s, 3H), 2.23 (s, 3H), 3.29 (bs, 1H), 5.30 (s, 1H), 6.63-7.65 (m, 3H)
*IR (KBr) $cm^{-1}$:
3465, 1691, 1609, 1500, 1266, 967
m.p.: 55.8-58.3 °C

Reference Example 27:

The procedure of Reference Example 21 was repeated using the compound prepared in Reference Example 20, whereby 1-(2,4-difluorophenyl)-1-hydroxy-2,2-dimethyl-3-pentanone (see Fig. 2) was obtained as a white needle.
*NMR ($CDCl_3$) δ [ppm]:
0.95-1.19 (m, 3H), 1.06 (s, 3H), 1.14 (s. 3H), 2.41-2.72 (m, 2H), 3.43 (d, 1H, J=4.0Hz), 5.28 (d, 1H, J=4.0Hz), 6.61-7.02 (m, 2H), 7.28-7.60 (m, 1H)
*IR (KBr) $cm^{-1}$:
3440, 1699, 1619, 1507, 1142, 1093, 967
m.p.: 59.3-60.4 °C

Reference Example 28:

n-Butyl lithium (182 ml; sol. in 1.6 M hexane) was added dropwise to a solution of diisopropylamine (40.8 ml) in ether (700 ml) at -60 °C over 20 min. The reaction mixture was stirred at the same temperature for 30 min, then at room temperature for 40 min. A solution of dicyclopropyl-methanone (58.3 g) in ether (450 ml) was added dropwise to the reaction mixture at room temperature over 20 min and the mixture was stirred at the same temperature for 1.5 h. A solution of 2,4-difluorobenzaldehyde (37.6 g) in ether (870 ml) was added dropwise to the reaction mixture at room temperature over 20 min, followed by stirring at the same temperature for 30 min.

The reaction mixture was poured into a mixture of ice and 1 N HCl and organic layer was separated, followed by further extraction with ether (500 ml). The organic layers were combined, washed with water

(500 ml) and brine (500 ml) and dried over anhydrous sodium sulfate.

The desiccant was filtered off and the solvent was concentrated under vacuum; thereafter, the residue was purified by silica gel column chromatography (eluant: hexane/ethyl acetate = 19/1 - 6/1). The fractions containing the end product were collected and concentrated to dryness under vacuum, to give cyclopropyl [1-[(2,4-difluorophenyl)hydroxymethyl]cyclopropyl]-methanone (19.7 g; see Fig. 2) was obtained as acolorless oil.

NMR ($CDCl_3$) $\delta$ [ppm]:

0.75-1.12 (m, 6H), 1.32-1.60 (m, 3H), 3.93 (d, 1H, J=5.3Hz), 5.45 (d, 1H, J=5.3Hz), 6.71-6.92 (m, 2H), 7.45-7.56 (m, 1H)

IR (KBr) $cm^{-1}$:

3444, 1664, 1618, 1502, 1402, 1095, 964

Reference Example 29:

The procedure of Reference Example 28 was repeated using t-butyl cyclopropylmethanone in place of dicyclopropylmethanone, whereby 1-[1-[(2,4-difluorophenyl)hydroxymethyl]cyclopropyl]-2,2-dimethyl-1-propanone (see Fig. 2) was obtained as ayellow oil.

*NMR ($CDCl_3$) $\delta$ [ppm]:

0.73-1.54 (m, 4H), 1.03 (s, 9H), 4.11 (d, 1H, J=6.3Hz), 4.94 (d, 1H, J=6.3Hz), 6.61-7.01 (m, 2H), 7.23-7.58 (m, 1H)

IR (KBr) $cm^{-1}$:

3432, 1684, 1616, 1502, 1273, 1095, 1059, 966

Reference Example 30:

Synthesis of 1-[1-[(2,4-difluorophenyl)(methoxymethoxy)-methyl]cyclopropyl]-3-methyl-1-butanone

a) Synthesis of Ethyl 1-[(2,4-difluorophenyl)(methoxymethoxy)methyl]cyclopropanecarboxylate

A solution of Ethyl 1-[(2,4-difluorophenyl)hydroxymethyl]-cyclopropanecarboxylate (23.7 g) in tetrahydrofuran (200 ml) was added dropwise to 60% sodium hydride (4.37 g) under an argon atmosphere at room temperature over 15 min and the mixture was heated under reflux for 1 h.

The reaction mixture was cooled to room temperature and a solution of chloromethyl methyl ether (7.45 g) in tetrahydrofuran (40 ml) was added dropwise over 20 min, followed by heating under reflux for 1.5 h. The reaction mixture was poured into ice water and extraction was carried out with ether (200 ml × 3). The organic layers were combined, washed with brine (200 ml) and dried over anhydrous sodium sulfate.

The desiccant was filtered off and the solvent was concentrated under vacuum and the residue was purified by silica gel column chromatography (eluant: ethyl acetate/hexane = 1/10).

The fractions containing the end product were collected and concentrated under vacuum to give Ethyl 1-[(2,4-difluorophenyl)(methoxymethoxy)methyl]cyclopropanecarboxylate (13.7 g; see Fig. 2) as a yellow oil.

NMR ($CDCl_3$) $\delta$ [ppm]:

0.71-0.76 (m, 1H), 0.96-1.08 (m, 1H), 1.17-1.27 (m, 5H), 3.31 (s, 3H), 4.09 (q, 2H, J=7.3Hz), 4.55 (d, 1H, J=6.6Hz), 4.65 (d, 1H, J=6.6Hz), 5.57 (s, 1H), 6.73-6.89 (m, 2H), 7.41-7.50 (m, 1H)

IR (KBr) $cm^{-1}$:

1726, 1504, 1151, 1142, 1039, 966

b) Synthesis of 1-[(2,4-difluorophenyl)(methoxymethoxy)methyl]cyclopropanemethanol

Diisobutyl aluminum hydride (32 ml; sol. in 1.5 M hexane) was added dropwise to a solution of Ethyl 1-[(2,4-difluorophenyl)(methoxymethoxy)methyl]cyclopropanecarboxylate (13.7 g) in toluene (125 ml) under an argon atmosphere at -78°C over 5 min and the mixture was stirred at the same temperature for 30 min.

To the reaction mixture, 1 N HCl (60 ml) was added dropwise at -78°C over 20 min and after heating to room temperature, the mixture was stirred overnight. The reaction mixture was purified by extraction with ether. After further extraction with ether (100 ml), the organic layers were combined, washed with 1 N HCl (100 ml × 2), water (100 ml) and brine (100 ml), and dried over anhydrous sodium sulfate.

The desiccant was filtered off and the solvent was concentrated to dryness under vacuum to give 1-[-(2,4-difluorophenyl)(methoxymethoxy)methyl]cyclopropanemethanol (12.6 g; see Fig. 2) as a yellow oil.

NMR ($CDCl_3$) $\delta$ [ppm]:

0.36-0.76 (m, 4H), 2.58 (bs, 1H), 3.13 (d,1H, J=11.5Hz), 3.41 (s, 3H), 3.81 (d, 1H, J=11.6Hz), 4.48 (d, 1H, J=6.6Hz), 4.60 (d, 1H, J=6.9Hz), 4.86 (s, 1H), 6.74-6.87 (m, 1H), 6.88-6.95 (m, 1H), 7.37-7.53 (m, 1H)
IR (KBr) $cm^{-1}$:
3390, 1618, 1502, 1099, 1090, 1041, 964

c) Synthesis of 1-[(2,4-difluorophenyl)(methoxymethoxy)methyl]cyclopropanecarboxyaldehyde

A solution of dimethyl sulfoxide (12.3 ml) in dichloromethane (20 ml) was added dropwise to a solution of oxalyl chloride (9.57 g) in dichloromethane (90 ml) under a nitrogen atmosphere at -70°C over 20 min and the mixture was stirred at the same temperature for 25 min.

A solution of 1-[(2,4-difluorophenyl)(methoxymethoxy)methyl]cyclopropanemethanol (12.6 g) in dichloromethane (300 ml) was added dropwise to the stirred reaction mixture at the same temperature over 30 min, followed by stirring at the same temperature for 20 min. Thereafter, the temperature was raised to -30°C and stirring was continued for 80 min; subsequently, triethylamine (17.7 g) was added dropwise at the same temperature over 10 min, followed by stirring at the same temperature for 30 min.

A saturated aqueous solution of sodium hydrogencarbonate (60 ml) was added to the reaction mixture at -30°C. The aqueous layer was extracted with dichloromethane (50 ml) and the organic layers were combined, washed with water (100 ml) and brine (100 ml) and dried over anhydrous sodium sulfate.

The desiccant was filtered off and the solvent was concentrated under vacuum and the residue was purified by silica gel column chromatography (eluant: ethyl acetate/hexane = 1/20). The fractions containing the end product were collected and concentrated under vacuum to give 1-[(2,4-difluorophenyl)-(methoxymethoxy)methyl]cyclopropanecarboxyaldehyde (7.35 g; see Fig. 2) as a pale yellow oil.
NMR ($CDCl_3$) δ [ppm]:
0.78-0.90 (m, 1H), 1.13-1.25 (m, 3H), 3.38 (s. 3H), 4.58 (d, 1H, J=6.9Hz), 4.68 (d, 1H, J=6.9Hz), 5.49 (s, 1H), 6.74-6.93 (m, 2H), 7.41-7.50 (m, 1H), 9.20 (s, 1H)
IR (KBr) $cm^{-1}$:
1716, 1618, 1502, 1151, 1140, 1090, 1041, 964

d) Synthesis of 1-[1-[(2,4-difluorophenyl)(methoxymethoxy)methyl]cyclopropyl]-3-methyl-1-butanol

A solution of isobutyl magnesium bromide [as prepared from magnesium (1.98 g) and isobutyl bromide (8.01 ml) in ether (95 ml)] was added dropwise to a solution of 1-[(2,4-difluorophenyl)(methoxymethoxy)-methyl]cyclopropanecarboxyaldehyde (9.50 g) in ether (190 ml) over 30 min and the mixture was stirred at the same temperature for 10 min, then at room temperature for 1 h.

The reaction mixture was poured into crushed ice (300 g), adjusted to a pH of 2 - 3 with dilute sulfuric acid, and organic layer was separated. Following further extraction with ether (100 ml × 2), the organic layers were combined, washed with water (100 ml), a saturated aqueous solution of sodium hydrogencarbonate (100 ml) and brine (100 ml), and dried over anhydrous sodium sulfate.

The desiccant was filtered off and the solvent was concentrated under vacuum and the residue was purified by silica gel column chromatography (eluant: ethyl acetate/hexane = 1/8). The fractions containing the end product were collected and concentrated under vacuum to give 1-[1-[(2,4-difluorophenyl)-(methoxymethoxy)methyl]cyclopropyl]-3-methyl-1-butanol (6.84 g; see Fig. 2) as a colorless oil.
NMR ($CDCl_3$) δ [ppm]:
0.06-0.22 and 0.42-0.65 (m, 4H), 0.85-0.95 (m, 6H), 1.17-1.40 and 1.52-2.00 (m, 3H), 3.31-3.38 and 3.66-3.73 (m, 1H), 3.40 and 3.44 (s, 3H), 4.46-4.59 (m, 2H), 5.22 and 5.33 (s, 1H), 6.74-6.92 (m, 2H), 7.31-7.44 (m, 1H)
IR (KBr) $cm^{-1}$:
1618, 1502, 1271, 1151, 1138, 1090, 1036, 964

e) Synthesis of 1-[1-[(2,4-difluorophenyl)(methoxymethoxy)methyl]cyclopropyl]-3-methyl-1-butanone

A solution of dimethyl sulfoxide (5.97 ml) in dichloromethane (11 ml) was added to a solution of oxalyl chloride (4.66 g) in dichloromethane (43 ml) under a nitrogen atmosphere at -70°C over 20 min, and the mixture was stirred at the same temperature for 30 min.

A solution of 1-[1-[(2,4-difluorophenyl)(methoxymethoxy)methyl]cyclopropyl]-3-methyl-1-butanol (7.00 g) in dichloromethane (181 ml) was added dropwise to the stirred reaction mixture at the same temperature over 20 min; the mixture was stirred at the same temperature for 10 min and then the temperature was raised to between -15 and -10°C, followed by stirring at the same temperature for 30 min. Thereafter,

triethylamine (8.63 g) was added dropwise at the same temperature for 10 min, followed by stirring at that temperature for 30 min. To the stirred reaction mixture, a saturated aqueous solution of sodium hydrogencarbonate (60 ml) was added at -20 °C. The aqueous layer was extracted with dichloromethane (50 ml) and the organic layers were combined, washed with water (100 ml) and brine (100 ml), and dried over anhydrous sodium sulfate.

The desiccant was filtered off and the solvent was concentrated under vacuum and the residue was purified by silica gel column chromatography (eluant: ethyl acetate/hexane = 1/20). The fractions containing the end product were collected and concentrated under vacuum to give 1-[1-[(2,4-difluorophenyl)-(methoxymethoxy)methyl]cyclopropyl]-3-methyl-1-butanone (5.61 g; see Fig. 2) as a colorless oil.

NMR ($CDCl_3$) δ [ppm]:

0.61-0.69 (m, 1H), 0.86 (d, 6H, J=6.3Hz), 0.95-1.27 (m, 3H), 2.10-2.23 (m, 3H), 3.38 (s, 3H), 4.54 (d, 1H, J=6.9Hz), 4.63 (d, 1H, J=6.9Hz), 5.78 (s, 1H), 6.74-6.89 (m, 2H), 7.34-7.43 (m, 1H)

IR (KBr) $cm^{-1}$:

1691, 1618, 1502, 1271, 1153, 1090, 1032, 964

f) Synthesis of 1-[1-[(2,4-difluorophenyl)hydroxymethyl]-cyclopropyl]-3-methyl-1-butanone

Water (32 ml) and 6 N HCl (80 ml) were added to a solution of 1-[1-[(2,4-difluorophenyl)-(methoxymethoxy)methyl]cyclopropyl]-3-methyl-1-butanone (5.34 g) in tetrahydrofuran (32 ml) and the mixture was stirred under heating at 50 - 60 °C for 4 h.

The reaction mixture was cooled to room temperature and, after addition of brine (150 ml), extraction was carried out with ether (150 ml × 2). The organic layers were combined, washed with a saturated aqueous solution of sodium hydrogencarbonate (100 ml), water (100 ml) and brine (100 ml), and dried over anhydrous sodium sulfate.

The desiccant was filtered off and the solvent was concentrated under vacuum and the residue was purified by silica gel column chromatography (eluant: ethyl acetate/hexane = 1/19). The fractions containing the end product were collected and concentrated under vacuum to give 1-[1-[(2,4-difluorophenyl)hydroxymethyl]cyclopropyl]-3-methyl-1-butanone (4.23 g) as a colorless oil.

NMR ($CDCl_3$) δ [ppm]:

0.68-0.81 (m, 1H), 0.86 (d, 3H, J=6.6Hz), 0.88 (d, 3H, J=6.6Hz), 0.96-1.06 (m, 1H), 1.99 (d, 1H, J=5.9Hz), 1.99 (d, 1H, J=7.6Hz), 2.05-2.26 (m, 1H), 3.91 (d, 1H, J=5.6Hz), 5.38 (d, 1H, J=5.6Hz), 6.71-6.91 (m, 2H), 7.46-7.55 (m, 1H)

IR (KBr) $cm^{-1}$:

3433, 1678, 1618, 1502, 1138, 1095, 964

Reference Example 31:

a) Synthesis of (Z)-1-[1-[(2,4-difluorophenyl)-hydroxymethyl]cyclopropyl]-2-methyl-1-propanone oxime

A hydroxylamine hydrochloride (28.2 g) was added to a solution of 1-[1-[(2,4-difluorophenyl)-hydroxymethyl]-cyclopropyl]-2-methyl-1-propanone (20.7 g) in pyridine (200 ml) and the mixture was heated under reflux for 4 h.

The reaction mixture was concentrated under vacuum and the residue was partitioned between ethyl acetate (500 ml) and 2 N HCl (300 ml). Following further extraction with ethyl acetate (100 ml), the organic layers were combined, washed with 1 N HCl (100 ml), water (100 ml) and brine (100 ml), and dried over anhydrous sodium sulfate.

The desiccant was filtered off and the solvent was concentrated under vacuum and the residue was purified by silica gel column chromatography (eluant: hexane/ethyl acetate = 10/1 - 7/1). The fractions containing the end product were collected and concentrated under vacuum to give (Z)-1-[1-[(2,4-difluorophenyl)-hydroxymethyl]cyclopropyl]-2-methyl-1-propanone oxime (8.76 g; see Fig. 2) as a colorless crystal.

*NMR (DMSO-$d_6$) δ [ppm]:

0.48-1.27 (m, 4H), 0.69 (d, 3H, J=6.9Hz), 0.86 (d, 3H, J=6.6Hz), 1.91-2.32 (m, 1H), 5.27 (d, 1H, J=5.3Hz), 5.42 (d, 1H, J=5.3Hz), 6.89-7.23 (m, 2H), 7.34-7.66 (m, 1H), 10.54 (s, 1H)

IR (KBr) $cm^{-1}$:

3292, 1616, 1502, 1429, 1273, 1142, 1099, 966

m.p.: 95.8-103.6 °C

b) Synthesis of (E)-1-[1-[(2,4-difluorophenyl)-hydroxymethyl]cyclopropyl]-2-methyl-1-propanone oxime

The purification by silica gel column chromatography in step a) was continued under the same conditions, except that the density gradient of the eluant (hexane/ethyl acetate) was varied from 7/1 to 5/1. The fractions containing the end product were collected and concentrated under vacuum to give (E)-1-[1-[-(2,4-difluorophenyl)-hydroxymethyl]cyclopropyl]-2-methyl-1-propanone oxime (9.23 g; see Fig. 2) as a colorless crystal.

*NMR (DMSO-$d_6$) $\delta$ [ppm]:

0.57-0.88 (m, 4H), 1.14 (d, 6H, J = 7.3Hz), 2.65-3.09 (m, 1H), 5.11 (d, 1H, J = 5.6Hz), 5.39 (d, 1H, J = 5.6Hz), 6.88-7.25 (m, 2H), 7.35-7.71 (m, 1H),10.22 (s, 1H)

IR (KBr) $cm^{-1}$:

3309, 1620, 1502, 1273, 1095, 1051, 966

m.p.: 90.4-95.3 °C

Thus, the reactions carried out in the Reference Examples or similar reactions, namely, reactions using ketone and hydroxylamine or alkoxylamine, allow both (E) and (Z) forms to be obtained as separate compounds. In the present invention, distinction between the (E) and (Z) forms is made by the increase or decrease of chemical shifts in NMR for protons in the alkyl group ($R^3$) in each general formula that are in $\alpha$-position with respect to oxime, and the isomer at the lower magnetic field is designated the (E) form whereas the one at the higher field as the (Z) form. Unless otherwise specified, the physical data listed in the following reference examples and examples are those for the (E) form as obtained separately.

Reference Examples 32 - 46:

The procedure of Reference Example 31 was repeated to prepare the compounds shown in Fig. 3 and their physical data are listed in Table 5 (t-Bu, t-butyl group; i-Bu, isobutyl group; i-Pr, isopropyl group; n-Pr, n-propyl group; Et, ethyl group).

Table 5-1

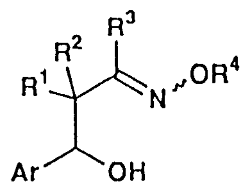

| REF. EX. | Ar | R¹ R² | | $R^3$ | $R^4$ | NMR ppm | IR $cm^{-1}$ | M.P. °C |
|---|---|---|---|---|---|---|---|---|
| 32 | 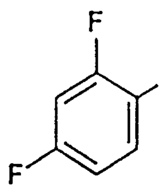 | 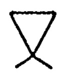 | | Et | H | *DMSO-d6: 0.48-1.18(m, 7H), 2.01-2.49(m, 2H), 5.09(d, 1H, J=5.3Hz), 5.39(d, 1H, J=5.3Hz), 6.78-7.26(m, 2H), 7.29-7.65(m, 1H), 10.21(s,1H) | KBr: 3338, 1620, 1502, 1429, 1273, 1140, 1095, 966 | OIL |
| 33 | 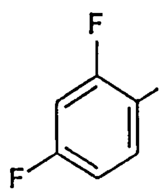 | 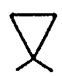 | | n-Pr | H | $CDCl_3$: 0.59-0.68(m, 1H), 0.73-0.91(m, 6H), 1.29-1.53(m, 2H), 1.95-2.11(m, 2H), 3.63(bs, 2H), 5.10(s, 1H), 6.72-6.80(m, 1H), 6.83-6.90(m, 1H), 7.44-7.53(m, 1H) | KBr: 3272, 1620, 1502, 1095, 1049, 968, 966 | 71.3-74.5 |

Table 5-2

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 34 | 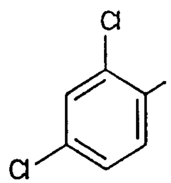 | 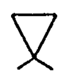 | | Et | H | $CDCl_3$: 0.30-0.36(m, 1H), 0.69-0.95(m, 3H), 1.07-1.13(m, 3H), 1.95-2.08(m, 1H), 2.27-2.40(m, 1H), 5.55(s, 1H), 7.23-7.27(m, 1H), 7.35(d, 1H, J=2.0Hz), 7.50(d, 1H, J=8.3Hz) | KBr: 3284, 1589, 1470, 1065, 1032, 947 | 100.0-103.5 |
| 35 | 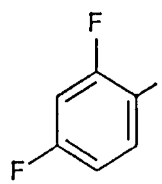 | 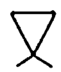 | | 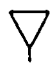 | H | *DMSO-d6: 0.25-1.13(m, 8H), 1.49-1.85(m, 1H), 5.18(d, 1H, J=5.3Hz), 5.41(d, 1H, J=5.3Hz), 6.88-7.71(m 3H), 10.31(s, 1H) | KBr: 3338, 1620, 1502, 1095, 1043, 964 | 93.5-99.4 |
| 36 | 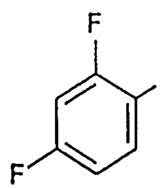 | 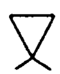 | | t-Bu | H | *DMSO-d6: 0.45-1.07(m, 4H), 1.30(s, 9H), 5.21(d, 1H, J=5.3Hz), 5.35(d, 1H, J=5.3Hz), 6.83-7.22(m, 2H), 7.27-7.67(m 1H), 10.36(s, 1H) | KBr: 3365, 1620, 1502, 1142, 1093, 966 | 108.4-111.8 |
| 37 | 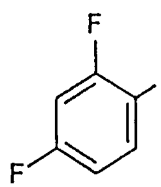 | 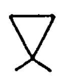 | | i-Bu | H | DMSO-d6: 0.65-0.87(m, 10H), 1.97-2.18(m, 2H), 2.22-2.33(m, 1H), 5.16(d, 1H, J=5.6Hz), 5.44(d, 1H, J=5.6Hz), 7.00-7.16(m, 2H), 7.45-7.54(m 1H), 10.24(s, 1H) | KBr: 3369, 1620, 1502, 1275, 1140, 1095, 1039, 966 | 46.4-54.2 |

Table 5-3

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 38 | 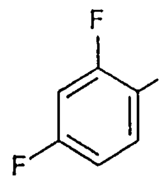 | $CH_3$ | $CH_3$ | $CH_3$ | H | *$CDCl_3$: 1.00(d, 3H, J=2.6Hz), 1.07(s, 3H), 1.92(s, 3H), 4.26(bs, 2H), 5.27(s, 1H), 6.63-6.97(m, 2H), 7.26-7.53(m, 1H) | KBr: 3307, 1620, 1502, 1427, 1092, 1043, 968 | 111.3-113.2 |
| 39 | 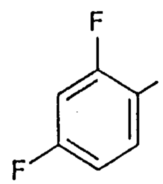 | 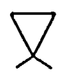 | | i-Pr | $CH_3$ | *$CDCl_3$: 0.39-0.93(m, 4H), 1.07(d, 3H, J=7.3Hz), 1.12(d, 3H, J=7.3Hz), 2.43-2.81(m, 1H), 3.85(s 3H), 3.87(s 1H), 5.09(s, 1H), 6.57-7.00(m, 2H), 7.34-7.65(m, 1H) | KBr: 3363, 1618, 1500, 1271, 1057, 1030, 887, 847 | 45.5-46.8 |
| 40 | 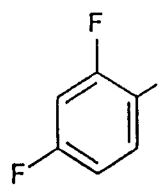 | 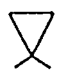 | | $CH_3$ | $CH_3$ | *$CDCl_3$: 0.48-2.10(m, 4H), 1.60(s, 3H), 3.88(s, 3H), 5.14(s, 1H), 6.66-7.02(m, 2H), 7.12-7.62(m, 1H) | neat: 3433, 1620, 1503, 1095, 1053, 966 | OIL |

Table 5-4

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 41 | 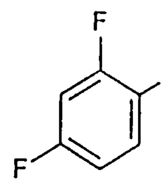 | 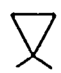 | | n-Pr | $CH_3$ | $CDCl_3$: 0.60-0.70(m, 1H), 0.73-0.87(m, 3H), 0.84(t, 3H, J=7.3Hz), 1.21-1.48(m, 2H), 1.85-2.17(m, 2H), 3.87(s, 3H), 5.05(s, 1H), 6.71-6.79(m, 1H), 6.83-6.90(m, 1H), 7.46-7.55(m, 1H) | neat: 3429, 1620, 1606, 1504, 1138, 1053, 966 | OIL |
| 42 | 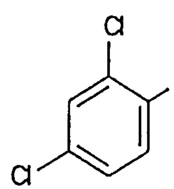 | 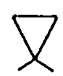 | | Et | $CH_3$ | $CDCl_3$: 0.29-0.92(m, 4H), 1.04(t, 3H, J=7.6Hz), 1.87-1.95(m, 1H), 2.19-2.29(m, 1H), 3.89(s, 3H), 4.00(s, 1H), 5.54(s, 1H), 7.23-7.27(m, 1H), 7.34(d, 1H, J=2.0Hz), 7.51(d, 1H, J=8.6Hz) | neat: 3429, 1589, 1470, 1381, 1053, 887 | OIL |
| 43 | 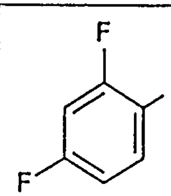 | 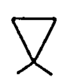 | | 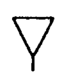 | $CH_3$ | *$CDCl_3$: 0.31-1.10(m, 8H), 1.41-1.78(m, 1H), 3.16(bs, 1H), 3.86(s, 3H), 5.00(s, 1H), 6.64-7.01(m, 2H), 7.31-7.63(m, 1H) | KBr: 3352, 1618, 1500, 1269, 1055, 1032, 887 | OIL |

Table 5-5

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 44 | 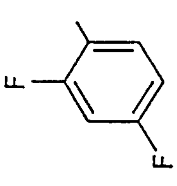 | 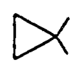 | | t-Bu | $CH_3$ | $CDCl_3$: 0.21-0.84(m, 4H), 1.33(s, 9H), 3.07(d, 1H, J=3.0Hz), 3.85(s, 3H), 5.29(bs, 1H), 6.55-6.98(m, 2H), 7.32-7.66(m, 1H) | KBr: 3448, 1620, 1604, 1502, 1093, 1039, 966 | OIL |
| 45 | 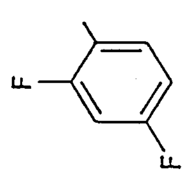 | 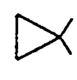 | | iBu | $CH_3$ | $CDCl_3$: 0.67-0.96(m, 10H), 1.75-2.00(m, 3H), 3.86(s, 3H), 4.94(s, 1H), 6.70-6.90(m, 2H), 7.49-7.58(m, 1H) | KBr: 3415, 1618, 1606, 1502, 1427, 1140, 1095, 1053, 966 | OIL |
| 46 | 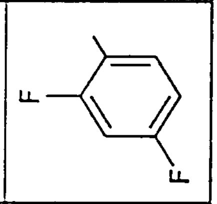 | $CH_3$ | $CH_3$ | $CH_3$ | $CH_3$ | $CDCl_3$: 0.97(d, 3H, J=3.0Hz), 1.05(s, 3H), 1.84(s, 3H), 3.90(s, 3H), 5.28(s, 1H), 6.60-7.68(m, 3H) | neat: 3452, 1619, 1605, 1506, 1052, 967 | OIL |

Reference Example 47:

Dimethyl sulfoxide (1.0 ml) was added to a solution of oxalyl chloride (0.54 ml) in dichloromethane (8.8 ml) under a nitrogen atmosphere at -70 ° C and the mixture was stirred for 15 min.

A solution of 1-(2,4-difluorophenyl)-1-hydroxy-2,2-dimethyl-3-pentanone (1.10 g) in dichloromethane (36 ml) was added dropwise to the reaction mixture at the same temperature over 20 min. After 10-min stirring, the mixture was warmed to -20 °C and stirred for 1 h. Triethylamine (1.98 ml) was added dropwise to the reaction mixture at -20 °C, followed by stirring at the same temperature for 35 min. A saturated aqueous solution of sodium hydrogencarbonate (10 ml) was added to the stirred reaction mixture at -20 °C. The aqueous layer was extracted with dichloromethane (20 ml × 2) and the organic layers were combined, washed with brine (50 ml) and dried over anhydrous sodium sulfate.

The desiccant was filtered off and the solvent was concentrated under vacuum and the residue was purified by silica gel column chromatography (eluant: hexane/ethyl acetate = 19/1). The fractions containing the end product were collected and concentrated under vacuum to give 1-(2,4-difluorophenyl)-2,2-dimethyl-1,3-pentanedione (1.04 g; see Fig. 4 for its structure) as a colorless oil.

*NMR ($CDCl_3$) δ [ppm]:

0.84-1.18 (m, 3H), 1.42 (s, 3H), 1.43 (s, 3H), 2.34-2.65 (m, 2H), 6.65-7.07 (m, 2H), 7.60-7.91 (m, 1H)

*IR (neat) $cm^{-1}$:

1685, 1612, 1498, 1425, 1266, 1097, 974

Reference Example 48:

The procedure of Example 47 was repeated using the compound prepared in Reference Example 23, whereby 1-[1-(2,4-difluorobenzoyl)cyclopropyl]-2-methyl-1-propanone (see Fig. 4) was obtained as acolorless oil.

*NMR ($CDCl_3$) δ [ppm]:

1.01(t, H, J = 7.3Hz), 1.58 (s, 4H), 2.34 (q, 2H, J = 7.3Hz), 6.63-7.10 (m, 2H), 7.73-8.09 (m, 1H)

IR (neat) $cm^{-1}$:

1678, 1610, 1429, 1313, 1271, 1099

Reference Example 49:

60% Sodium hydride (1.37 g) was added to a solution of 1-[1-[(2,4-difluorophenyl)hydroxymethyl]-cyclopropyl]-2-methyl-1-propane oxime (9.20 g) in dimethylformamide (150 ml) and, following warming to room temperature, the mixture was stirred for 30 min. The reaction mixture was again cooled with ice water and a solution of chloromethyl methyl ether (2.57 ml) in dimethylformamide (5 ml) was added over 10 min, followed by stirring at the same temperature for 20 min and at room temperature for 30 min.

The reaction mixture was poured into ice water, adjusted to a pH near 2 with 1 N HCl, subjected to extraction with ethyl acetate (100 ml × 2), washed with 1 N HCl (50 ml), water (50 ml) and brine (50 ml), and dried over anhydrous sodium sulfate.

The desiccant was filtered off and the solvent was concentrated under vacuum and the residue was purified by silica gel column chromatography (eluant: ethyl acetate/hexane = 1/8). The fractions containing the end product were collected and concentrated under vacuum to give 1-[1-[(2,4-difluorophenyl)-hydroxymethyl]cyclopropyl]-2-methyl-1-propanone O-(methoxymethyl)oxime (7.20 g; see Fig. 4) as a colorless oil.

*NMR ($CDCl_3$) δ [ppm]:

0.43-1.45 (m, 4H), 1.11 (d, 3H, J = 6.9Hz), 1.15 (d, 3H, J = 6.9Hz), 2.39-2.90 (m, 1H), 3.41 (s, 3H), 3.52 (s, 1H), 5.05 (d, 1H, J = 7.3Hz), 5.10 (d, 1H, J = 7.3Hz), 6.62-7.00 (m, 2H), 7.36-7.70 (m, 1H)

IR (KBr) $cm^{-1}$:

3442, 1620, 1502, 1140, 1093, 999, 966

Reference Examples 50 - 57:

The procedure of Reference Example 49 was repeated to prepare the compounds shown in Fig. 4 and their physical data are listed in Table 6 (t-Bu, t-butyl group; 1-Bu, isobutyl group; i-Pr, isopropyl group; n-Pr, n-propyl group; Et, ethyl group).

Table 6-1

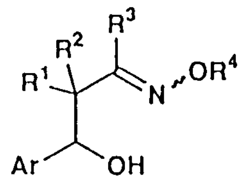

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 50 | 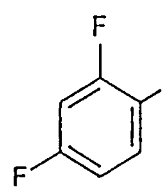 | 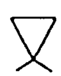 | | i-Pr | (Z) $CH_2OCH_3$ | *$CDCl_3$: 0.69-1.11(m, 4H), 0.79(d, 3H, J=6.9Hz), 1.05(d, 3H, J=6.6Hz), 1.95-2.45(m, 1H), 3.51(s, 3H), 4.49(d, 1H, J=4.6Hz), 4.81(d, 1H, J=4.6Hz), 5.19(s, 2H), 6.60-7.00(m, 2H), 7.35-7.67(m, 1H) | KBr: 3469, 1618, 1502, 1159, 1093, 1001, 966 | OIL |

Table 6-2

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 51 | 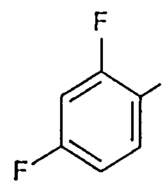 | 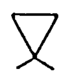 | | Et | $CH_2OCH_3$ | *$CDCl_3$: 0.42-1.38(m, 4H), 0.93(t, 3H, J=7.6Hz), 1.88-2.27(m, 2H), 3.42(s, 3H), 4.20(d, 1H, J=5.9Hz), 5.05(d, 1H, J=7.3Hz), 5.07(d, 1H, J=5.9Hz), 5.14(d, 1H, J=7.3Hz), 6.57-7.02(m, 2H), 7.32-7.71(m, 1H) | KBr: 3442, 1620, 1502, 1140, 1093, 1012, 966 | OIL |
| 52 | 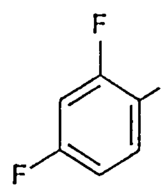 | 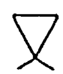 | | n-Pr | $CH_2OCH_3$ | $CDCl_3$: 0.67-0.92(m, 4H), 0.85(t, 3H, J=7.3Hz), 1.23-1.47(m, 2H), 1.59-(bs, 1H), 1.86-2.09(m, 2H), 3.41(s, 3H), 5.02(s, 1H), 5.04(d, 1H, J=7.3Hz), 5.15(d, 1H, J=7.3Hz), 6.71-6.78(m, 1H), 6.82-6.89(m, 1H), 7.50-7.59(m, 1H) | neat: 3466, 1618, 1502, 1140, 1093, 851 | OIL |

Table 6-3

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 53 | 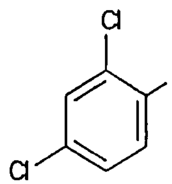 | 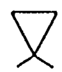 | | Et | $CH_2OCH_3$ | $CDCl_3$: 0.36-0.43(m, 1H), 0.73-0.91(m, 3H), 1.05-1.11(m, 3H), 1.93-2.06(m, 1H), 2.17-2.34(m, 1H), 3.24(bs, 1H), 3.44(s, 3H), 5.08(d, 1H, J=7.3Hz), 5.18(d, 1H, J=7.3Hz), 5.53(s, 1H), 7.23-7.26(m, 1H), 7.34(d, 1H, J=2.3Hz), 7.52(d, 1H, J=8.2Hz) | neat: 3433, 1589, 1470, 1159, 1140, 1086, 1014 | OIL |
| 54 | 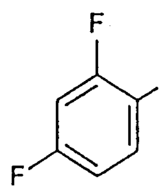 | 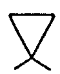 | | 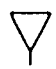 | $CH_2OCH_3$ | *$CDCl_3$: 0.40-1.13(m, 8H), 1.49-1.83(m, 1H), 3.40(s, 3H), 4.97-5.19(m, 3H), 6.59-7.01(m 2H), 7.36-7.65(m, 1H) | KBr: 3425, 1614, 1504, 1144, 1055, 1005, 964 | OIL |
| 55 | 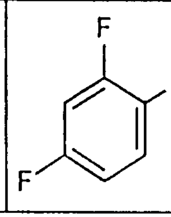 | 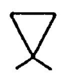 | | t-Bu | $CH_2OCH_3$ | *$CDCl_3$: 0.34-0.86(m, 4H), 1.30(s, 9H), 3.05(bs, 1H), 3.42(s, 3H), 5.11(s, 1H), 5.32(bs, 1H), 6.59-6.98(m, 2H), 7.33-7.64(m 1H) | KBr: 3450, 1618, 1502, 1157, 1095, 993, 966 | OIL |

Table 6-4

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 56 | 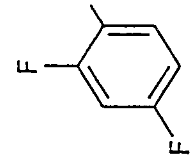 | 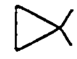 | | i-Bu | $CH_2OCH_3$ | $CDCl_3$: 0.70(d, 3H, J=6.3Hz), 0.74-1.01(m, 4H), 0.83(d, 3H, J=6.3Hz), 1.74-1.91(m, 2H), 2.01-2.09(m, 1H), 3.41(s, 3H), 4.88(s, 1H), 5.02(d, 1H, J=7.3Hz), 5.16(d, 1H, J=7.3Hz), 6.70-6.89(m, 2H), 7.54-7.64(m 1H) | KBr: 3433, 1618, 1502, 1140, 1093, 1007, 966, 849 | OIL |
| 57 | 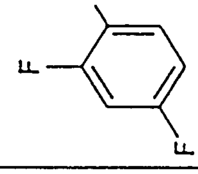 | $CH_3$ | $CH_3$ | $CH_3$ | $CH_2OCH_3$ | *$CDCl_3$: 1.01(d, 3H, J=2.6Hz), 1.07(s, 3H), 1.91(s, 3H), 3.45(s, 3H), 5.10(d, 1H, J=7.3Hz), 5.17(d, 1H, J=7.3Hz), 5.27(s, 1H), 6.63-7.00(m, 2H), 7.26-7.54(m, 1H) | neat: 3433, 1620, 1502, 1427, 1092, 1012, 966 | OIL |

Reference Example 58:

A solution of dimethyl sulfoxide (6.08 ml) in dichloromethane (44 ml) was added to a solution of oxalyl chloride (4.75 g) in dichloromethane (44 ml) under a nitrogen atmosphere at -70 °C over 10 min, and the

mixture was stirred at the same temperature for 20 min. To the stirred reaction mixture, a solution of 1-[1-[-(2,4-difluorophenyl)hydroxymethyl]cyclopropyl]-2-methyl-1-propanone O-methoxymethyl oxime (7.10 g) in dichloromethane (184 ml) was added at the same temperature over 10 min, followed by stirring at the same temperature for 20 min. Thereafter, the mixture was heated to -20 °C and stirred for an additional hour. Subsequently, triethylamine (12.1 ml) was added dropwise at -20 °C over 10 min, followed by stirring at the same temperature for 20 min.

A saturated aqueous solution of sodium hydrogencarbonate (200 ml) was added to the reaction mixture at -20 °C. The aqueous layer was extracted with dichloromethane (70 ml × 2) and the organic layers were combined, washed with brine (70 ml) and dried over anhydrous sodium sulfate. The desiccant was filtered off and the solvent was concentrated to dryness under vacuum to give (2,4-difluorophenyl)[((1-methoxymethoxyimino-2-methyl)propyl]cyclopropyl]methanone (7.62 g; see Fig. 4) as a colorless oil.

It should be noted here that the synthetic method employed in Reference Examples 58 - 74 was by Process G.

*NMR ($CDCl_3$) δ [ppm]:

0.95 (d, 6H, J = 6.9Hz), 1.32-1.65 (m, 4H), 2.60-3.10 (m, 1H), 3.36 (s, 3H), 5.05 (s, 2H), 6.70-7.04 (m, 2H), 7.71-7.99 (m, 1H)

IR (KBr) $cm^{-1}$:

1687, 1610, 1303, 1271, 1147, 1107, 995

Reference Examples 59 - 74:

The procedure of Reference Example 58 was repeated to prepare the compounds shown in Figs. 4 and 5 and their physical data are listed in Table 7 (t-Bu, t-butyl group; i-Bu, isobutyl group; i-Pr, isopropyl group; n-Pr, n-propyl group; Et, ethyl group).

Table 7-1

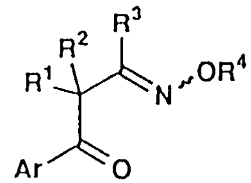

| REF. EX. | Ar | R¹ R² | R³ | R⁴ | NMR ppm | IR cm⁻¹ | M.P. °C |
|---|---|---|---|---|---|---|---|
| 59 | 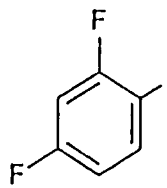 | 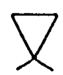 | i-Pr | (Z) $CH_2OCH_3$ | *$CDCl_3$: 1.07(d, 6H, J=6.9Hz), 1.14-1.34(m, 2H), 1.53-1.74(m, 2H), 2.36-2.72(m, 1H), 3.23(s, 3H), 4.90(s, 2H), 6.66-7.00(m, 2H), 7.38-7.68(m, 1H) | KBr: 1689, 1614, 1271, 1099, 1001, 984 | OIL |
| 60 | 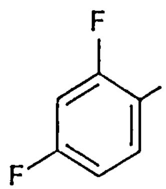 | 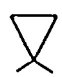 | Et | $CH_2OCH_3$ | *$CDCl_3$: 1.00(t, 3H, J=7.6Hz), 1.20-1.74(m, 4H), 2.01-2.34(m, 2H), 3.29(s, 3H), 4.99(s, 2H), 6.66-7.05(m, 2H), 7.58-7.94(m, 1H) | KBr: 1686, 1612, 1302, 1271, 1146, 1011, 995 | OIL |

Table 7-2

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 61 | 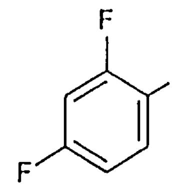 | 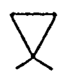 | | n-Pr | $CH_2OCH_3$ | $CDCl_3$: 0.84-0.89(m, 3H), 1.35-1.49(m, 4H), 1.52-1.57(m, 2H), 2.09-2.15(m, 2H), 3.29(s, 3H), 4.99(s, 2H), 6.79-6.94(m, 2H), 7.72-7.81(m, 1H) | neat: 1686, 1610, 1271, 1146, 1007 | OIL |
| 62 | 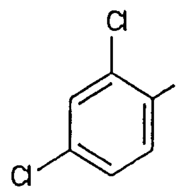 | 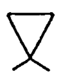 | | Et | $CH_2OCH_3$ | $CDCl_3$: 1.01(t, 3H, J=7.6Hz), 1.49-1.67(m, 4H), 2.25(q, 2H, J=7.6Hz), 3.33(S, 3H), 5.03(S, 2H), 7.19-7.27(m, 1H), 7.43(d, 1H, J=2.0Hz), 7.49(d, 1H, J=8.3Hz) | neat: 1689, 1583, 1294, 1011, 987 | OIL |
| 63 | 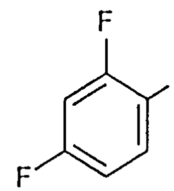 | 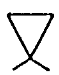 | | 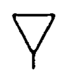 | $CH_2OCH_3$ | *$CDCl_3$: 0.60-1.02(m, 4H), 1.19-1.80(m, 5H), 3.36(s, 3H), 5.09(s, 2H), 6.71-7.01(m 2H), 7.70-7.93(m, 1H) | KBr: 1687, 1610, 1302, 1271, 1153, 1105, 995 | OIL |
| 64 | 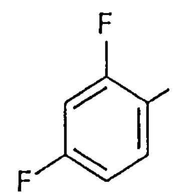 | 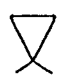 | | t-Bu | $CH_2OCH_3$ | *$CDCl_3$: 1.05(s, 9H), 1.19-1.76(m, 4H), 3.39(s, 3H), 5.08(s, 2H), 6.70-6.98(m, 2H), 7.71-8.00(m 1H) | KBr: 1687, 1610, 1302, 1271, 1159, 1107, 993, | OIL |

Table 7-3

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 65 | 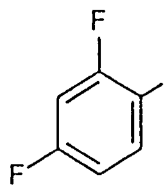 | 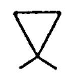 | | i-Bu | $CH_2OCH_3$ | $CDCl_3$: 0.84(d, 6H, J=6.3Hz), 1.43-1.61(m, 4H), 1.87-2.04(m, 1H), 2.09(d, 2H, J=6.8Hz), 3.30(s, 3H), 5.00(s, 2H), 6.79-6.93(m, 2H), 7.73-7.82(m, 1H) | KBr: 1686, 1612, 1302, 1271, 1147, 1103, 1007, 997, 854 | OIL |
| 66 | 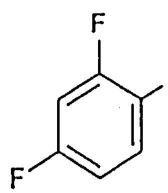 | $CH_3$ | $CH_3$ | $CH_3$ | $CH_2OCH_3$ | *$CDCl_3$: 1.42(s, 6H), 1.87(s, 3H), 3.35(s, 3H), 5.06(s, 2H), 6.73-6.99(m, 2H), 7.53-7.79(m, 1H) | neat: 1699, 1610, 1157, 1097, 1007, 980 | OIL |
| 67 | 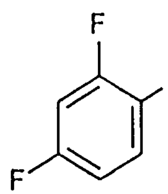 | 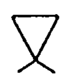 | | i-Pr | $CH_3$ | *$CDCl_3$: 0.90(d, 6H, J=6.9Hz), 1.31-1.61(m, 4H), 2.80(sep, 1H, J=6.9Hz), 3.79(s 3H), 6.68-7.04(m, 2H), 7.65-7.97(m, 1H) | KBr: 2968, 1686, 1610, 1271, 1038 | OIL |
| 68 | 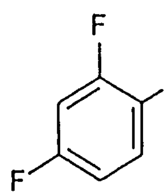 | 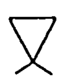 | | $CH_3$ | $CH_3$ | *$CDCl_3$: 1.36-1.42(m, 2H), 1.49-1.55(m, 2H), 1.58(s, 3H), 3.71(s, 3H), 6.72-6.96(m, 2H), 7.56-7.86(m, 1H) | neat: 1682, 1610, 1302, 1271, 1053, 991 | OIL |

Table 7-4

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 69 | 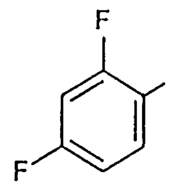 | 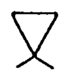 | | n-Pr | $CH_3$ | $CDCl_3$: 0.84(t, 3H, J=7.3Hz), 1.32-1.47(m, 4H), 1.51-1.58(m, 2H), 2.03-2.09(m, 2H), 3.73(s, 3H), 6.79-6.94(m, 2H), 7.69-7.78(m, 1H) | neat: 1684, 1610, 1271, 1101, 1053, 995 | OIL |
| 70 | 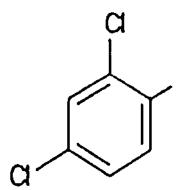 | 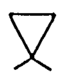 | | Et | $CH_3$ | $CDCl_3$: 0.97(t, 3H, J=7.6Hz), 1.49(t, 2H, J=3.0Hz), 1.54-1.58(m, 2H), 2.20(q, 2H, J=7.6Hz), 3.78(s, 3H), 7.26(dd, 1H, J=2.0, 8.6Hz), 7.42(d, 1H, J=2.0Hz), 7.46(d, 1H, J=8.3Hz) | neat: 1689, 1585, 1294, 1051, 991 | OIL |
| 71 | 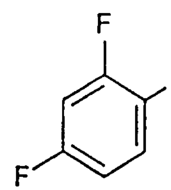 | 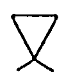 | | 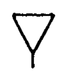 | $CH_3$ | *$CDCl_3$: 0.43-1.01(m, 4H), 1.13-1.83(m, 5H), 3.85(s, 3H), 6.71-7.02(m, 2H), 7.47-7.90(m, 1H) | KBr: 1686, 1610, 1502, 1302, 1271, 1061, 1038 | OIL |
| 72 | 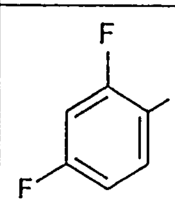 | 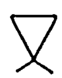 | | t-Bu | $CH_3$ | *$CDCl_3$: 1.00(s, 9H), 1.19-1.71(m, 4H), 3.82(s, 3H), 6.70-7.01(m, 2H), 7.62-7.96(m, 1H) | KBr: 1687, 1610, 1300, 1271, 1107, 1039 | OIL |

Table 7-5

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 73 | 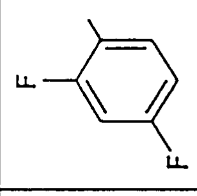 | 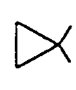 | | i-Bu | $CH_3$ | $CDCl_3$: 0.80(d, 6H, J=6.3Hz), 1.40-1.55(m, 4H), 1.81-1.98(m, 1H), 2.02(d, 2H, J=6.8Hz), 3.71(s, 3H), 6.80-6.94(m, 2H), 7.71-7.79(m, 1H) | KBr: 1687, 1610, 1302, 1271, 1053 | OIL |
| 74 | 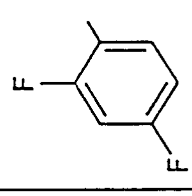 | $CH_3$ | $CH_3$ | $CH_3$ | $CH_3$ | *$CDCl_3$: 1.40(s, 6H), 1.80(s, 3H), 3.80(s, 3H), 6.60-7.02(m, 2H), 7.44-7.77(m, 1H) | *neat: 1702, 1612, 1259, 1049, 982 | OIL |

Reference Example 75:

Methylhydroxylamine hydrochloride(1.08 g) and pyridine (1.40 ml) were added to a solution of 1-(2,4-difluorophenyl)-2,2-dimethyl-1,3-pentanedione (3.46 g) in ethanol (120 ml) and the mixture was heated

under reflux for 4 h. The reaction mixture was concentrated under vacuum and the residue was purified by silica gel column chromatography (eluant: hexane/ether = 180/1). The fractions containing the end product were concentrated under vacuum to give 1-(2,4-difluorophenyl)-3-methoxyimino-2,2-dimethyl-1-pentanone (1.84 g; see Fig. 5) as a colorless oil.

It should be noted here that the synthesis procedure employed in Reference Examples 75 and 76 was by Process J.

*NMR ($CDCl_3$) δ [ppm]:

1.08 (t, 3H, J=7.6Hz), 1.41 (s, 6H), 2.05-2.42 (m, 2H), 3.79 (s, 3H), 6.68-7.00 (m, 2H), 7.46-7.75 (m, 1H)

*IR (neat) $cm^{-1}$:

1701, 1611, 1501, 1259, 1099, 1052, 981

Reference Example 76:

The procedure of Refernce Example 75 was repeated using the compound prepared in Reference Example 48, whereby 1-[1-(2,4-difluorobenzoyl)cyclopropyl]-1-propanone O-methyl oxime (see Fig. 5) was obtained as acolorless oil.

*NMR ($CDCl_3$) δ [ppm]:

0.78-1.08 (m, 3H), 1.32-1.57 (m, 4H), 1.94-2.32 (m, 2H), 3.73 (s, 3H), 6.70-7.04 (m, 2H), 7.59-7.90 (m, 1H)

IR (neat) $cm^{-1}$:

1684, 1610, 1425, 1302, 1271, 1101, 1051, 997

Reference Example 77:

60% Sodium hydride (2.16 g) was washed with n-hexane; following the addition of dimethyl sulfoxide (83.0 ml) under a nitrogen atmosphere, the mixture was stirred at 60 - 70°C for 1 h. The reaction mixture was allowed to cool at room temperature and, following the addition of trimethyl sulfoxonium iodide (19.8 g), the mixture was stirred for 2 h; thereafter, a solution of (2,4-difluorophenyl)[[(1-methoxymethoxyimino-2-methyl)propyl]cyclopropyl]methanone (7.56 g) in dimethyl sulfoxide (55.0 ml) was added at 20 - 25°C and the mixture was stirred at room temperature for 24 h.

The reaction mixture was poured over ice and extractions were carried out with ether (150 ml × 3). The organic layers were combined, washed with water (80 ml) and brine (80 ml), and dried over anhydrous magnesium sulfate. The desiccant was filtered off and the solvent was concentrated under vacuum and the residue was purified by silica gel column chromatography (eluant: 5% ethyl acetate/hexane). The fractions containing the end product were collected and concentrated to dryness under vacuum to give 1-[1-[2-(2,4-difluorophenyl)oxiran-2-yl]cyclopropyl]-2-methylpropanone O-(methoxymethyl)oxime (4.82 g; see Fig. 5) as a colorless oil of a mixture containing small amounts of starting materials.

*NMR ($CDCl_3$) δ [ppm]:

0.64-1.03 (m, 4H), 1.15 (d, 6H, J=6.9Hz), 2.55-3.09 (m, 1H), 2.96 (d, 1H, J=5.3Hz), 3.01 (d, 1H, J=5.3Hz), 3.25 (s, 3H), 4.93 (s, 2H), 6.61-7.00 (m, 2H), 7.33-7.63 (m, 1H)

IR (neat) $cm^{-1}$:

1620, 1506, 1271, 1146, 1103, 997

Reference Examples 78 - 95:

The procedure of Reference Example 77 was repeated to prepare the compounds shown in Figs. 5 - 7 and their physical data are listed in Table 8 (t-Bu, t-butyl group; i-Bu, isobutyl group; i-Pr, isopropyl group; n-Pr, n-propyl group; Et, ethyl group).

Table 8-1

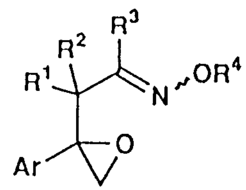

| REF. EX. | Ar | $R^1$ $R^2$ | $R^3$ | $R^4$ | NMR ppm | IR $cm^{-1}$ | M.P. °C |
|---|---|---|---|---|---|---|---|
| 78 | 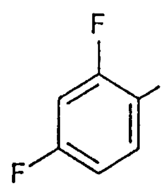 | 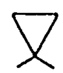 | i-Pr | (Z) $CH_2OCH_3$ | *$CDCl_3$: 0.54-1.25(m, 4H), 0.71(d, 3H, J=6.9Hz), 0.98(d, 3H, J=6.6Hz), 1.95-2.38(m, 1H), 2.91(d, 1H, J=5.4Hz), 3.12(d, 1H, J=5.4Hz), 3.45(s, 3H), 4.96-5.15(m, 2H), 6.60-6.92(m, 2H), 7.20-7.49(m, 1H) | KBr: 1618, 1508, 1159, 1142, 997, 968 | OIL |
| 79 | 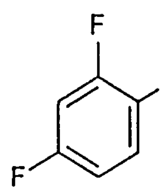 | 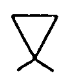 | Et | $CH_2OCH_3$ | *$CDCl_3$: 0.57-1.38(m, 7H), 1.92-2.74(m, 2H), 2.99(s, 2H), 3.25(s, 3H), 4.98(s, 2H), 6.54-7.02(m, 2H), 7.29-7.64(m, 1H) | KBr: 1618, 1508, 1146, 1101, 1088, 1012, 968 | OIL |

Table 8-2

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 80 | 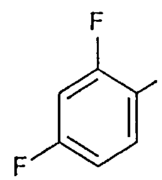 | 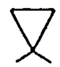 | | n-Pr | $CH_2OCH_3$ | $CDCl_3$: 0.68-0.75(m, 1H), 0.84-0.91(m, 4H), 1.01-1.07(m, 2H), 1.35-1.51(m, 2H), 2.01-2.17(m, 1H), 2.34-2.47(m, 1H), 3.00(s, 2H), 3.25(s, 3H), 4.98(s, 2H), 6.71-6.86(m, 2H), 7.43-7.52(m, 1H) | neat: 1618, 1601, 1508, 1101, 1007, 851 | OIL |
| 81 | 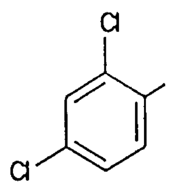 | 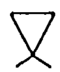 | | Et | $CH_2OCH_3$ | $CDCl_3$: 0.82-0.98(m, 4H), 1.06(t, 3H, J=7.6Hz), 2.29-2.51(m, 2H), 2.90(d, 1H, J=5.0Hz), 3.08(d, 1H, J=5.0Hz), 3.29(S, 3H), 4.98(d, 1H, J=7.3Hz), 5.03(d, 1H, J=7.3Hz), 7.22(dd, 1H, J=2.3, 8.3Hz), 7.33(d, 1H, J=2.0Hz), 7.40(d, 1H, J=8.3Hz) | neat: 1589, 1473, 1149, 1086, 1011 | OIL |
| 82 | 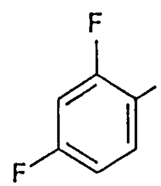 | 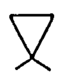 | | 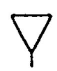 | $CH_2OCH_3$ | *$CDCl_3$: 0.60-1.85(m, 9H), 2.81-3.06(m, 2H), 3.21(s, 3H), 4.93(s, 2H), 6.65-7.01(m, 2H), 7.23-7.59(m, 1H) | KBr: 1612, 1506, 1271, 1153, 1103, 995 | OIL |

Table 8-3

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 83 | 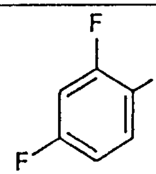 | 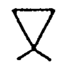 | | t-Bu | $CH_2OCH_3$ | *$CDCl_3$: 0.59-1.08(m, 4H), 1.31(s, 9H), 2.85-3.15(m, 2H), 3.23(s, 3H), 4.95(s, 2H), 6.56-6.86(m, 2H), 7.28-7.61(m 1H) | KBr: 1620, 1510, 1151, 1092, 1001, 970 | OIL |
| 84 | 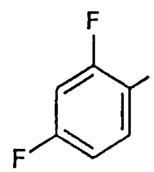 | 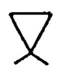 | | i-Bu | $CH_2OCH_3$ | $CDCl_3$: 0.65-0.93(m, 2H), 0.75(d, 3H, J=6.4Hz), 0.84(d, 3H, J=6.4Hz), 1.04-1.12(m, 2H), 1.87-1.99(m, 2H), 2.37-2.47(m, 1H), 2.97-3.03(m, 2H), 3.27(s, 3H), 4.97(d, 1H, J=7.3Hz), 4.99(d, 1H, J=7.3Hz), 6.71-6.85(m, 2H), 7.47-7.56(m, 1H) | KBr: 1618, 1506, 1271, 1147, 1101, 1009, 968, 851 | OIL |
| 85 | 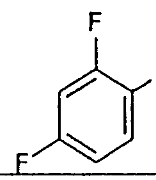 | $CH_3$ | $CH_3$ | $CH_3$ | $CH_2OCH_3$ | *$CDCl_3$: 1.17(s, 6H), 2.00(s, 3H), 2.73(dd, 1H, J=1.0, 4.6Hz), 3.14(d, 1H, J=4.6Hz), 3.29(s, 3H), 5.00(s, 2H), 6.64-6.90(m, 2H), 7.16-7.42(m, 1H) | neat: 1618, 1506, 1144, 1009, 968 | OIL |
| 86 | 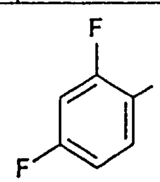 | 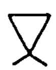 | | i-Pr | $CH_3$ | *$CDCl_3$: 0.71-0.99(m, 4H), 1.11(d, 6H, J=6.9Hz), 2.78(sep, 1H, J=6.9Hz), 2.87-3.07(m, 2H), 3.65(s 3H), 6.56-6.99(m, 2H), 7.28-7.60(m, 1H) | KBr: 1614, 1506, 1271, 1103, 1039, 968 | OIL |

Table 8-4

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 87 | 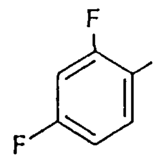 | 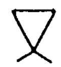 | | $CH_3$ | $CH_3$ | *$CDCl_3$: 0.48-1.08(m, 4H), 1.81(s, 3H), 2.93(d, 1H, J=5.2Hz), 3.02(d, 1H, J=5.0Hz), 3.72(s, 3H), 6.63-6.95(m, 2H), 7.28-7.54(m, 1H) | neat: 1618, 1506, 1271, 1142, 1053, 968 | OIL |
| 88 | 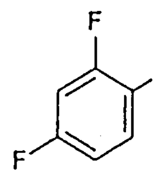 | 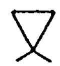 | | Et | $CH_3$ | *$CDCl_3$: 0.59-1.26(m, 7H), 1.91-2.52(m, 2H), 2.99(s, 1H), 3.70(s, 3H), 6.59-6.94(m, 2H), 7.26-7.57(m, 1H) | neat: 1618, 1506, 1142, 1101, 1049, 968, 852 | OIL |
| 89 | 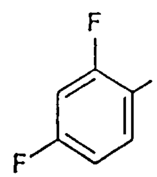 | 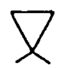 | | n-Pr | $CH_3$ | $CDCl_3$: 0.63-0.72(m, 1H), 0.82-0.89(m, 4H), 0.98-1.04(m, 2H), 1.34-1.48(m, 2H), 2.00-2.10(m, 1H), 2.29-2.40(m, 1H), 2.99(d, 1H, J=5.9Hz), 3.00(d, 1H, J=5.0Hz), 3.70(s, 3H), 6.71-6.87(m, 2H), 7.40-7.48(m, 1H) | neat: 1618, 1506, 1142, 1101, 1053, 968 | OIL |
| 90 | 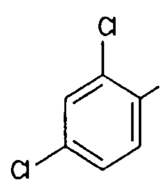 | 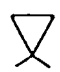 | | Et | $CH_3$ | $CDCl_3$: 0.76-1.06(m, 4H), 1.03(t, 3H, J=7.6Hz), 2.30-2.40(m, 2H), 2.90(d, 1H, J=5.0Hz), 3.09(d, 1H, J=5.0Hz), 3.74(s, 3H), 7.22(dd, 1H, J=2.3, 8.2Hz), 7.34(d, 1H, J=2.0Hz), 7.38(d, 1H, J=8.6Hz) | neat: 1589, 1473, 1379, 1105, 1051, 887 | OIL |

Table 8-5

| REF. EX. | Ar | $R^1$ | $R^2$ | $R^3$ | $R^4$ | NMR ppm | IR $cm^{-1}$ | M.P. °C |
|---|---|---|---|---|---|---|---|---|
| 91 | 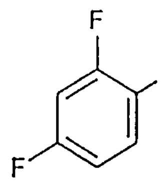 | 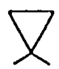 | | 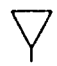 | $CH_3$ | *$CDCl_3$: 0.59-1.13(m, 8H), 1.44-1.79(m, 1H), 2.87(d, 1H, J=4.9Hz), 3.01(d, 1H, J=4.9Hz), 3.68(s, 3H), 6.59-7.02(m, 2H), 7.24-7.57(m, 1H) | KBr: 1614, 1506, 1425, 1271, 1103, 1061, 1038, 968 | OIL |
| 92 | 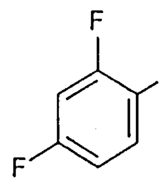 | 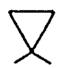 | | t-Bu | $CH_3$ | *$CDCl_3$: 0.60-1.08(m, 4H), 1.27(s, 9H), 2.85-3.14(m, 2H), 3.66(s, 3H), 6.58-6.99(m, 2H), 7.23-7.62(m, 1H) | KBr: 1612, 1504, 1302, 1271, 1039, 968 | OIL |
| 93 | 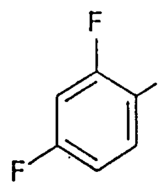 | 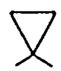 | | i-Bu | $CH_3$ | $CDCl_3$: 0.62-0.91(m, 2H), 0.72(d, 3H, J=6.3Hz), 0.82(d, 3H, J=6.4Hz), 1.01-1.12(m, 2H), 1.82-1.92(m, 2H), 2.31-2.40(m, 1H), 2.97-3.03(m, 2H),3.70(s, 3H), 6.71-6.86(m, 2H), 7.44-7.52(m, 1H) | KBr: 1618, 1506, 1269, 1101, 1051, 968 | OIL |
| 94 | 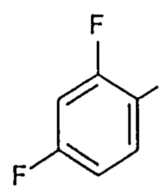 | $CH_3$ | $CH_3$ | $CH_3$ | $CH_3$ | *$CDCl_3$: 1.14(s, 6H), 1.93(s, 3H), 2.72(d, 1H, J=4.6Hz), 3.13(d, 1H, J=4.6Hz), 3.73(s, 3H), 6.60-7.02(m, 2H), 7.08-7.44(m, 1H) | neat: 1618, 1506, 1423, 1051, 968 | OIL |

Table 8-6

| REF. EX. | Ar | $R^1$ | $R^2$ | $R^3$ | $R^4$ | NMR ppm | IR $cm^{-1}$ | M.P. °C |
| --- | --- | --- | --- | --- | --- | --- | --- | --- |
| 95 | 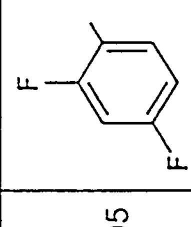 | $CH_3$ | $CH_3$ | Et | $CH_3$ | *$CDCl_3$: 1.06(t, 3H, J=7.6Hz), 1.17(s, 6H), 2.22-2.58(m, 2H), 2.71(d, 1H, J=4.8Hz), 3.13(d, 1H, J=4.8Hz), 3.71(s, 3H), 6.59-6.98(m, 2H), 7.10-7.40(m, 1H) | *neat: 1612, 1507, 1269, 1142, 1099, 1051, 968 | OIL |

Reference Example 96:

4 N HCl (0.33 ml) was added to a solution of 1-[1-[1-(2,4-difluorophenyl)-1-hydroxy-2-(1H-1,2,4-triazol-1-yl)ethyl]cyclopropyl-1-propane O-methyl oxime (16 ml) in acetone (0.63 ml) and the mixture was heated

under reflux for 4 h.

The reaction mixture was concentrated under vacuum and ethyl acetate (10 ml) and water (10 ml) were added to the residue for extraction. Further extraction was caried out with ethyl acetate (10 ml) and the organic layers were combined, washed with water (10 ml) and brine (10 ml) and dried over anhydrous sodium sulfate.

The desiccant was filtered off and the solvent was concentrated under vacuum and the residue was purified by silica gel column chromatography (eluant: hexane/ethyl acetate = 3/2). The fractions containing the end product were collected, concentrated under vacuum and crystallized from a mixture of hexane and ether to give 1-[1-[1-(2,4-difluorophenyl)-1-hydroxy-2-(1H-1,2,4-triazol-1-yl)ethyl]cyclopropyl]-1-propanone (11 mg; see Fig. 7) as a colorless crystal.

It should be noted here that the synthesis procedure employed in Reference Examples 96 - 101 was by Process F.

*NMR ($CDCl_3$) δ [ppm]:

0.87-1.48 (m, 4H), 0.94 (t, 3H, J = 7.3Hz), 2.11 (q, 2H, J = 7.3Hz), 4.88 (d, 1H, J = 14.0Hz), 4.98 (d, 1H, J = 14.0Hz), 5.41 (s, 1H), 6.55-6.91 (m, 2H), 7.31-7.63 (m, 1H), 7.78 (s, 1H), 8.11 (s, 1H)

IR (KBr) $cm^{-1}$:

3234, 1687, 1612, 1497, 1416, 1142, 966

m.p.: 118.2-120.8 °C

Reference Examples 97 - 101:

The procedure of Reference Example 96 was repeated to prepare the compounds shown in Fig. 7 and their physical data are listed in Table 9 (t-Bu, t-butyl group; i-Bu, isobutyl group; i-Pr, isopropyl group; n-Pr, n-propyl group; Et, ethyl group).

Table 9-1

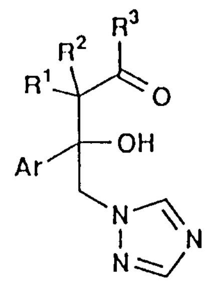

| REF. EX. | Ar | $R^1$ | $R^2$ | $R^3$ | NMR ppm | IR $cm^{-1}$ | M.P. °C |
|---|---|---|---|---|---|---|---|
| 97 | 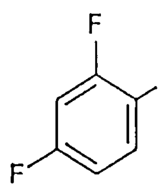 | 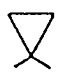 | | $CH_3$ | *$CDCl_3$: 0.60-1.00(m, 4H), 1.90(s, 3H), 4.92(d, 1H, J=14.0Hz), 5.00(d, 1H, J=14.0Hz), 5.27(s, 3H), 6.60-6.96(m, 2H), 7.35-7.62(m, 1H), 7.81(s, 1H), 8.11(s, 1H) | neat: 3271, 1678, 1614, 1502, 1275, 1107, 968 | OIL |
| 98 | 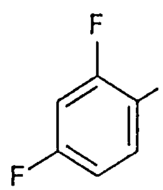 | 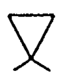 | | n-Pr | $CDCl_3$: 0.80(t, 3H, J=7.3Hz), 0.99-1.57(m, 6H), 1.95-2.09(m, 2H), 4.85(d, 1H, J=13.7Hz), 5.00(d, 1H, J=14.2Hz), 5.39(s, 1H), 6.67-6.85(m, 2H), 7.39-7.52(m, 1H), 7.79(s, 1H), 8.11(s, 1H) | neat: 3138, 1684, 1614, 1500, 1275, 1140, 1107, 970 | OIL |

Table 9-2

| REF. EX. | Ar | $R^1$ | $R^2$ | $R^3$ | NMR ppm | IR $cm^{-1}$ | M.P. °C |
|---|---|---|---|---|---|---|---|
| 99 | 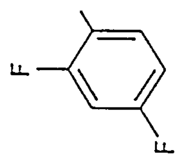 | $CH_3$ | $CH_3$ | $CH_3$ | *$CDCl_3$: 1.17(S, 3H), 1.19(d, 3H, J=3.6Hz), 2.32(s, 3H), 4.26-4.53(m, 1H), 5.28(dd, 1H, J=2.6, 14.2Hz), 5.55(s, 1H), 6.45-6.93(m, 2H), 7.38-7.83(m, 1H), 7.71(s, 1H), 8.03(d, 1H, J=2.0Hz) | KBr: 3375, 1699, 1612, 1275, 1250, 1149, 1099, 970 | 79.3-80.8 |
| 100 | 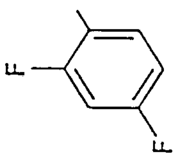 | $CH_3$ | $CH_3$ | Et | $CDCl_3$: 0.93-1.08(m, 3H), 1.08-1.30(m, 6H), 2.42-2.60(m, 1H), 2.70-2.88(m, 1H), 4.41(d, 1H, J=14.2Hz), 5.25(d, 1H, J=14.2Hz), 5.58(s, 1H), 6.61-6.70(m, 1H), 6.75-6.85(m, 1H), 7.55-7.71(m, 1H), 7.71(s, 1H), 8.03(s, 1H) | KBr: 3379, 1699, 1614, 1512, 1490, 1147, 1099, 968 | 101.4-103.1 |
| 101 | 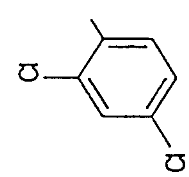 | 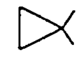 | | Et | $CDCl_3$: 0.91(t, 3H, J=7.3Hz), 1.11-1.54(m, 4H), 2.05-2.26(m, 2H), 4.56(d, 1H, J=14 .2Hz), 4.97(d, 1H, J=13.9Hz), 7.10-7.14(m, 1H), 7.35(d, 1H, J=2.0Hz), 7.39(d, 1H, J=9.0Hz), 7.76(s, 1H), 7.99(s, 1H) | KBr: 3286, 1687, 1377, 1128, 1093, 797 | 110.6-122.2 |

Example 1:

1H-1,2,4-Triazole (3.79 g) was added to a solution of potassium t-butoxide (3.14 g) in dimethyl formamide (33 ml) under an argon atmosphere at room temperature and the mixture was stirred at the same temperature for 10 min. To the reaction mixture, 1-[1-[2-(2,4-difluorophenyl)oxiran-2-yl]cyclopropyl]-2-methyl-1-propanone O-(methoxymethyl)oxime (4.46 g) was added and the mixture was stirred at 90 - 95 °C

for 2 h.

The stirred reaction mixture was poured into ice water and extracted with ethyl acetate (150 ml × 2). The organic layers were combined, washed with water (100 ml) and brine (100 ml) and dried over anhydrous sodium sulfate.

The desiccant was filtered off and the solvent was concentrated under vacuum and the residue was purified by silica gel column chromatography (eluant: ethyl acetate/hexane = 1/2 - 2/3). The fractions containing the end product were collected, concentrated under vacuum and crystallized from a mixture of hexane and ether to give 1-[1-[1-(2,4-difluorophenyl)-1-hydroxy-2-(1H-1,2,4-triazol-1-yl)ethyl]cyclopropyl]-2-methyl-1-propanone O-(methoxymethyl)oxime (4.13 g; for its structure, see Fig. 8) as a colorless crystal.

NMR ($CDCl_3$) δ [ppm]:

0.74-0.96 (m, 4H), 0.99 (d, 3H, J = 7.3Hz), 1.10 (d, 3H, J = 6.9Hz), 2.57-2.73 (m, 1H), 3.39 (s, 3H), 4.51 (d, 1H, J = 14.2Hz), 4.99-5.13 (m, 1H), 5.03 (d, 1H, J = 7.3Hz), 5.07 (d, 1H, J = 7.3Hz), 5.31 (s, 1H,), 6.62-6.85 (m, 2H), 7.45-7.85 (m, 1H), 7.71 (s, 1H), 8.11 (s, 1H)

IR (KBr) $cm^{-1}$:

3248, 1610, 1500, 1421, 1279, 1144, 1090, 1053, 999

m.p.: 121.0-123.3 °C

Example 2:

The procedure of Example 1 was repeated using the compound prepared in Reference Example 86, whereby 1-[1-[1-(2,4-difluorophenyl)-1-hydroxy-2-(1H-1,2,4-triazol-2-yl)ethyl]cyclopropyl]-2-methyl-1-propanone O-methyl oxime (see Fig. 8) was obtained as acolorless crystal.

NMR ($CDCl_3$) δ [ppm]:

0.71-0.94 (m, 4H), 0.97 (d, 3H, J = 6.9Hz), 1.06 (d, 3H, J = 6.9Hz), 2.52-2.71 (m, 1H), 3.82 (s, 3H), 4.48 (d, 1H, J = 14.2Hz), 5.06 (d, 1H, J = 14.2Hz), 5.30 (s, 1H), 6.63-6.82 (m, 2H), 7.44-7.58 (m, 1H), 7.70 (s, 1H), 8.09 (s, 1H)

IR (KBr) $cm^{-1}$:

3111, 1614, 1518, 1500, 1277, 1147, 1039, 970

m.p.: 89.7-90.9 °C

Example 3:

The compound obtained in Example 1 was split by high-performance liquid chromatography (mobile phase: hexane/isopropyl alcohol = 19/1) using an chiral HPLC column for optical resolution (CHIRALCEL O.D. of Daicel Chemical Industries, Ltd.; o.d. 0.46 cm × h. 25 cm). The 16- to 18-min peaks were recovered several times at a flow rate of 1 ml/min and concentrated to dryness under vacuum to give (+)-1-[1-[1-(2,4-difluorophenyl)-1-hydroxy-2-(1H-1,2,4-triazol-1-yl)ethyl]cyclopropyl]-2-methyl-1-propanone O-(methoxymethyl)oxime (see Fig. 8) as a colorless oil.

NMR ($CDCl_3$) δ [ppm]:

0.73-0.97 (m, 4H), 0.99 (d, 3H, J = 7.3Hz), 1.10 (d, 3H, J = 7.3Hz), 2.53-2.73 (m, 1H), 3.39 (s, 3H), 4.48-4.54 (m, 1H), 4.99-5.10 (m, 3H), 5.31 (bs, 1H), 6.65-6.81 (m, 2H), 7.45-7.58 (m, 1H), 7.71 (s, 1H), 8.12 (s, 1H)

IR (KBr) $cm^{-1}$:

3398, 1616, 1500, 1275, 1142, 1086, 993, 966 Specific rotation: +43.1° (c = 0.32)

Example 4:

Subsequent to the separation in Example 3, the 31- to 35-min peaks were recovered several times, concentrated under vacuum and crystallized from a mixture of hexane and ether to give (-)-1-[1-[1-(2,4-difluorophenyl)-1-hydroxy-2-(1H-1,2,4-triazol-1-yl)ethyl]cyclopropyl]-2-methyl-1-propanone O-(methoxymethyl)oxime (see Fig. 8) as a colorless crystal.

NMR ($CDCl_3$) δ [ppm]:

0.75-0.97 (m, 4H), 0.99 (d, 3H, J = 7.3Hz), 1.10 (d, 3H, J = 7.3Hz), 2.57-2.73 (m, 1H), 3.39 (s, 3H), 4.48-4.55 (m, 1H), 5.00-5.10 (m, 3H), 5.31 (s,

IR (KBr) $cm^{-1}$:

3398, 1616, 1500, 1275, 1142, 1086, 993, 966

Specific rotation: -46.6° (c = 0.15)

Example 5:

Using the compound prepared in Example 2, the 11- to 12.5-min peaks were recovered several times under the same separative conditions as in Example 3; the collected peaks were concentrated under vacuum and crystallized from a mixture of hexane and ether to give (+)-1-[1-[1-(2,4-difluorophenyl)-1-hydroxy-2-(1H-1,2,4-triazol-1-yl)ethyl]cyclopropyl]-2-methyl-1-propanone O-methyl oxime (see Fig. 8) as a colorless crystal.

NMR ($CDCl_3$) δ [ppm]:

0.72-0.92 (m, 4H), 0.97 (d, 3H, J=6.9Hz), 1.07 (d, 3H, J=6.9Hz), 2.54-2.71 (m, 1H), 3.81 (s, 3H), 4.45-4.54 (m, 1H), 5.02-5.09 (m, 1H), 5.30 (br, 1H), 6.64-6.80 (m, 2H), 7.44-7.58 (m, 1H), 7.71 (s, 1H), 8.10 (s, 1H)

IR (KBr) $cm^{-1}$:

3131, 1620, 1500, 1273, 1140, 1109, 1038, 966

m.p.: 133.8-149.6°C

Specific rotation: +52.5° (c = 0.08)

Example 6:

Subsequent to the separation in Example 5, the 22- to 24.5-min peaks were recovered several times, concentrated under vacuum and crystallized from a mixture of hexane and ether to give (-)-1-[1-[1-(2,4-difluorophenyl)-1-hydroxy-2-(1H-1,2,4-triazol-1-yl)ethyl]cyclopropyl]-2-methyl-1-propanone O-methyl oxime (see Fig. 8) as a colorless crystal.

NMR ($CDCl_3$) δ [ppm]:

0.74-0.93 (m, 4H), 0.96 (d, 3H, J=6.9Hz), 1.07 (d, 3H, J=6.9Hz), 2.54-2.71 (m, 1H), 3.81 (s, 3H), 4.44-4.54 (m, 1H), 5.02-5.09 (m, 1H), 5.32 (br, 1H), 6.64-6.80 (m, 2H), 7.44-7.58 (m, 1H), 7.71 (s, 1H), 8.10 (s, 1H)

IR (KBr) $cm^{-1}$:

3111, 1614, 1518, 1500, 1147, 1039, 970

m.p.: 100.3-103.9°C

Specific rotation: -56.0° (c = 0.15)

Examples 7 - 22:

The procedure of Example 1 was repeated to prepare the compounds shown in Figs. 8 and 9 and their physical data are listed in Table 10 (t-Bu, t-butyl group; i-Bu, isobutyl group; i-Pr, isopropyl group; n-Pr, n-propyl group; Et, ethyl group).

Table 10-1

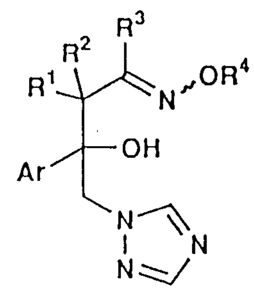

| EX. | Ar | $R^1$ | $R^2$ | $R^3$ | $R^4$ | NMR ppm | IR $cm^{-1}$ | M.P. °C |
|---|---|---|---|---|---|---|---|---|
| 7 | 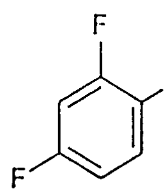 | 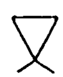 | | i-Pr | (Z) $CH_2OCH_3$ | $CDCl_3$: 0.83-1.01(m, 7H), 1.11(d, 3H, J=6.6Hz), 2.47-2.60(m, 1H), 3.50(s, 3H), 4.27(d, 2H, J=14.2Hz), 5.01-5.24(m, 3H), 5.50(s, 1H), 6.67-6.79(m, 2H), 7.42-7.53(m, 1H), 7.67(s, 1H), 8.03(s, 1H) | KBr: 3429, 1616, 1500, 1275, 1140, 982, 852 | OIL |

Table 10-2

| EX. | Ar | $R^1$ | $R^2$ | $R^3$ | $R^4$ | NMR ppm | IR $cm^{-1}$ | M.P. °C |
|---|---|---|---|---|---|---|---|---|
| 8 | 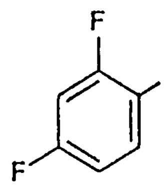 | 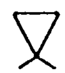 | | n-Pr | $CH_2OCH_3$ | $CDCl_3$: 0.70-1.05(m, 4H), 0.81(t, 3H, J=7.3Hz), 1.20-1.52(m, 2H), 1.66-1.77(m, 1H), 2.14-2.24(m, 1H), 3.39(s, 3H), 4.61(dd, 1H, J=1.3, 15.5Hz), 5.01-5.07(m, 1H), 5.07(s, 2H), 5.38(s, 1H), 6.64-6.83(m, 2H), 7.49-7.58(m, 1H), 7.75(s, 1H), 8.17(s, 1H) | KBr: 3205, 1616, 1502, 1142, 1018, 866 | 102.9-104.0 |
| 9 | 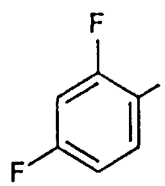 | 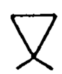 | | n-Pr | $CH_3$ | $CDCl_3$: 0.71-1.04(m, 7H), 1.08-1.44(m, 2H), 1.69-1.77(m, 1H), 2.08-2.19(m, 1H), 3.83(s, 3H), 4.50-4.56(m, 1H), 5.07(dd, 1H, J=1.3, 14.0Hz), 5.43(bs, 1H), 6.64-6.82(m, 2H), 7.47-7.56(m, 1H), 7.73(s, 1H), 8.13(s, 1H) | KBr: 3109, 1614, 1500, 1147, 1111, 1049, 970 | 87.1-88.9 |
| 10 | 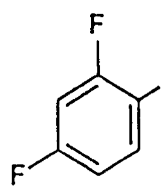 | 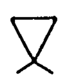 | | Et | $CH_2OCH_3$ | *$CDCl_3$: 0.56-1.38(m, 7H), 1.52-1.98(m, 1H), 2.02-2.64(m, 1H), 3.40(s, 3H), 4.65(dd, 1H, J=1.7, 14.2Hz), 5.05(dd, 1H, J=1.7, 14.2Hz), 5.09(s, 2H), 5.32(s, 1H), 6.45-6.93(m, 2H), 7.35-7.71(m, 1H), 7.75(s, 1H), 8.17(d, 1H, J=1.0Hz) | KBr: 3105, 1612, 1518, 1500, 1144, 1014, 1003, 970 | 90.4-92.7 |

Table 10-3

| EX. | Ar | $R^1$ | $R^2$ | $R^3$ | $R^4$ | NMR ppm | IR $cm^{-1}$ | M.P. °C |
|---|---|---|---|---|---|---|---|---|
| 11 | 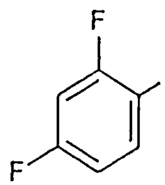 | 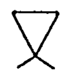 | | Et | $CH_3$ | *$CDCl_3$: 0.73-1.08(m, 7H), 1.55-2.44(m, 2H), 3.85(s, 3H), 4.57(dd, 1H, J=1.7, 14.2Hz), 5.09(dd, 1H, J=1.7, 14.2Hz), 5.34(s, 1H), 6.53-6.91(m, 2H), 7.37-7.71(m, 1H), 7.73(s, 1H), 8.13(d, 1H, J=1.0Hz) | KBr: 3107, 1612, 1500, 1146, 1109, 1043, 968, 868 | 101.8-103.2 |
| 12 | 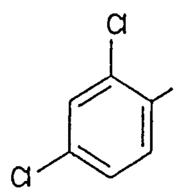 | 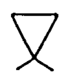 | | Et | $CH_2OCH_3$ | $CDCl_3$: 0.70-0.97(m, 1H), 0.94(t, 3H, J=7.6Hz), 1.06-1.25(m, 3H), 1.90-2.05(m, 1H), 2.18-2.31(m, 1H), 3.31(S, 3H), 4.40(d, 1H, J=13.9Hz), 4.98(d, 1H, J=7.3Hz), 5.05(d, 1H, J=7.3Hz), 5.33(d, 1H, J=14.2Hz), 7.10(dd, 1H, J=2.3, 8.6Hz), 7.30(d, 1H, J=2.0Hz), 7.53(d, 1H, J=8.6Hz), 7.74(s, 1H), 8.19(s, 1H) | KBr: 3159, 1585, 1520, 1369, 1144, 1024, 852 | 111.4-115.8 |

Table 10-4

| EX. | Ar | $R^1$ | $R^2$ | $R^3$ | $R^4$ | NMR ppm | IR $cm^{-1}$ | M.P. °C |
|---|---|---|---|---|---|---|---|---|
| 13 | 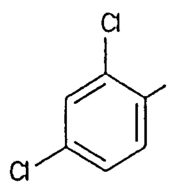 | 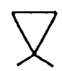 | | Et | $CH_3$ | $CDCl_3$: 0.83-1.19(m, 4H), 0.90(t, 3H, J=7.6Hz), 1.85-1.98(m, 1H), 2.15-2.28(m, 1H), 3.78(s, 3H), 4.34(d, 1H, J=13.9Hz), 5.31-5.36(m, 2H), 7.09-7.13(m, 1H), 7.30(d, 1H, J=2.3Hz), 7.53(d, 1H, J=8.6Hz), 7.70(s, 1H), 8.08(s, 1H) | KBr: 3375, 1583, 1516, 1468, 1049, 1028, 889 | 117.6-120.6 |
| 14 | 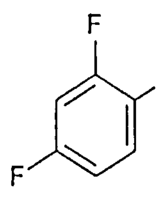 | 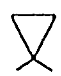 | | $CH_3$ | $CH_3$ | *$CDCl_3$: 0.48-1.11(m, 4H), 1.57(s, 3H), 3.86(s, 3H), 4.47-4.61(m, 1H), 5.09-5.25(m, 1H), 5.15(s, 1H), 6.53-6.88(m, 2H), 7.43-7.75(m, 1H), 7.75(s, 1H), 8.14(s, 1H) | KBr: 3165, 1614, 1502, 1111, 1055, 874 | 96.2-98.0 |
| 15 | 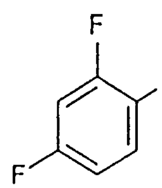 | 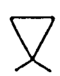 | | 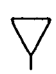 | $CH_2OCH_3$ | $CDCl_3$: 0.33-1.10(m, 8H), 1.28-1.40(m, 1H), 3.44(s, 3H), 4.68(dd, 1H, J=2.0, 14.2Hz), 5.03(d, 1H, J=6.9Hz), 5.11(d, 1H, J=6.9Hz), 5.16(bs, 1H), 5.19(dd, 1H, J=1.7, 14.2Hz), 6.63-6.86(m, 2H), 7.55-7.65(m, 1H), 7.78(s, 1H), 8.24(s, 1H) | KBr: 3250, 1610, 1500, 1423, 1146, 1090, 1053, 995 | 120.1-121.2 |

Table 10-5

| EX. | Ar | $R^1$ | $R^2$ | $R^3$ | $R^4$ | NMR ppm | IR $cm^{-1}$ | M.P. °C |
|---|---|---|---|---|---|---|---|---|
| 16 | 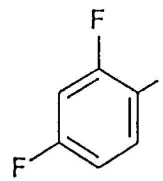 | 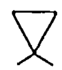 | | 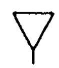 | $CH_3$ | $CDCl_3$: 0.35-1.09(m, 8H), 1.27-1.40(m, 1H), 3.84(s, 3H), 4.52-4.59(m, 1H), 5.09(bs, 1H), 5.25-5.32(m, 1H), 6.62-6.83(m, 2H), 7.50-7.62(m, 1H), 7.76(s, 1H), 8.19(s, 1H) | KBr: 3111, 1614, 1520, 1500, 1109, 1063, 1043, 970 | 90.9-96.2 |
| 17 | 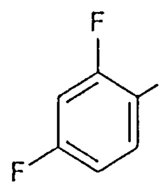 | 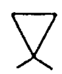 | | i-Bu | $CH_2OCH_3$ | $CDCl_3$: 0.65(d, 3H, J=6.8Hz), 0.76-1.12(m, 4H), 0.79(d, 3H, J=6.8Hz), 1.65-1.72(m, 1H), 1.84-1.99(m, 1H), 2.10-2.17(m, 1H), 3.35(s, 3H), 4.53(d, 1H, J=14.4Hz), 4.93(d, 1H, J=14.4Hz), 5.01(d, 1H, J=7.3Hz), 5.05(d, 1H, J=7.3Hz), 5.66(s, 1H), 6.66-6.79(m, 2H), 7.43-7.52(m, 1H), 7.71(s, 1H), 8.51(s, 1H) | KBr: 3215, 1612, 1498, 1273, 1142, 1088, 1003, 966, 864 | 118.3-120.8 |

Table 10-6

| EX. | Ar | $R^1$ | $R^2$ | $R^3$ | $R^4$ | NMR ppm | IR $cm^{-1}$ | M.P. °C |
|---|---|---|---|---|---|---|---|---|
| 18 | 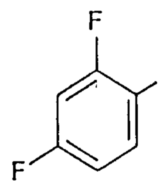 | 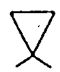 | | i-Bu | $CH_3$ | $CDCl_3$: 0.61(d, 3H, J=6.4Hz), 0.78(d, 3H, J=6.8Hz), 0.83-1.12(m, 3H), 1.23-1.31(m, 1H), 1.63-1.70(m, 1H), 1.76-1.91(m, 1H), 2.05-2.12(m, 1H), 3.79(s, 3H), 4.46(d, 1H, J=14.2Hz), 4.94(d, 1H, J=14.2Hz), 5.75(s, 1H), 6.66-6.79(m, 2H), 7.40-7.49(m, 1H), 7.70(s, 1H), 8.11(s, 1H) | KBr: 3113, 1612, 1516, 1500, 1146, 1109, 1051, 970 | 79.8-83.3 |
| 19 | 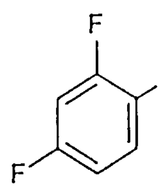 | 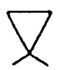 | | t-Bu | $CH_2OCH_3$ | $CDCl_3$: 0.47-1.05(m, 4H), 1.21-1.37(m, 9H), 3.45(s, 3H), 4.23-4.70(m, 1H), 5.08-5.74(m, 4H), 6.58-6.86(m, 2H), 7.43-7.65(m 1H), 7.71(s, 1H), 8.11(s, 1H) | KBr: 3398, 1616, 1502, 1275, 1142, 1099, 987 | OIL |
| 20 | 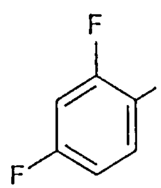 | 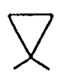 | | t-Bu | $CH_3$ | $CDCl_3$: 0.53-0.96(m, 3H), 1.19-1.25(m, 1H), 1.30(s, 9H), 4.02(s, 3H), 4.23(d, 1H, J=14.0Hz), 5.25(d, 1H, J=14.0Hz), 5.87(s, 1H), 6.63-6.83(m, 2H), 7.41-7.52(m, 1H), 7.61(s, 1H), 8.07(s, 1H) | KBr: 3217, 1614, 1514, 1498, 1107, 1045, 1032, 858 | 131.6-141.7 |

Table 10-7

| EX. | Ar | $R^1$ | $R^2$ | $R^3$ | $R^4$ | NMR ppm | IR $cm^{-1}$ | M.P. °C |
|---|---|---|---|---|---|---|---|---|
| 21 | 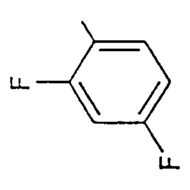 | $CH_3$ | $CH_3$ | $CH_3$ | $CH_3$ | *$CDCl_3$: 1.16-1.22(m, 6H), 1.88(s, 3H), 3.87(s, 3H), 4.26-4.53(m, 1H), 5.16-5.43(m, 1H), 5.43(s, 1H), 6.48-6.96(m, 2H), 7.47-7.80(m, 1H), 7.71(s, 1H), 8.08(s, 1H) | * KBr: 3104, 1614, 1519, 1500, 1107, 1053 | 104.7-107.0 |
| 22 | 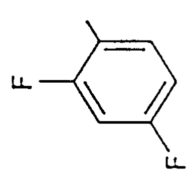 | $CH_3$ | $CH_3$ | Et | $CH_3$ | *$CDCl_3$: 1.04(t, 3H, J=7.6Hz), 1.14-1.26(m, 6H), 2.08-2.53(m, 2H), 3.88(s, 3H), 4.31-4.54(m, 1H), 5.17-5.41(m, 1H), 5.56(s, 1H), 6.47-6.91(m, 2H), 7.43-7.69(m, 1H), 7.72(s, 1H), 8.10(d, 1H, J=2.0Hz) | * KBr: 3101, 1612, 1517, 1501, 1146, 1106, 1055, 967 | 96.0-96.9 |

Example 23:

A solution of bromodimethylborane (2.9 ml) in 1,2-dichloroethane (13 ml) was added dropwise to a solution of 1-[1-[1-(2,4-difluorophenyl)-1-hydroxy-2-(1H-1,2,4-triazol-1-yl)ethyl]cyclopropyl]-2-methyl-1-propanone O-(methoxymethyl)oxime (3.91 g) in dichloromethane (85 ml) in an argon atmosphere at a

controlled temperature between -70 and -65 ° C.

The reaction mixture was stirred at the same temperature for 2 h, then at -40 ° C for 1 h; thereafter, the reaction mixture was cooled again to -70 ° C. The cooled reaction mixture was added to a mixture of a saturated aqueous solution of sodium hydrogencarbonate (147 ml) and tetrahydrofuran (293 ml) with ice-cooling over 30 min, followed by stirring at room temperature for 2 h and subsequent extraction. Further extraction was carried out with ethre (100 ml × 2) and the organic layers were combined, washed with a saturated aqueous solution of sodium hydrogencarbonate (147 ml), water (100 ml) and brine (100 ml), and dried over anhydrous sodium sulfate.

The desiccant was filtered off and the solvent was concentrated under vacuum and the residue was purified by silica gel column chromatography (eluant: ethyl acetate/hexane = 1/1 - 3/2). The fractions containing the end product were collected and concentrated under vacuum. The crystallized residue were collected and washed with a mixture of ether and hexane to give 1-[1-[1-(2,4-difluorophenyl)-1-hydroxy-2-(1H- 1,2,4-triazol-1-yl)ethyl]cyclopropyl]-2-methyl-1-propanone oxime (3.15 g; for its structure, see Fig. 9) as a colorless crystal.

NMR (DMSO-$d_6$) δ [ppm]:

0.63-0.73 (m, 1H), 0.84-1.12 (m, 3H), 0.93 (d, 3H, J = 6.9Hz), 1.02 (d, 3H, J = 7.3Hz), 2.63-2.76 (m, 1H), 4.32 (d, 1H, J = 14.2Hz), 4.75 (d, 1H, J = 14.2Hz), 5.72 (s, 1H), 6.83-6.91 (m, 1H), 7.03-7.12 (m, 1H), 7.25-7.35 (m, 1H), 7.67 (s, 1H), 8.24 (s, 1H), 10.36 (s, 1H)

IR (KBr) $cm^{-1}$:

3280, 1616, 1514, 1497, 1134, 1082, 968

m.p.: 152.1-153.9 ° C

Examples 24 - 29:

The procedure of Example 23 was repeated to prepare the compounds shown in Figs. 9 and 10 and their physical data are listed in Table 11 (t-Bu, t-butyl group; i-Bu, isobutyl group; i-Pr, isopropyl group; n-Pr, n-propyl group; Et, ethyl group).

Table 11-1

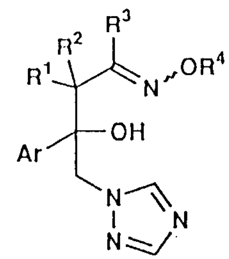

| EX. | Ar | $R^1$ | $R^2$ | $R^3$ | $R^4$ | NMR ppm | IR $cm^{-1}$ | M.P. °C |
|---|---|---|---|---|---|---|---|---|
| 24 | 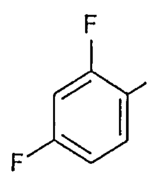 | 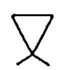 | | Et | H | *DMSO-d6: 0.44-1.42(m, 7H), 1.62-2.42(m, 2H), 4.42(d, 1H, J=14.2Hz), 4.86(d, 1H, J=14.2Hz), 5.75(s, 1H), 6.66-7.56(m, 3H), 7.70(s, 1H), 8.29(s, 1H), 10.49(s, 1H) | KBr: 3234, 1616, 1514, 1495, 1416, 1134, 1107, 1082, 970 | 133.8-141.6 |
| 25 | 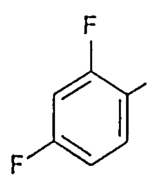 | 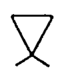 | | n-Pr | H | DMSO-d6: 0.53-0.70(m, 1H), 0.76(t, 3H, J=7.3Hz), 0.84-1.45(m, 5H), 1.85-1.95(m, 1H), 2.09-2.25(m, 1H), 4.36(d, 1H, J=13.9Hz), 4.81(d, 1H, J=14.1Hz), 5.77(s, 1H), 6.85-6.92(m, 1H), 7.02-7.10(m, 1H), 7.27-7.36(m, 1H), 7.68(s, 1H), 8.26(s, 1H), 10.50(s, 1H) | KBr: 3334, 1616, 1512, 1497, 1281, 1149, 966 | 180.0-182.3 |

Table 11-2

| EX. | Ar | $R^1$ | $R^2$ | $R^3$ | $R^4$ | NMR ppm | IR $cm^{-1}$ | M.P. °C |
|---|---|---|---|---|---|---|---|---|
| 26 | 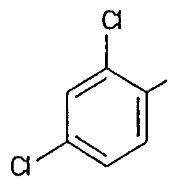 | 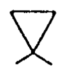 | | Et | H | $CDCl_3$: 0.86-1.29(m, 4H), 0.96(t, 3H, J=7.6Hz), 1.97-2.10(m, 1H), 2.23-2.37(m, 1H), 4.29(d, 1H, J=13.9Hz), 5.36(d, 1H, J=13.9Hz), 7.10(dd, 1H, J=2.3, 8.6Hz), 7.32(d, 1H, J=2.0Hz), 7.52(d, 1H, J=8.6Hz), 7.69(s, 1H), 8.25(s, 1H) | KBr: 3296, 1516, 1279, 1134, 1090, 931 | 200.0-202.0 |
| 27 | 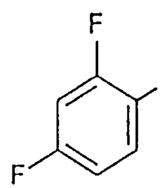 | 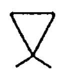 | | $CH_3$ | H | *DMSO-d6: 0.36-1.20(m, 4H), 3.33(s, 3H), 4.41(d, 1H, J=14.2Hz), 4.90(d, 1H, J=14.5Hz), 5.69(s, 1H), 6.72-7.44(m, 3H), 7.70(s, 1H), 8.29(s, 1H), 10.51(s, 1H) | KBr: 3253, 1614, 1518, 1495, 1144, 1034 | 170.0-175.2 |
| 28 | 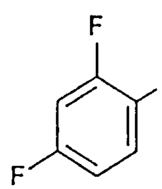 | 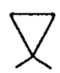 | | 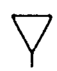 | H | DMSO-d6: 0.32-1.01(m, 8H), 1.37-1.48(m, 1H), 4.47-4.54(m, 1H), 5.14-5.20(m, 1H), 5.59(s, 1H), 6.89-7.10(m, 2H), 7.37-7.47(m, 1H), 7.74(s, 1H), 8.35(s, 1H), 10.56(s, 1H) | KBr: 3307, 3178, 1616, 1516, 1498, 1138, 1036, 966 | 131.4-132.9 |

Table 11-3

| EX. | Ar | $R^1$ | $R^2$ | $R^3$ | $R^4$ | NMR ppm | IR $cm^{-1}$ | M.P. °C |
|---|---|---|---|---|---|---|---|---|
| 29 | 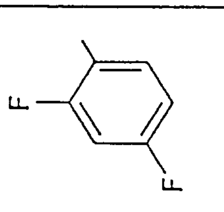 | $CH_3$ | $CH_3$ | $CH_3$ | H | *$CDCl_3$: 1.17(s, 3H), 1.21(d, 3H, J=3.0Hz), 1.97(s, 3H), 4.38(dd, 1H, J=2.0, 14.2Hz), 5.17-5.36(m, 1H), 5.53(s, 1H), 6.50-6.89(m, 2H), 7.48-7.72(m, 1H), 7.72(s, 1H), 8.09(d, 1H, J=2.3Hz), 8.48(s, 1H) | KBr: 3201, 1614, 1500, 1140, 1101, 849 | 129.9-133.0 |

Example 30:

60% Sodium hydride (59.2 mg) was added to a solution of 1-[1-[1-(2,4-difluorophenyl)-1-hydroxy-2-(1H-1,2,4-triazol-1-yl)ethyl]cyclopropyl]-2-methyl-1-propanone oxime (200 mg) in dimethylformamide (2 ml) with

ice-water-cooling, and the mixture was stirred at the same temperature for 1 h. To the stirred reaction mixture, (1H-1,2,4-triazol-1-yl)methyl chloride hydrochloride (109 mg) was added under cooling with ice water and the mixture was stirred at the same temperature for 2 h.

The reaction mixture was poured into ice water and extraction was carried out with ethyl acetate (20 ml × 3). The organic layers were combined, washed with water (20 ml) and brine (20 ml) and dried over anhydrous sodium sulfate. The desiccant was filtered off and the solvent was concentrated under vacuum and the residue was purified by silica gel column chromatography (eluant: ethyl acetate/hexane = 2/1). The fractions containing the end product were collected and concentrated under vacuum to give 1-[1-[1-(2,4-difluorophenyl)-1-hydroxy-2-(1H-1,2,4-triazol-1-yl)ethyl]cyclopropyl]-2-methyl-1-propanone O-[(1H-1,2,4-triazol-1-yl)methyl]oxime (222 mg; for its structure, see Fig. 10) as a white crystal.

NMR ($CDCl_3$) δ [ppm]:

0.68-0.91 (m, 4H), 0.98 (d, 6H, J = 6.9Hz), 2.56-2.75 (m, 1H), 4.33(d, 1H, J = 14.8Hz), 4.97-5.03 (m, 1H), 5.19 (s, 1H), 5.95 (s, 2H), 6.56-6.79 (m, 2H), 7.38-7.48 (m, 1H), 7.72 (s, 1H), 7.79 (s, 1H), 8.11 (s, 1H), 8.30 (s, 1H)

IR (KBr) $cm^{-1}$:

3404, 1614, 1510, 1279, 1140, 1024, 968

m.p.: 104.4-108.3 °C

Examples 31 - 43:

The procedure of Example 30 was repeated to prepare the compounds shown in Figs. 10 and 11 and their physical data are listed in Table 12 (t-Bu, t-butyl group; i-Bu, isobutyl group; i-Pr, isopropyl group; n-Pr, n-propyl group; Et, ethyl group; MEM, methoxyethoxymethyl group).

Table 12-1

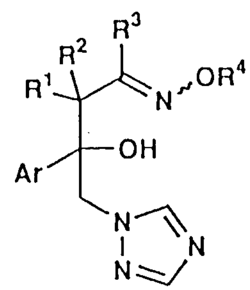

| EX. | Ar | R¹ R² | R³ | R⁴ | NMR ppm | IR $cm^{-1}$ | M.P. °C |
|---|---|---|---|---|---|---|---|
| 31 | 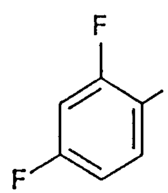 | 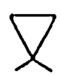 | i-Pr | 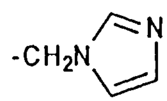 | $CDCl_3$: 0.60-0.95(m, 4H), 0.99(d, 3H, J=5.9Hz), 0.99(d, 3H, J=7.3Hz), 2.62-2.72(m, 1H), 4.19(d, 1H, J=14.7Hz), 4.98(d, 1H, J=16.1Hz), 5.16(bs, 1H), 5.72(s, 2H), 6.58-6.76(m, 2H), 7.07(s, 1H), 7.36-7.45(m, 1H), 7.69(s, 2H), 8.05(s, 1H) | KBr: 3405, 1616, 1502, 1275, 1107, 1078, 1012, 968 | OIL |

Table 12-2

| EX. | Ar | $R^1$ | $R^2$ | $R^3$ | $R^4$ | NMR ppm | IR $cm^{-1}$ | M.P. °C |
|---|---|---|---|---|---|---|---|---|
| 32 | 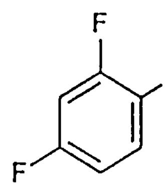 | 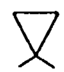 | | i-Pr | 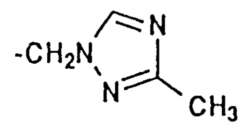 | $CDCl_3$: 0.66-0.85(m, 3H), 0.85-1.04(m, 1H), 0.95(d, 3H, J=6.8Hz), 0.99(d, 3H, J=6.8Hz), 2.50-2.72(m, 1H), 2.59(s, 3H), 4.32(d, 1H, J=15.1Hz), 4.86-4.96(m, 1H), 5.17(s, 1H), 5.88(s, 2H), 6.56-6.78(m, 2H), 7.28-7.44(m, 1H), 7.71(s, 1H), 7.82(s, 1H), 8.12(s, 1H) | KBr: 3396, 1616, 1500, 1277, 1142, 1030, 966 | OIL |
| 33 | 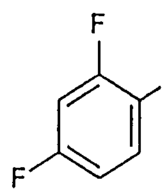 | 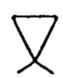 | | i-Pr | 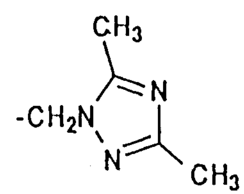 | $CDCl_3$: 0.67-0.85(m, 3H), 0.85-1.06(m, 1H), 0.94(d, 3H, J=6.8Hz), 1.00(d, 3H, J=7.3Hz), 2.30(s, 3H), 2.53(s, 3H), 2.55-2.70(m, 1H), 4.35(dd, 1H, J=1.5, 14.2Hz), 4.92(dd, 1H, J=1.5, 14.2Hz), 5.18(s, 1H), 5.78(d, 1H, J=11.7Hz), 5.79(d, 1H, J=11.7Hz), 6.58-6.76(m, 2H), 7.30-7.42(m, 1H), 7.71(s, 1H), 8.15(s, 1H) | neat: 3365, 1616, 1502, 1419, 1383, 1140, 1030, 1007, 966 | OIL |

Table 12-3

| EX. | Ar | R¹ | R² | R³ | R⁴ | NMR ppm | IR cm⁻¹ | M.P. °C |
|---|---|---|---|---|---|---|---|---|
| 34 | 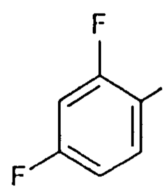 | 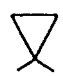 | | i-Pr | 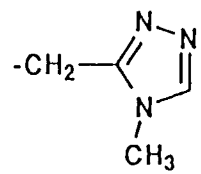 | CDCl3: 0.65-0.84(m, 3H), 0.84-1.06(m, 1H), 0.93(d, 3H, J=6.8Hz), 0.99(d, 3H, J=6.8Hz), 2.50-2.68(m, 1H), 3.72(s, 3H), 4.30-4.40(m, 1H), 4.98-5.10(m, 1H), 5.21(d, 1H, J=13.2Hz), 5.25(d, 1H, J=13.7Hz), 6.56-6.67(m, 1H), 6.69-6.79(m, 1H), 7.29-7.53(m, 1H), 7.71(s, 1H), 8.16(s, 1H), 8.18(d, 1H, J=1.0Hz) | KBr: 3034, 1616, 1529, 1500, 1273, 1140, 1001, 966 | OIL |
| 35 | 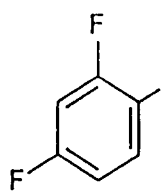 | 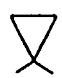 | | i-Pr | 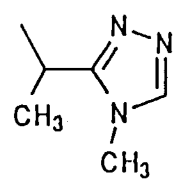 | CDCl3: 0.69-0.87(m, 3H), 0.90-1.14(m, 7H), 1.71-1.85(m, 3H), 2.56-2.73(m, 1H), 3.68(s, 3H), 4.24-4.42(m, 1H), 4.84-5.02(m, 1H), 5.17(bs, 1H), 5.30-5.41(m, 1H), 6.56-6.70(m, 1H), 6.70-6.84(m, 1H), 7.35-7.53(m, 1H), 7.70 and 7.72(s, 1H), 8.07(s, 1H), 8.13(d, 1H, J=2.0Hz) | KBr: 3396, 1616, 1500, 1273, 1140, 1107, 966 | OIL |

Table 12-4

| EX. | Ar | $R^1$ | $R^2$ | $R^3$ | $R^4$ | NMR ppm | IR $cm^{-1}$ | M.P. °C |
|---|---|---|---|---|---|---|---|---|
| 36 | 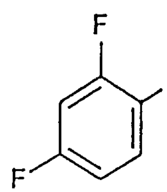 | 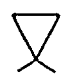 | | i-Pr | 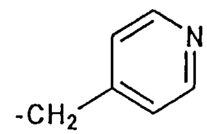 | $CDCl_3$: 0.73-0.94(m, 4H), 1.05(d, 3H, J=6.9Hz), 1.13(d, 3H, J=7.3Hz), 2.59-2.75(m, 1H), 4.33-4.40(m, 1H), 4.89-4.96(m, 1H), 5.08(s, 2H), 5.11(s, 1H), 6.62-6.78(m, 2H), 7.25(d, 2H, J=5.8Hz), 7.30-7.43(m, 1H), 7.70(s, 1H), 7.95(d, 1H, J=1.0Hz), 8.60(d, 2H, J=5.8Hz) | KBr: 3115, 1612, 1603, 1520, 1500, 1416, 1146, 860 | 99.2-109.0 |
| 37 | 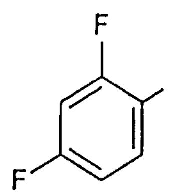 | 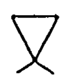 | | i-Pr | 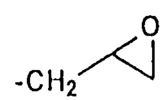 | $CDCl_3$: 0.73-1.12(m, 10H), 2.56-2.69(m, 2H), 2.81-2.87(m, 1H), 3.14-3.22(m, 1H), 3.89-3.98(m, 1H), 4.27-4.37(m, 1H), 4.46-4.58(m, 1H), 4.99-5.16(m, 1H), 5.25-5.31(m, 1H), 6.64-6.80(m, 2H), 7.48-7.57(m, 1H), 7.70(s, 1H), 8.12 and 8.18(s, 1H) | KBr: 3398, 1616, 1500, 1275, 1140, 1034, 968 | OIL |

Table 12-5

| EX. | Ar | $R^1$ | $R^2$ | $R^3$ | $R^4$ | NMR ppm | IR $cm^{-1}$ | M.P. °C |
|---|---|---|---|---|---|---|---|---|
| 38 | 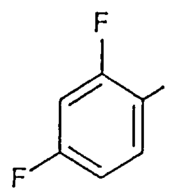 | 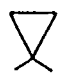 | | i-Pr | Et | $CDCl_3$: 0.78-0.96(m, 4H), 0.95(d, 3H, J=6.9Hz), 1.07(d, 3H, J=7.3Hz), 1.23(t, 3H, J=6.9Hz), 2.52-2.68(m, 1H), 4.06(q, 2H, J=6.9Hz)), 4.43-4.49(m, 1H), 4.99-5.05(m, 1H), 5.32(s, 1H), 6.65-6.79(m, 2H), 7.44-7.53(m, 1H), 7.70(s, 1H), 8.08(s, 1H) | KBr: 3400, 1616, 1500, 1275, 1140, 1041, 968 | OIL |
| 39 | 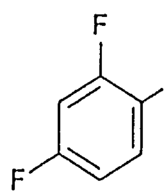 | 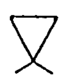 | | i-Pr | $CH_2CH_2OCH_3$ | $CDCl_3$: 0.75-0.93(m, 4H), 0.97(d, 3H, J=6.8Hz), 1.09(d, 3H, J=7.3Hz), 2.53-2.67(m, 1H), 3.37(s, 3H), 3.58(t, 2H, J=4.9Hz), 4.17(t, 2H, J=4.9Hz), 4.50(d, 1H, J=13.7Hz), 5.01(dd, 1H, J=1.5, 13.7Hz), 5.29(s, 1H), 6.65-6.80(m, 2H), 7.44-7.54(m, 1H), 7.70(s, 1H), 8.10(s, 1H) | KBr: 3250, 1610, 1502, 1144, 1055, 1026, 964 | 92.4-98.2 |

Table 12-6

| EX. | Ar | $R^1$ | $R^2$ | $R^3$ | $R^4$ | NMR ppm | IR $cm^{-1}$ | M.P. °C |
|---|---|---|---|---|---|---|---|---|
| 40 | 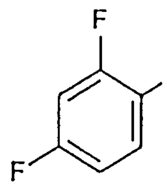 | 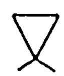 | | i-Pr | $CH_2CN$ | $CDCl_3$: 0.68-0.81(m, 2H), 0.84-1.01(m, 2H), 1.08(s, 3H), 1.10(s, 3H), 2.72-2.82(m, 1H), 4.52(dd, 1H, J=1.7, 14.2Hz), 4.69(s, 2H), 5.24-5.30(m, 1H), 5.26(s, 1H), 6.63-6.71(m, 1H), 6.76-6.83(m, 1H), 7.48-7.57(m, 1H), 7.73(s, 1H), 8.11(d, 1H, J=1.7Hz) | neat: 3396, 1616, 1502, 1275, 1142, 1039, 852 | OIL |
| 41 | 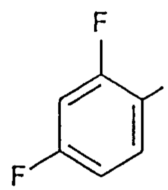 | 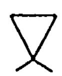 | | i-Pr | $CH_2SCH_3$ | $CDCl_3$: 0.77-1.02(m, 4H), 0.94(d, 3H, J=6.9Hz), 1.08(d, 3H, J=7.3Hz), 2.18(s, 3H), 2.53-2.66(m, 1H), 4.53(d, 1H, J=15.2Hz), 5.04(dd, 1H, J=1.7, 13.9Hz), 5.09(s, 2H), 5.31(s, 1H), 6.66-6.79(m, 2H), 7.48-7.57(m, 1H), 7.71(s, 1H), 8.14(s, 1H) | neat: 3386, 1616, 1498, 1140, 989, 968 | OIL |

Table 12-7

| EX. | Ar | $R^1$ | $R^2$ | $R^3$ | $R^4$ | NMR ppm | IR $cm^{-1}$ | M.P. °C |
|---|---|---|---|---|---|---|---|---|
| 42 | 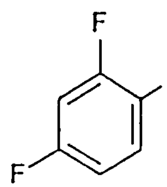 | 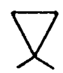 | | i-Pr | n-Pr | $CDCl_3$: 0.77-1.00(m, 4H), 0.93(t, 3H, J=7.6Hz), 0.94(d, 3H, J=6.9Hz), 1.07(d, 3H, J=6.9Hz), 1.58-1.71(m, 2H), 2.51-2.66(m, 1H), 3.94-3.99(m, 2H), 4.45(d, 1H, J=14.2Hz), 4.98-5.03(m, 1H), 5.31(s, 1H), 6.65-6.79(m, 2H), 7.44-7.53(m, 1H), 7.69(s, 1H), 8.07(s, 1H) | KBr: 3400, 1616, 1500, 1275, 1140, 1105, 985 | OIL |
| 43 | 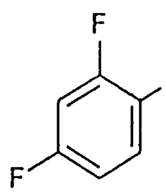 | 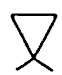 | | i-Pr | MEM | $CDCl_3$: 0.67-0.98(m, 4H), 0.99(d, 3H, J=6.8Hz), 1.09(d, 3H, J=7.3Hz), 2.55-2.74(m, 1H), 3.35(s, 3H), 3.48-3.60(m, 2H), 3.66-3.78(m, 2H), 4.44-4.59(m, 1H), 5.05(dd, 1H, J=2.0, 14.2Hz), 5.13(d, 1H, J=7.3Hz), 5.16(d, 1H, J=7.3Hz), 5.28(s, 1H), 6.60-6.80(m, 2H), 7.42-7.61(m, 1H), 7.71(s, 1H), 8.13(s, 1H) | KBr: 3394, 1616, 1500, 1275, 1140, 1107, 991 | OIL |

Example 44:

A solution of 1-[1-[1-(2,4-difluorophenyl)-1-hydroxy-2-(1H-1,2,4-triazol-1-yl)ethyl]cyclopropyl]-2-methyl-1-propanone oxime (200 mg) in 1,2-dibromoethane (0.4 ml) and toluene (1 ml) was added dropwise to an

emulsion of toluene (3 ml) and a 50% aqueous solution of sodium hydroxide (0.06 ml) at 20 °C over 5 min, and the mixture was stirred at the same temperature for 30 min. Thereafter, 1,2-dibromoethane (0.4 ml) and a 50% aqueous solution of sodium hydroxide (0.06 ml) were added dropwise and the mixture was stirred for 30 min.

The addition of the two reagents and the subsequent stirring were repeated two more times and water (3 ml) was added for extraction. Further extraction was carried out with ethyl acetate (5 ml × 2) and the organic layers were combined, washed with water (10 ml) and brine (10 ml) and dried over anhydrous sodium sulfate. The desiccant was filtered off and the solvent was concentrated under vacuum and the residue was purified by silica gel column chromatography (eluant: ethyl acetate/hexane = 1/3). The fractions containing the end product were collected and concentrated under vacuum to give 1-[1-[1-(2,4-difluorophenyl)-1-hydroxy-2-(1H-1,2,4-triazol-1-yl)ethyl]cyclopropyl]-2-methyl-1-propanone O-(2-bromoethyl)-oxime (150 mg; for its structure, see Fig. 11) as a colorless oil.

NMR ($CDCl_3$) δ [ppm]:

0.71-0.91 (m, 4H), 1.07 (d, 3H, J = 7.3Hz), 1.12 (d, 3H, J = 7.3Hz), 2,59-2.80 (m, 1H), 3.50 (t, 2H, J = 6.1Hz), 4.30 (t, 2H, J = 6.1Hz), 4.46 (dd, 1H, J = 1.5, 14.2Hz), 5.04-5.10 (m, 1H), 5.18 (s,1H), 6.64-6.81 (m, 2H), 7.47-7.56 (m, 1H), 7.71 (s, 1H), 8.06 (s, 1H)

IR (KBr) $cm^{-1}$:

3412, 1616, 1500, 1275, 1140, 1011, 968

Example 45:

Potassium t-butoxide (25.2 mg) was added to a solution of 1-[1-[1-(2,4-difluorophenyl)-1-hydroxy-2-(1H-1,2,4-triazol-1-yl)ethyl]cyclopropyl]-2-methyl-1-propanone O-(2-bromoethyl)oxime (0.10 g) and 1H-1,2,4-triazol (0.06 g) in dimethylformamide (2 ml) and the mixture was stirred at 80 - 85 °C for 1.5 h. More potassium t-butoxide (30 mg) was added and the mixture was stirred at the same temperature for 1 h.

The reaction mixture was poured into ice water and extraction was carried out with ethyl acetate (20 ml × 2). The organic layers were combined, washed with water (10 ml) and brine (10 ml) and dried over anhydrous sodium sulfate.

The desiccant was filtered off and the solvent was concentrated under vacuum and the residue was purified by silica gel column chromatography (eluant: ethyl acetate/hexane = 1/1). The fractions containing the end product were collected and concentrated under vacuum to give 1-[1-[1-(2,4-difluorophenyl)-1-hydroxy-2-(1H-1,2,4-triazol-1-yl)ethyl]cyclopropyl]-2-methyl-1-propanone O-[2-(1H-1,2,4-triazol-1-yl)ethyl]-oxime (72 mg; for its structure, see Fig. 12) as a colorless oil.

NMR ($CDCl_3$) δ [ppm]:

0.69-0.92 (m, 4H), 0.96 (d, 3H, J = 6.8Hz), 0.97 (d, 3H, J = 7.3Hz), 2.56-2.75 (m, 1H), 4.32-4.49 (m, 5H), 5.05 (dd, 1H, J = 2.0, 14.2Hz), 5.16 (s, 1H), 6.63-6.82 (m, 2H), 7.47-7.56 (m, 1H), 7.72 (s, 1H), 7.95 (s, 1H), 8.01 (s, 1H), 8.06 (d, 1H, J = 1.5Hz)

IR (KBr) $cm^{-1}$:

3431, 1616, 1502, 1275, 1140, 1043, 966

Examples 46 - 59:

The procedure of Example 30 was repeated to prepare the compounds shown in Figs. 12 and 13 and their physical data are listed in Table 13 (t-Bu, t-butyl group; i-Bu, isobutyl group; i-Pr, isopropyl group; n-Pr, n-propyl group; Et, ethyl group; MEM, methoxyethoxymethyl group).

Table 13-1

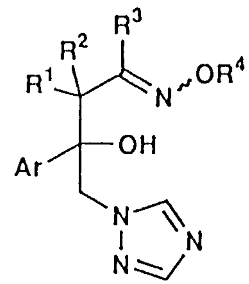

| EX. | Ar | $R^1$ | $R^2$ | $R^3$ | $R^4$ | NMR ppm | IR $cm^{-1}$ | M.P. °C |
|---|---|---|---|---|---|---|---|---|
| 46 | 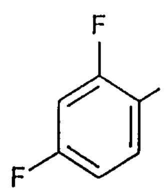 | 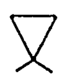 | | n-Pr | 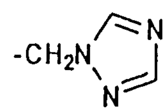 | $CDCl_3$: 0.62-1.05(m, 7H), 1.09-1.47(m, 2H), 1.53-1.65(m, 1H), 2.16(dt, 1H, J=4.9, 11.7Hz), 4.37(dd, 1H, J=1.5, 14.2Hz), 4.94-5.00(m, 1H), 5.26(s, 1H), 5.97(s, 2H), 6.56-6.64(m, 1H), 6.72-6.79(m, 1H), 7.42-7.51(m, 1H), 7.74(s, 1H), 8.02(s, 1H), 8.15(d, 1H, J=1.5Hz), 8.33(s,1H) | KBr: 3466, 1612, 1510, 1282, 1043, 1028 | 142.1-143.7 |

Table 13-2

| EX. | Ar | $R^1$ | $R^2$ | $R^3$ | $R^4$ | NMR ppm | IR $cm^{-1}$ | M.P. °C |
|---|---|---|---|---|---|---|---|---|
| 47 | 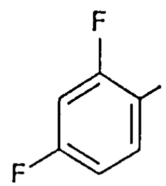 | 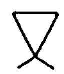 | | n-Pr | 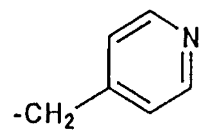 | $CDCl_3$: 0.66-1.04(m, 4H), 0.83(t, 3H, J=7.3Hz), 1.21-1.56(m, 2H), 1.70-1.80(m, 1H), 2.19-2.30(m, 1H), 4.38-4.43(m, 1H), 4.91(dd, 1H, J=1.5, 14.2Hz), 5.08(s, 2H), 5.21(s, 1H), 6.61-6.77(m, 2H), 7.22(d, 2H, J=5.9Hz), 7.35-7.44(m, 1H), 7.72(s, 1H), 7.92(s, 1H), 8.60(d, 2H, J=5.9Hz) | KBr: 3116, 1612, 1520, 1500, 1416, 1110, 1053, 970 | 112.2-114.8 |
| 48 | 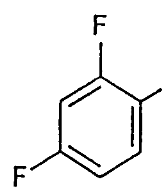 | 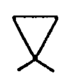 | | n-Pr | $CH_2SCH_3$ | $CDCl_3$: 0.70-1.08(m, 4H), 0.81(t, 3H, J=7.3Hz), 1.19-1.54(m, 2H), 1.62-1.74(m, 1H), 2.08-2.19(m, 1H), 2.19(s, 3H), 4.66(dd, 1H, J=1.5, 14.2Hz), 5.06-5.11(m, 1H), 5.11(s, 2H), 5.37(s, 1H), 6.65-6.82(m, 2H), 7.50-7.59(m, 1H), 7.75(s, 1H), 8.20(s, 1H) | KBr: 3170, 1612, 1510, 1500, 1269, 1043, 991, 968 | 65.0-82.1 |

Table 13-3

| EX. | Ar | $R^1$ | $R^2$ | $R^3$ | $R^4$ | NMR ppm | IR cm$^{-1}$ | M.P. °C |
|---|---|---|---|---|---|---|---|---|
| 49 | 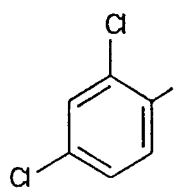 | 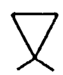 | | Et | 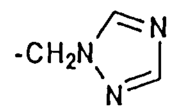 | $CDCl_3$: 0.79-0.91(m, 4H), 1.00-1.14(m, 3H), 1.82-1.95(m, 1H), 2.18-2.31(m, 1H), 4.24(d, 1H, J=14.2Hz), 5.26(bs, 1H), 5.32(d, 1H, J=14.2Hz), 5.92(s, 2H), 7.04(dd, 1H, J=2.3, 8.6Hz), 7.21(d, 1H, J=2.3Hz), 7.40(d, 1H, J=8.9Hz), 7.77(s, 1H), 8.01(s, 1H), 8.30(s, 1H), 8.34(s, 1H) | KBr: 3409, 1581, 1512, 1279, 1142, 1028, 860 | 125.0-128.0 |
| 50 | 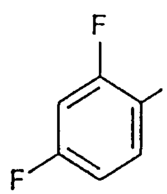 | 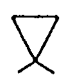 | | Et | Et | $CDCl_3$: 0.71-1.03(m, 4H), 0.90(t, 3H, J=7.6Hz), 1.24(t, 3H, J=6.9Hz), 1.70-1.83(m, 1H), 2.14-2.27(m, 1H), 4.10(q, 2H, J=6.9Hz), 4.55(dd, 1H, J=1.3, 13.9Hz), 5.04-5.10(m, 1H), 5.38(bs, 1H), 6.63-6.82(m, 2H), 7.47-7.56(m, 1H), 7.74(s, 1H), 8.13(s, 1H) | KBr: 3105, 1612, 1500, 1109, 1041, 968 | 86.5-87.5 |

Table 13-4

| EX. | Ar | $R^1$ | $R^2$ | $R^3$ | $R^4$ | NMR ppm | IR $cm^{-1}$ | M.P. °C |
|---|---|---|---|---|---|---|---|---|
| 51 | 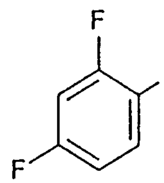 | 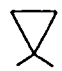 | | Et | -$CH_2$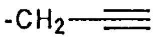 | *$CDCl_3$: 0.67-1.09(m, 7H), 1.46-2.36(m, 2H), 2.46(t, 3H, J=2.3Hz), 4.65(d, 2H, J=2.3Hz), 4.75(dd, 1H, J=1.3, 14.2Hz), 5.06(dd, 1H, J=1.3, 14.2Hz), 5.26(s, 1H), 6.53-6.91(m, 2H), 7.40-7.72(m, 1H), 7.76(s, 1H), 8.21(s, 1H) | KBr: 3288, 1612, 1518, 1500, 1109, 1039, 970 | 97.6-100.2 |
| 52 | 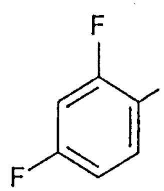 | 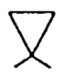 | | Et | $CH_2CN$ | *$CDCl_3$: 0.61-1.15(m, 7H), 1.46-1.90(m, 1H), 2.06-2.50(m, 1H), 4.40-4.66(m, 1H), 4.72(s, 2H), 5.21-5.48(m, 1H), 5.29(s, 1H), 6.47-6.94(m, 2H), 7.43-7.82(m, 1H), 7.76(s, 1H), 8.16(d, 1H, J=1.7Hz) | KBr: 3134, 1616, 1500, 1412, 1138, 1063, 970 | 104.3-105.4 |
| 53 | 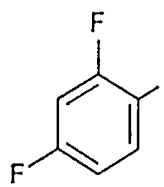 | 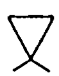 | | Et | $CH_2SCH_3$ | $CDCl_3$: 0.73-1.26(m, 4H), 0.92(t, 3H, J=7.6Hz), 1.70-1.83(m, 1H), 2.12-2.25(m, 1H), 2.20(s, 3H), 4.69-4.75(m, 1H), 5.10-5.15(m, 1H), 5.13(s, 2H), 6.65-6.82(m, 2H), 7.49-7.58(m, 1H), 7.82(s, 1H), 8.45(s, 1H) | KBr: 3132, 1612, 1500, 1147, 1109, 968 | 100.6-103.2 |

Table 13-5

| EX. | Ar | $R^1$ | $R^2$ | $R^3$ | $R^4$ | NMR ppm | IR $cm^{-1}$ | M.P. °C |
|---|---|---|---|---|---|---|---|---|
| 54 | 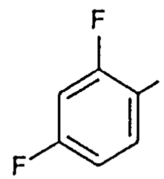 | 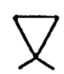 | | Et | 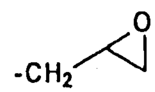 | $CDCl_3$: 0.70-1.02(m, 7H), 1.61-2.31(m, 2H), 2.61-3.41(m, 2H), 3.88-4.02(m, 1H), 4.28-4.51(m, 1H), 4.58-4.71(m, 1H), 5.06-5.22(m, 1H), 5.29-5.38(m, 1H), 6.62-6.79(m, 2H), 7.47-7.61(m, 1H), 7.67-7.75(m, 1H), 8.10-8.28(m, 1H) | neat: 3394, 1616, 1500, 1419, 1275, 1140, 968 | OIL |
| 55 | 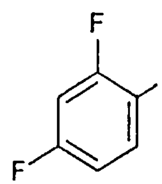 | 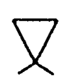 | | Et | $CH_2CH_2Br$ | *$CDCl_3$: 0.68-1.12(m, 7H), 1.62-2.44(m, 2H), 3.50(t, 2H, J=5.9Hz), 4.33(t, 2H, J=5.9Hz), 4.39-4.64(m, 1H), 5.01-5.26(m, 1H), 5.21(s, 1H), 6.53-6.93(m, 2H), 7.39-7.73(m, 1H), 7.74(s, 1H), 8.08(d, 1H, J=0.9Hz) | KBr: 3113, 1612, 1500, 1109, 1030, 970, 860 | 91.2-93.5 |
| 56 | 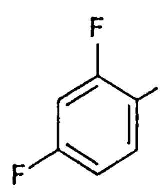 | 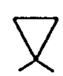 | | Et | n-Bu | $CDCl_3$: 0.74-1.04(m, 7H), 0.89(t, 3H, J=7.6Hz), 1.21-1.82(m, 5H), 2.18-2.26(m, 1H), 4.04(t, 2H, J=6.6Hz), 4.53(d, 1H, J=14.5Hz), 5.05(d, 1H, J=13.9Hz), 5.38(s, 1H), 6.63-6.81(m, 2H), 7.41-7.55(m, 1H), 7.73(s, 1H), 8.12(s, 1H) | KBr: 3107, 1612, 1518, 1500, 1109, 968 | 65.3-73.8 |

Table 13-6

| EX. | Ar | $R^1$ | $R^2$ | $R^3$ | $R^4$ | NMR ppm | IR $cm^{-1}$ | M.P. °C |
|---|---|---|---|---|---|---|---|---|
| 57 | 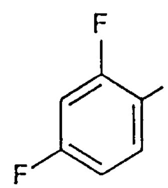 | 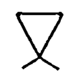 | | Et | MEM | $CDCl_3$: 0.70-1.06(m, 4H), 0.92(t, 3H, J=7.6Hz), 1.69-1.82(m, 1H), 2.18-2.31(m, 1H), 3.36(s, 3H), 3.55-3.59(m, 2H), 3.66-3.82(m, 2H), 4.66-4.72(m, 1H), 5.06-5.12(m, 1H), 5.16(d, 1H, J=7.3Hz), 5.22(d, 1H, J=7.3Hz), 5.36(bs, 1H), 6.64-6.72(m, 1H), 6.76-6.83(m, 1H), 7.51-7.60(m, 1H), 7.79(s, 1H), 8.32(s, 1H) | KBr: 3115, 1614, 1500, 1109, 1011, 968 | 81.2-87.1 |
| 58 | 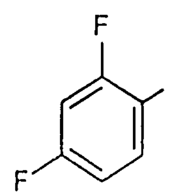 | 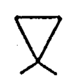 | | Et | 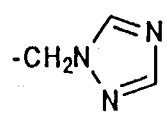 | $CDCl_3$: 0.63-1.04(m, 4H), 0.85(t, 3H, J=7.6Hz), 1.54-1.65(m, 1H), 2.16-2.29(m, 1H), 4.38(dd, 1H, J=1.7, 14.2Hz), 4.99(dd, 1H, J=2.0, 14.2Hz), 5.26(s, 1H), 5.99(s, 2H), 6.54-6.63(m, 1H), 6.73-6.80(m, 1H), 7.43-7.53(m, 1H), 7.75(s, 1H), 8.03(s, 1H) 8.15(d, 1H, J=1.3Hz), 8.34(s, 1H) | KBr: 3400, 1614, 1510, 1144, 1030, 968 | 110.0-121.7 |

Table 13-7

| EX. | Ar | $R^1$ | $R^2$ | $R^3$ | $R^4$ | NMR ppm | IR $cm^{-1}$ | M.P. °C |
|---|---|---|---|---|---|---|---|---|
| 59 | 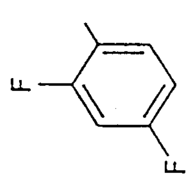 | 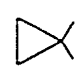 | | Et | 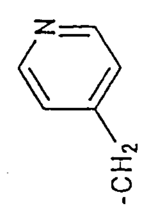 | $CDCl_3$: 0.68-1.01(m, 7H), 1.78-1.91(m, 1H), 2.27-2.42(m, 1H), 3.10(bs, 1H), 4.40-4.46(m, 1H), 4.96(dd, 1H, J=1.7, 14.2Hz), 5.15(s, 2H), 5.19(s, 1H), 6.61-6.80(m, 2H), 7.36(d, 2H, J=5.9Hz), 7.35-7.55(m, 1H), 7.74(s, 1H), 7.95(d, 1H, J=1.0Hz), 8.63(d, 2H, J=5.6Hz) | KBr: 3116, 1612, 1605, 1500, 1416, 1109, 1041, 970, 862 | 116.3-127.0 |

Example 60:

A mixture of the compound (75 mg) prepared in Example 55 and 4-methylpiperazine (850 mg) was stirred overnight at room temperature under a nitrogen atmosphere. The reaction mixture was concentrated

under vacuum and purified by silica gel column chromatography [eluant: ethyl acetate/hexane = 1/1 to 5% triethylamine and (methylene chloride/methanol = 9/1)].

The fractions containing the end product were collected and concentrated under vacuum to give 1-[1-[1-(2,4-difluorophenyl)-1-hydroxy-2-[1H-1,2,4-triazol-1-yl)ethyl]cyclopropyl]-1-propanone O-[2-(4-methylpiperazin-1-yl)ethyl]oxime (72 mg; for its structure, see Fig. 13) as a pale brown crystal.

NMR ($CDCl_3$) δ [ppm]:

0.72-1.01 (m, 4H), 0.90 (t, 3H, J = 7.6Hz), 1.70-1.83 (m, 1H), 2.14-2.27 (m, 1H), 2.36 (s, 3H), 2.45-2.73-(m, 8H), 2.68 (t, 2H, J = 5.8Hz), 4.19 (t,2H,J = 5.8Hz), 4.53 (dd, 1H, J = 1.5, 12.5Hz), 5.06 (dd, 1H, J = 1.5, 12.5Hz), 5.33 (s, 1H), 6.62-6.83 (m, 2H), 7.45-7.56 (m, 1H), 7.74 (s, 1H), 8.12 (d, 1H, J = 1.0Hz)

IR (KBr) $cm^{-1}$:

3105, 1612, 1500, 1279, 1144, 1109, 1039, 968

m.p.: 80.9-84.9 °C

Example 61:

Pyridine (256 mg) and O-ethyl hydroxylamine hydrochloride (316 mg) were added to a solution of 4-(2,4-difluorophenyl)-4-hydroxy-5-(1H, 1,2,4-triazol-1-yl)-3,3-dimethyl-2-pentanone (200 mg) in ethanol (2.50 ml) at room temperature and the mixture was heated under reflux for 3 h.

The reaction mixture was concentrated under vacuum and both ethyl acetate (30 ml) and 1 N HCl (20 ml) were added for extraction. Further extraction was conducted with ethyl acetate (30 ml) and the organic layers were combined, washed with 1 N HCl (10 ml), water (10 ml) and brine (10 ml) and dried over anhydrous sodium sulfate.

The desiccant was filtered off and the solvent was concentrated under vacuum and the precipitating crystal grains were collected, followed by washing with hexane to give 4-(2,4-difluorophenyl)-4-hydroxy-5-(1H-1,2,4-triazol-1-yl)-3,3-dimethyl-2-pentanone O-ethyl oxime (184 mg; see Fig. 13) as a white crystal.

NMR ($CDCl_3$) δ [ppm]:

1.16 (s, 3H), 1.21 (d, 3H, J = 3.0Hz), 1.27 (t, 3H, J = 6.9Hz), 1.89 (s, 3H), 4.09-4.16 (m, 2H), 4.36-4.42 (m, 1H), 5.29 (dd, 1H, J = 2.6, 14.2Hz), 5.48 (s, 1H), 6.59-6.67 (m, 1H), 6.75-6.83 (m, 1H), 7.56-7.66 (m, 1H), 7.72 (s, 1H), 8.09 (s, 1H)

IR (KBr) $cm^{-1}$:

3174, 1612, 1502, 1273, 1103, 1047, 947

m.p.: 110.0-112.6 °C

Examples 62 - 71:

The procedure of Example 30 was repeated to prepare the compounds shown in Figs. 13 and 14 and their physical data are listed in Table 14 (t-Bu, t-butyl group; i-Bu, isobutyl group; i-Pr, isopropyl group; n-Pr, n-propyl group; Et, ethyl group; MEM, methoxyethoxymethyl group).

Table 14-1

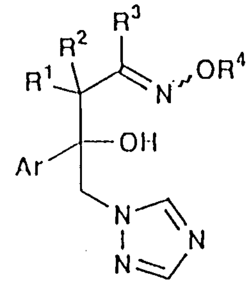

| EX. | Ar | $R^1$ | $R^2$ | $R^3$ | $R^4$ | NMR ppm | IR $cm^{-1}$ | M.P. °C |
|---|---|---|---|---|---|---|---|---|
| 62 | 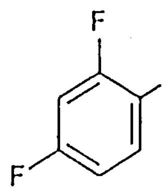 | $CH_3$ | $CH_3$ | $CH_3$ | 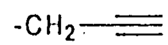 | *$CDCl_3$: 1.19-1.23(m, 6H), 1.92(s, 3H), 2.43(t, 1H, J=2.3Hz), 4.34-4.51(m, 1H), 4.68(d, 2H, J=2.3Hz), 5.27-5.39(m, 1H), 5.39(s, 1H), 6.52-6.87(m, 2H), 7.48-7.80(m, 1H), 7.71(s, 1H), 8.07(d, 1H, J=2.0Hz) | KBr: 3290, 1612, 1500, 1273, 1105, 1045, 968 | 81.0-82.2 |
| 63 | 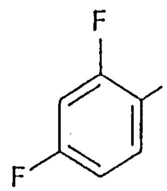 | $CH_3$ | $CH_3$ | $CH_3$ | $CH_2CN$ | $CDCl_3$: 1.20(s, 3H), 1.22(s, 3H), 1.93(s, 3H), 4.31-4.37(m, 1H), 4.73(s, 2H), 5.38(s, 1H), 5.36-5.42(m, 1H), 6.58-6.66(m, 1H), 6.77-6.84(m, 1H), 7.59-7.68(m, 1H), 7.71(s, 1H), 8.09(d, 1H, J=2.3Hz) | KBr: 3178, 1612, 1500, 1146, 1105, 1065, 852 | 99.0-100.5 |

Table 14-2

| EX. | Ar | $R^1$ | $R^2$ | $R^3$ | $R^4$ | NMR ppm | IR $cm^{-1}$ | M.P. °C |
|---|---|---|---|---|---|---|---|---|
| 64 | 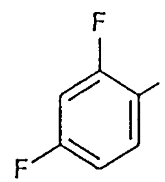 | $CH_3$ | $CH_3$ | $CH_3$ | $CH_2SCH_3$ | $CDCl_3$: 1.20(s, 3H), 1.21(d, 3H, J=3.0Hz), 1.90(s, 3H), 2.24(s, 3H), 4.38-4.44(m, 1H), 5.17(s, 2H), 5.34(dd, 1H, J=2.6, 14.2Hz), 5.40(s, 1H), 6.58-6.67(m, 1H), 6.75-6.83(m, 1H), 7.58-7.67(m, 1H), 7.71(s, 1H), 8.09(d, 1H, J=2.3Hz) | KBr: 3188, 1612, 1500, 1146, 1003, 854 | 95.7-99.6 |
| 65 | 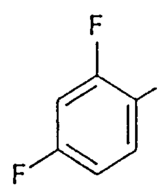 | $CH_3$ | $CH_3$ | $CH_3$ | $CH_2OCH_3$ | *$CDCl_3$: 1.19-1.24(m, 6H), 1.95(s, 3H), 3.43(s, 3H), 4.36(dd, 1H, J=2.0, 14.2Hz), 5.12(s, 2H), 5.23-5.41(m, 1H), 5.41(s, 1H), 6.49-6.86(m, 2H), 7.48-7.75(m, 1H), 7.71(s, 1H), 8.08(d, 1H, J=2.3Hz) | KBr: 3201, 1612, 1502, 1142, 1105, 1014 | 88.1-88.8 |
| 66 | 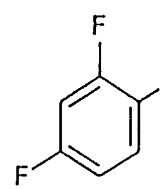 | $CH_3$ | $CH_3$ | $CH_3$ | $CH_2CH_2Br$ | *$CDCl_3$: 1.11-1.30(m, 6H), 1.95(s; 3H), 3.52(t, 2H, J=6.3Hz), 4.33(t, 2H, J=6.3Hz), 4.23-4.46(m, 1H), 5.16-5.40(m, 1H), 5.34(s, 1H), 6.47-6.91(m, 2H), 7.44-7.76(m, 1H), 7.71(s, 1H), 8.02-8.11(m, 1H) | KBr: 3431, 1612, 1518, 1500, 1146, 1105, 679 | 101.8-104.9 |

Table 14-3

| EX. | Ar | $R^1$ | $R^2$ | $R^3$ | $R^4$ | NMR ppm | IR $cm^{-1}$ | M.P. °C |
|---|---|---|---|---|---|---|---|---|
| 67 | 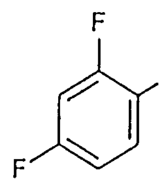 | $CH_3$ | $CH_3$ | $CH_3$ | 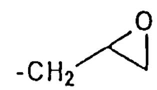 | *$CDCl_3$: 1.10-1.28(m, 6H), 1.90 and1.94(s, 3H), 2.56-3.34(m, 3H), 3.85-4.52(m, 3H), 5.20-5.48(m, 2H), 6.47-6.91(m, 1H), 6.76(s, 1H), 7.47-7.79(m, 1H), 7.70(s, 1H), 8.04-8.16(m, 1H) | KBr: 3190, 1612, 1518, 1500, 1273, 1146, 1105, 1043, 966 | 67.8-73.8 |
| 68 | 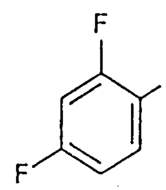 | $CH_3$ | $CH_3$ | $CH_3$ | n-Bu | *$CDCl_3$: 0.78-1.92(m, 13H), 1.88(s, 3H), 4.07(t, 2H, J=6.3Hz), 4.30-4.50(m, 1H), 5.20-5.40(m, 1H), 5.47(s, 1H), 6.48-6.89(m, 2H), 7.47-7.74(m, 1H), 7.71(s, 1H), 8.07(d, 1H, J=2.0Hz) | KBr: 3176, 1612, 1500, 1146, 1105, 1045 | 95.0-97.0 |
| 69 | 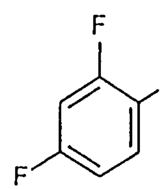 | $CH_3$ | $CH_3$ | $CH_3$ | MEM | *$CDCl_3$: 1.19-1.22(m, 6H), 1.94(s, 3H), 3.30(s, 3H), 3.49-3.58(m, 2H), 3.72-3.82(m, 2H), 4.28-4.47(m, 1H), 5.21(d, 1H, J=7.6Hz), 5.24(d, 1H, J=2.3Hz), 5.27-5.57(m, 1H), 5.38(s, 1H), 6.49-6.89(m, 2H), 7.49-7.77(m, 1H), 7.70(s, 1H), 8.09(d, 1H, J=2.3Hz) | KBr: 3180, 1614, 1502, 1105, 1012, 876 | 57.6-60.3 |

Table 14-4

| EX. | Ar | $R^1$ | $R^2$ | $R^3$ | $R^4$ | NMR ppm | IR $cm^{-1}$ | M.P. °C |
|---|---|---|---|---|---|---|---|---|
| 70 | 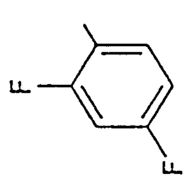 | $CH_3$ | $CH_3$ | $CH_3$ | 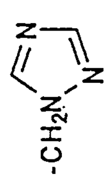 | *$CDCl_3$: 1.08-1.26(m, 6H), 1.90(s, 3H), 3.99-4.22(m, 1H), 4.97-5.22(m, 1H), 5.33(s, 1H), 5.97(s, 2H), 6.42-6.88(m, 2H), 7.38-7.73(m, 1H), 7.69(s, 1H), 7.95(s, 1H), 8.03(d, 1H, J=2.3Hz), 8.28(s, 1H) | KBr: 3402, 1612, 1510, 1277, 1144, 1036, 868 | 120.8-123.0 |
| 71 | 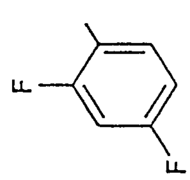 | $CH_3$ | $CH_3$ | $CH_3$ | 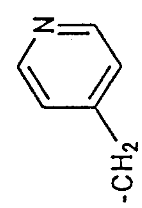 | $CDCl_3$: 1.15(s, 6H), 1.98(s, 3H), 4.21-4.26(m, 1H), 5.09-5.15(m, 1H), 5.11(s, 2H), 5.34(s, 1H), 6.55-6.64(m, 1H), 6.73-6.80(m, 1H), 7.26(d, 2H, J=5.9Hz), 7.54-7.63(m, 1H), 7.70(s, 1H), 7.97(d, 1H, J=2.3Hz), 8.57(d, 2H, J=5.3Hz) | KBr: 3182, 1612, 1506, 1416, 1144, 968 | 78.0-80.6 |

Example 72:

Three drops of 10% HCl-methanol were added to a suspension of 1-[1-[1-(2,4-difluorophenyl)-1-hydroxy-2-(1H-1,2,4-triazol-1-yl)ethyl]cyclopropyl]-2-methyl-1-propanone oxime (90.1 mg) in methyl orthoformate (0.28 ml) at room temperature and the mixture was stirred at the same temperature for 1.5 h.

A saturated aqueous solution of sodium hydrogencarbonate (5 ml) was added to the stirred reaction mixture and extraction was carried out with ethyl acetate (10 ml × 2). The organic layers were combined, washed with water (10 ml) and brine (10 ml) and dried over anhydrous sodium sulfate.

The desiccant was filtered off and the solvent was concentrated under vacuum and the residue was purified by silica gel column chromatography (eluant: 2% methanolmethylene chloride). The fractions containing the end product were collected and concentrated under vacuum to give 1-[1-[1-(2,4-difluorophenyl)-1-hydroxy-2-(1H-1,2,4-triazol-1-yl)ethyl]cyclopropyl]-2-methyl-1-propanone O-(dimethoxymethyl)oxime (40.0 mg; see Fig. 14) as a colorless crystal.

NMR ($CDCl_3$) δ [ppm]:

0.65-0.98 (m, 4H), 1.05 (d, 3H, J=7.3Hz), 1.12 (d, 3H, J=6.8Hz), 2.57-2.76 (m, 1H), 3.38 (s, 3H), 3.39 (s, 3H), 4.48-4.61 (m, 1H), 5.04 (dd, 1H, J=1.5, 14.2Hz), 5.26 (s, 1H), 5.55 (s, 1H), 6.64-6.82 (m, 2H), 7.47-7.59 (m, 1H), 7.72 (s, 1H), 8.11 (s, 1H)

IR (KBr) $cm^{-1}$:

3267, 1612, 1500, 1275, 1198, 1144, 987

m.p.: 101.3-103.0 °C

Example 73:

The procedure of Example 72 was repeated using methyl orthoacetate in place of methyl orthoformate, whereby 1-[1-[1-(2,4-difluorophenyl)-1-hydroxy-2-(1H-1,2,4-triazol-1-yl)ethyl]cyclopropyl]-2-methyl-1-propanone O-(1-dimethoxyethyl)oxime (see Fig. 14) was obtained as a colorless oil.

NMR ($CDCl_3$) δ [ppm]:

0.72-0.92 (m, 4H), 0.97 (d, 3H, J=6.8Hz), 1.09 (d, 3H, J=7.3Hz), 1.56 (s, 3H), 2.50-2.67 (m, 1H), 3.30 (s, 3H), 3.32 (s, 3H), 4.58 (d, 1H, J=14.2Hz), 4.92-5.02 (m, 1H), 5.30 (s, 1H), 6.64-6.82 (m, 2H), 7.45-7.57 (m, 1H), 7.72 (s, 1H), 8.15 (s, 1H)

IR (KBr) $cm^{-1}$:

3398, 1616, 1500, 1142, 1107, 966

Example 74:

Anhydrous magnesium sulfate (51.4 mg) was added to a suspension of 1-[1-[1-(2,4-difluorophenyl)-1-hydroxy-2-(1H-1,2,4-triazol-1-yl)ethyl]cyclopropyl]-2-methyl-1-propanone oxime (90.1 mg) in isopropenyl methyl ether (1.5 ml) at room temperature; thereafter, p-toluenesulfonic acid monohydrate (1.3 mg) was added under cooling with ice in water, and the mixture was stirred at the same temperature for 1 h, then at room temperature for 3 h.

A saturated aqueous solution of sodium hydrogencarbonate (20 ml) was added to the stirred reaction mixture and extraction was conducted with ethyl acetate (20 ml × 2). The organic layers were combined, washed with water (10 ml) and brine (10 ml) and dried over anhydrous sodium sulfate.

The desiccant was filtered off and the solvent was concentrated under vacuum and the residue was purified by silica gel column chromatography (eluant: methanol/methylene chloride = 1/50). The fractions containing the end product were collected and concentrated under vacuum to give 1-[1-[1-(2,4-difluorophenyl)-1-hydroxy-2-(1H-1,2,4-triazol-1-yl)ethyl]cyclopropyl]-2-methyl-1-propanone O-(1-methoxy-1-methylethyl)oxime (95.0 mg; see Fig. 14) as a colorless oil.

NMR ($CDCl_3$) δ [ppm]:

0.64-0.88 (m, 3H), 0.91 (d, 3H, J=7.3Hz), 0.95-1.04 (m, 1H), 1.06 (d, 3H, J=7.3Hz), 1.42 (s, 3H), 1.44 (s, 3H), 2.42-2.61 (m, 1H), 3.20 (s, 3H), 4.53 (d, 1H, J=13.7Hz), 4.90-5.00 (m, 1H), 5.38 (bs, 1H), 6.67-6.81 (m, 2H), 7.43-7.56 (m, 1H), 7.70 (s, 1H), 8.15 (s, 1H)

IR (KBr) $cm^{-1}$:

3400, 1616, 1500, 1273, 1215, 1107, 1072, 966

Example 75:

The procedure of Example 74 was repeated using methyl vinyl ether in place of isopropenyl methyl ether, whereby 1-[1-[1-(2,4-difluorophenyl)-1-hydroxy-2-(1H-1,2,4-triazol-1-yl)ethyl]cyclopropyl]-2-methyl-1-propanone O-(1-methoxyethyl)oxime (see Fig. 14) was obtained as a colorless oil.

NMR ($CDCl_3$) δ [ppm]:

0.73-1.15 (m, 6H), 0.98 (d, 3H, J=6.8Hz), 1.24-1.45 (m, 1H), 1.37 and 1.39 (s, 1H), 2.54-2.72 (m, 1H),

3.29-3.42 (m, 3H), 4.48 (d, 1H, J = 13.7Hz), 4.80-5.46 (m, 2H), 5.26 and 5.28 (s, 1H), 6.63-6.80 (m, 2H), 7.44-7.55 (m, 1H), 7.71 (s, 1H), 8.08 and 8.10 (s, 1H)
IR (KBr) $cm^{-1}$:
3394, 1616, 1500, 1275, 1142, 1105, 966

Example 76:

The procedure of Example 30 was repeated using bromomethyl ethyl ether in place of (1H-1,2,4-triazol-1-yl)methylchloride hydrochloride, whereby 1-[1-[1-(2,4-difluorophenyl)-1-hydroxy-2-(1H-1,2,4-triazol-1-yl)-ethyl]cyclopropyl]-2-methyl-1-propanone O-(ethoxymethyl)oxime (see Fig. 14) was obtained as a colorless crystal.
NMR ($CDCl_3$) δ [ppm]:
0.68 = 1.03 (m, 4H), 0.96 (d, 3H, J = 7.3Hz), 1.08 (d, 3H, J = 6.8Hz), 1.13-1.26 (m, 3H), 2.53-2.72 (m, 1H), 3.50-3.68 (m, 2H), 4.45-4.64 (m, 1H), 4.96-5.05 (m, 1H), 5.08 (d, 1H, J = 7.3Hz), 5.11 (d, 1H, J = 7.3Hz), 5.30 (s, 1H), 6.65-6.79 (m, 2H), 7.45-7.55 (m, 1H), 7.70 (s, 1H), 8.11 (s, 1H)
IR (KBr) $cm^{-1}$:
3269, 1610, 1500, 1195, 1106, 1051, 993
m.p.: 107.6-118.6 °C

Formulation Examples

Examples of formulating pharmaceutical preparations containing selected compounds of the invention will now be described under A - E but it should be understood that the scope of the invention is by no means limited to those examples.

Example A: Tablet

| | |
|---|---|
| Compound of Example 4 | 100 g |
| Polyethylene glycol 6000 | 100 g |
| Sodium lauryl sulfate | 15 g |
| Cornstarch | 30 g |
| Lactose | 250 g |
| Magnesium stearate | 5 g |

The ingredients listed above were weighed; thereafter, polyethylene glycol 6000 was warmed to between 70 and 80 °C and the compound of the invention, sodium lauryl sulfate, cornstarch and lactose were added, followed by mixing. The mixture was cooled as such and the solidifying mixture was granulated by means of a comminuting mill. The granules were then mixed with magnesium stearate and compressed into tablets each weighing 250 mg.

Example B: Injection

| | |
|---|---|
| Compound of Example 30 | 1 g |
| Sodium chloride | 9 g |
| Sterile water for injection | 1000 ml |

The ingredients listed above were weighed and the first two were dissolved in the bacteriostatic water for injection. After filtration for sterilizing, 5-ml portions of the solution were distributed in 10-ml ampules, which were fused to formulate injections.

Example C: Capsule

| | |
|---|---|
| Compound of Example 6 | 100 g |
| Lactose | 250 g |
| Cornstarch | 50 g |
| Microcrystalline cellulose | 95 g |
| Magnesium stearate | 5 g |

The ingredients listed above were weighed and all except magnesium stearate were mixed uniformly. After addition of magnesium stearate, mixing was continued for several minutes. Metered amounts (250 mg) of the powder mixture were charged into hard capsules (No. 1) on an encapsulating machine so as to formulate capsules.

Example D: Tablet

| | |
|---|---|
| Compound of Example 1 | 200 g |
| Polyethylene glycol 6000 | 100 g |
| Sodium lauryl sulfate | 15 g |
| Cornstarch | 30 g |
| Lactose | 150 g |
| Magnesium stearate | 5 g |

The ingredients listed above were weighed; thereafter, polyethylene glycol 6000 was warmed to between 70 and 80 °C and the compound of the invention, sodium lauryl sulfate, cornstarch and lactose were added, followed by mixing. The mixture was cooled as such and the solidifying mixture was granulated by means of a communicating mill. The granules were then mixed with magnesium stearate and compressed into tablets each weighing 250 mg.

Example E: Suppository

| | |
|---|---|
| Compound of Example 2 | 50 g |
| Polyethylene glycol 1500 | 200 g |
| Polyethylene glycol 4000 | 200 g |

The compound of Example 2 was put in a mortar and ground thoroughly into a fine powder, which was melt-molded to formulate suppositories each weighing 1 g.

Thus, the triazole derivatives of the invention have been found to possess the following features: they have a broad spectrum of antimycotic activity: they exhibit an outstanding in vitro antimycotic activity against Aspergillus species; they exhibit a marked therapeutic effect in an animal experiment using Aspergillus infected in vivo laboratory animal models (mice); they exhibit a marked therapeutic effect in an animal experiment using Candida infected in vivo laboratory animal models (mice); they also exhibit a marked therapeutic effect in an animal experiment using immunocompromised and Candida infected in vivo laboratory animal models (mice); they have good enough solubility to be useful as injections; and they are highly safe to use. The triazole derivatives of the invention have been found to excel in at least one of these features.

Therefore, the triazole derivatives of the invention are anticipated to be extremely useful in the treatment of various superficial dermatomycoses (e.g. favus such as tinea corporis, tinea cruris, tinea manus, tinea pedis, tinea capitis, Celsus' kerion and tinea sycosis; candidosis such as buccal candidosis, dermal candidosis, candida sycosis and chronic mucocutaneous candidosis; tinea versicolor and Malassezia folliculitis), deep dermatomycoses (e.g. sporotrichosis and chromomycosis), and deep mycoses (mycoses in internal organs, e.g. fungemia, mycoses in respiratory organs, mycoses in digestive tracts, mycoses in urinary tracts and fungal meningitis).

## Claims

1. A compound represented by the following formula (I):

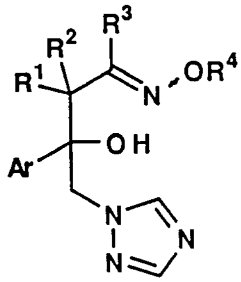

(I)

(where Ar is a phenyl group substituted by one or two halogen atoms; $R^1$ and $R^2$ are each a methyl group or, when taken together with the adjacent carbon atom, represent a cyclopropylidene group; $R^3$ is a straight-chained, branched or cyclic alkyl group having 1 - 4 carbon atoms; $R^4$ is a hydrogen atom, a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group; and the wavy line represents an E- or Z-type bond) or salts thereof.

2. A compound according to claim 1 wherein $R^3$ is an n-propyl or an isopropyl group.

3. A compound according to claim 2 wherein Ar is a 2,4-difluorophenyl group and $R^1$ and $R^2$ form a cyclopropylidene group as taken together with the adjacent carbon atom.

4. A compound according to claim 3 wherein $R^4$ is a methyl group, a methoxymethyl group or a 1H-(1,2,4-triazol-1-yl)methyl group.

5. A compound according to any one of claims 1 - 4 wherein the bond represented by the wavy line is of the E-type bond.

6. A process for producing a compound of following formula (Ia):

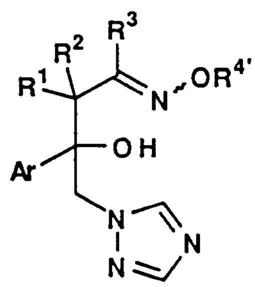

(Ia)

(where Ar is a phenyl group substituted by one or two halogen atoms; $R^1$ and $R^2$ are each a methyl group or, when taken together with the adjacent carbon atom, represent a cyclopropylidene group; $R^3$ is a straight-chained, branched or cyclic alkyl group habving 1 - 4 carbon atoms; $R^{4'}$ is a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group; and the wavy line represents an E- or Z-type bond) or a salt thereof by reacting an oxime derivative of the formula (II):

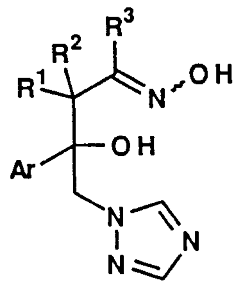

(II)

(where Ar is a phenyl group substituted by one or two halogen atoms; $R^1$ and $R^2$ are each a methyl group or, when taken together with the adjacent carbon atom, represent a cyclopropylidene group; $R^3$ is a straight-chained, branched or cyclic alkyl group having 1 - 4 carbon atoms; and the wavy line represents an E- or Z-type bond) with a compound of the following formula (III):

$R^{4'}$ - X (III)

(where $R^{4'}$ is a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group; and X is a leaving group) in the presence of a base.

**7.** A process for producing a compound of the formula (I) recited in claim 1 or a salt thereof by reacting an oxirane derivative of the following formula (IV):

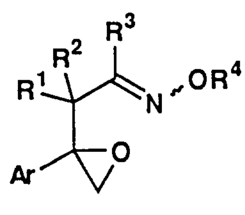

(IV)

(where Ar is a phenyl group substituted by one or two halogen atoms; $R^1$ and $R^2$ are each a methyl group or, when taken together with the adjacent carbon atom, represent a cyclopropylidene group; $R^3$ is a straight-chained, branched or cyclic alkyl group having 1 - 4 carbon atoms; $R^4$ is a hydrogen atom, a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group; and the wavy line represents an E- or Z-type bond) with a triazole derivative of the following formula (V):

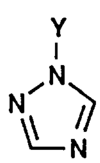

(V)

(where Y is a hydrogen atom or a trialkylsilyl group) in the presence of a base and optionally hydrolyzing the reaction product.

**8.** A process for producing a compound of the formula (I) recited in claim 1 or a salt thereof by reacting a ketone derivative of the following formula (VI):

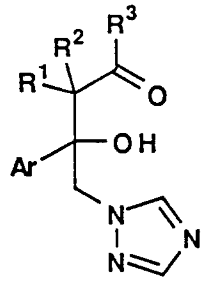

(VI)

(where Ar is a phenyl group substituted by one or two halogen atoms; $R^1$ and $R^2$ are each a methyl group or, when taken together with the adjacent carbon atom, represent a cyclopropylidene group; and $R^3$ is a straight-chained, branched or cyclic alkyl group having 1 - 4 carbon atoms) with a hydroxylamine derivative of the following formula (VII):

$R^4$ - $ONH_2$ (VII)

(where $R^4$ is a hydrogen atom, a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group).

**9.** An antimycotic containing at least one of the compounds represented by the following formula (I):

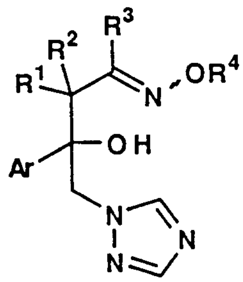

(I)

(where Ar is a phenyl group substituted by one or two halogen atoms; $R^1$ and $R^2$ are each a methyl group or, when taken together with the adjacent carbon atom, represent a cyclopropylidene group; $R^3$ is a straight-chained, branched or cyclic alkyl group having 1 - 4 carbon atoms; $R^4$ is a hydrogen atom, a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group; and the wavy line represents an E- or Z-type bond) or salts thereof as an active ingredient.

**10.** An antimycotic according to claim 9 wherein $R^3$ is an n-propyl or an isopropyl group.

**11.** An antimycotic according to claim 10 wherein Ar is a 2,4-difluorophenyl group and $R^1$ and $R^2$ from a cyclopropylidene group as taken together with the adjacent carbon atom.

**12.** An antimycotic according to claim 11 wherein $R^4$ is a methyl group, a methoxymethyl group or a 1H-(1,2,4-triazol-1-yl)methyl group.

**13.** A compound represented by the following formula (IV):

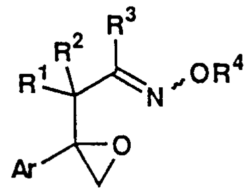

(IV)

(where Ar is a phenyl group substituted by one or two halogen atoms; $R^1$ and $R^2$ are each a methyl group or, when taken together with the adjacent carbon atom, represent a cyclopropylidene group; $R^3$ is a straight-chained, branched or cyclic alkyl group having 1 - 4 carbon atoms; $R^4$ is a hydrogen atom, a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group; and the wavy line represents an E- or Z-type bond) or a salt thereof.

**14.** A compound represented by the following formula (VIII):

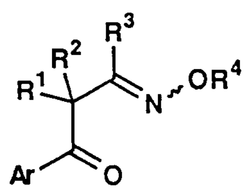

(VIII)

(where Ar is a phenyl group substituted by one or two halogen atoms; $R^1$ and $R^2$ are each a methyl group or, when taken together with the adjacent carbon atom, represent a cyclopropylidene group; $R^3$ is a straight-chained, branched or cyclic alkyl group having 1 - 4 carbon atoms; $R^4$ is a hydrogen atom, a dimethoxymethyl group, a dimethoxyethyl group or a straight-chained or branched alkyl group having 1 - 4 carbon atoms that may have at least one substituent selected from the group consisting of a 5- or 6-membered monocyclic hetero group having 1, 2 or 3 nitrogen atoms that may be substituted by 1 or 2 methyl groups, a methylthio group, a methoxy group, an ethoxy group, a methoxyethoxy group, a cyano group, a halogen atom, an ethynyl group or an epoxy group; and the wavy line represents an E- or Z-type bond) or a salt thereof.

# FIG. 1

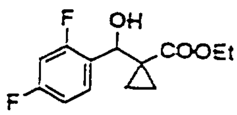
REF. EX. 1

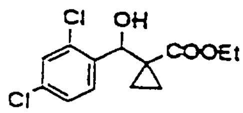
REF. EX. 2

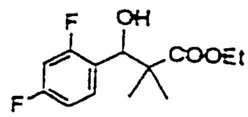
REF. EX. 3

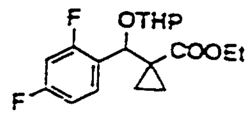
REF. EX. 4

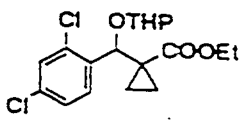
REF. EX. 5

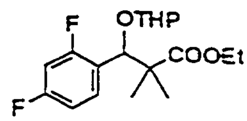
REF. EX. 6

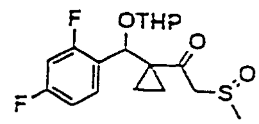
REF. EX. 7

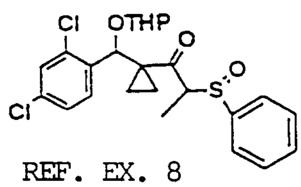
REF. EX. 8

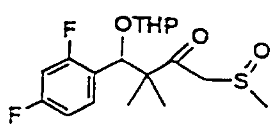
REF. EX. 9

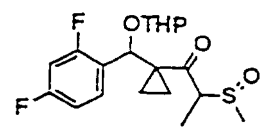
REF. EX. 10

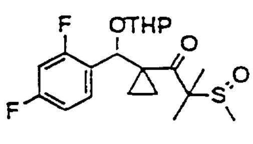
REF. EX. 11

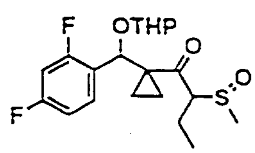
REF. EX. 12

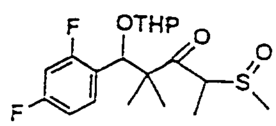
REF. EX. 13

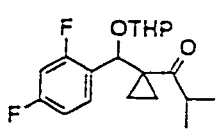
REF. EX. 14

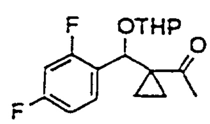
REF. EX. 15

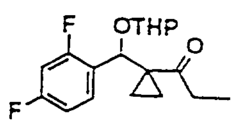
REF. EX. 16

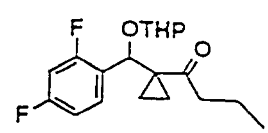
REF. EX. 17

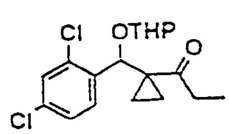
REF. EX. 18

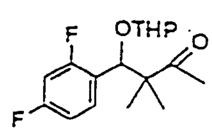
REF. EX. 19

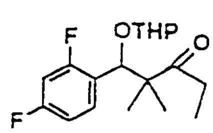
REF. EX. 20

FIG. 2

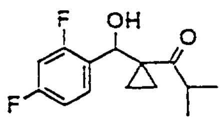

REF. EX. 21

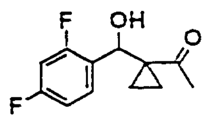

REF. EX. 22

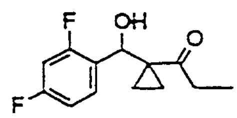

REF. EX. 23

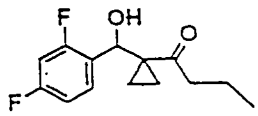

REF. EX. 24

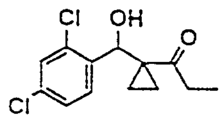

REF. EX. 25

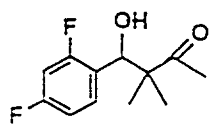

REF. EX. 26

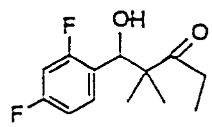

REF. EX. 27

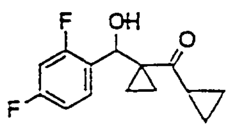

REF. EX. 28

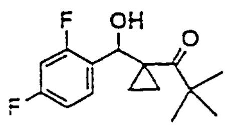

REF. EX. 29

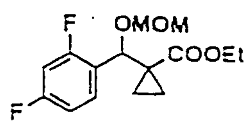

REF. EX. 30-a

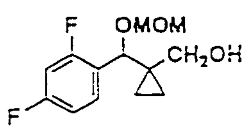

REF. EX. 30-b

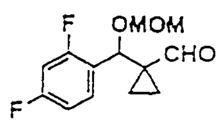

REF. EX. 30-c

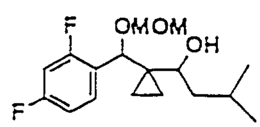

REF. EX. 30-d

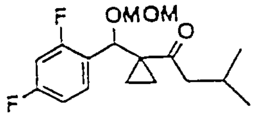

REF. EX. 30-e

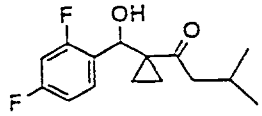

REF. EX. 30-f

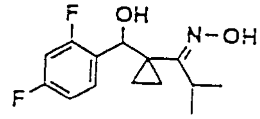

REF. EX. 31

# FIG. 3

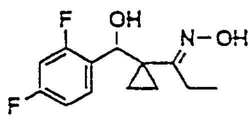

REF. EX. 32

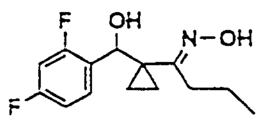

REF. EX.33

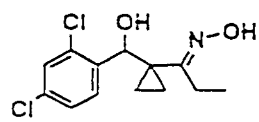

REF. EX. 34

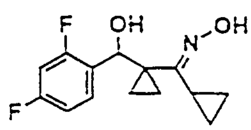

REF. EX. 35

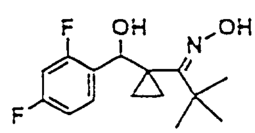

REF. EX. 36

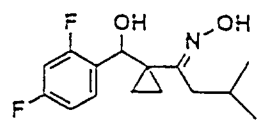

REF. EX. 37

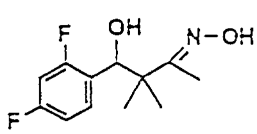

REF. EX. 38

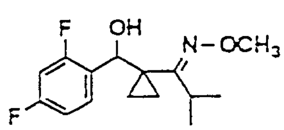

REF. EX. 39

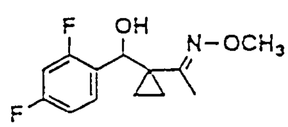

REF. EX. 40

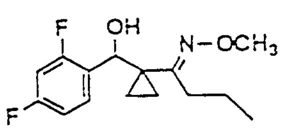

REF. EX. 41

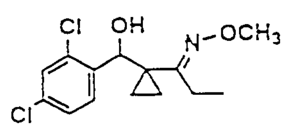

REF. EX. 42

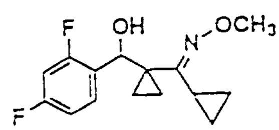

REF. EX. 43

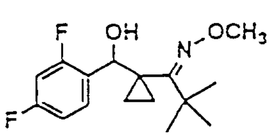

REF. EX. 44

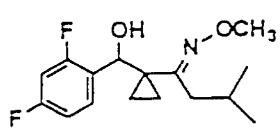

REF. EX. 45

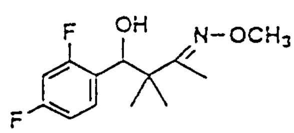

REF. EX. 46

FIG. 4

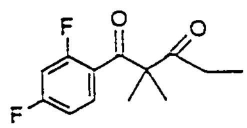

REF. EX. 47

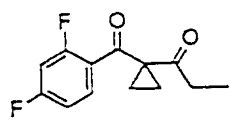

REF. EX. 48

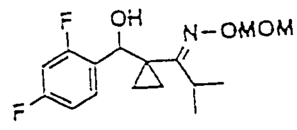

REF. EX. 49

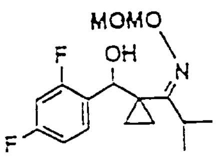

REF. EX. 50

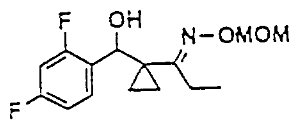

REF. EX. 51

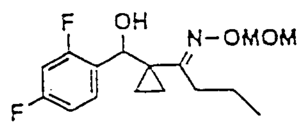

REF. EX. 52

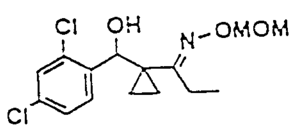

REF. EX. 53

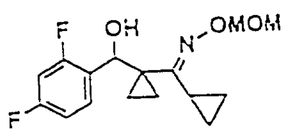

REF. EX. 54

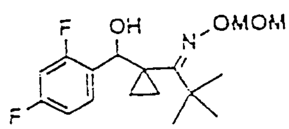

REF. EX. 55

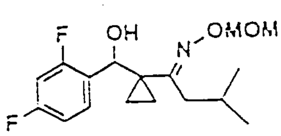

REF. EX. 56

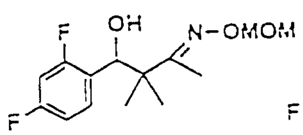

REF. EX. 57

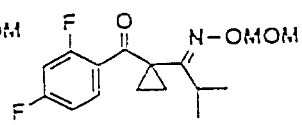

REF. EX. 58

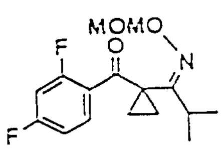

REF. EX. 59

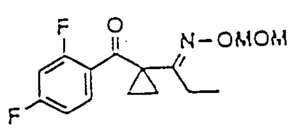

REF. EX. 60

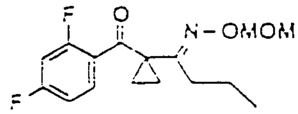

REF. EX. 61

# FIG. 5

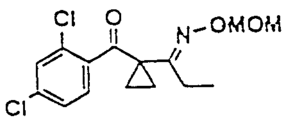

REF. EX. 62

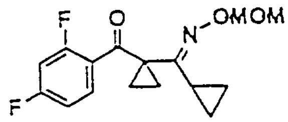

REF. EX. 63

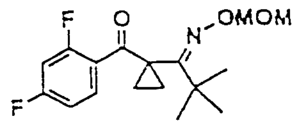

REF. EX. 64

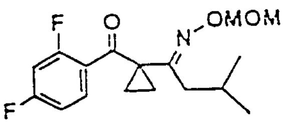

REF. EX. 65

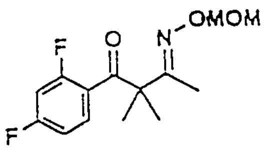

REF. EX. 66

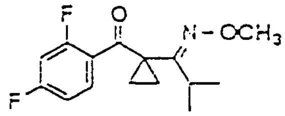

REF. EX. 67

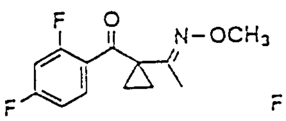

REF. EX. 68

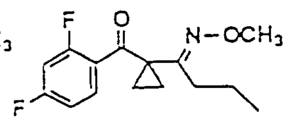

REF. EX. 69

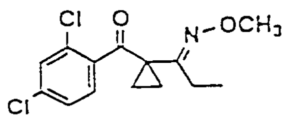

REF. EX. 70

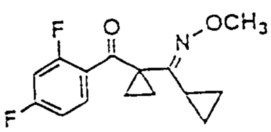

REF. EX. 71

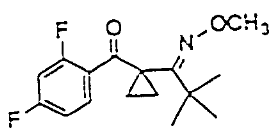

REF. EX. 72

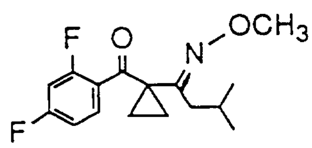

REF. EX. 73

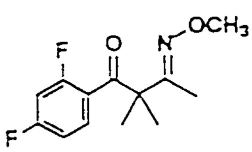

REF. EX. 74

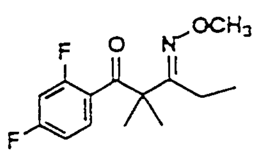

REF. EX. 75

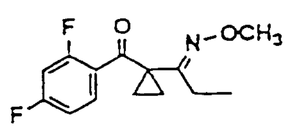

REF. EX. 76

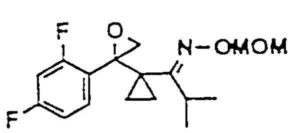

REF. EX. 77

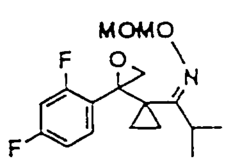

REF. EX. 78

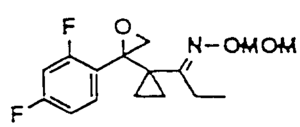

REF. EX. 79

# FIG. 6

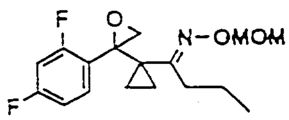
REF. EX. 80

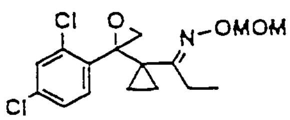
REF. EX. 81

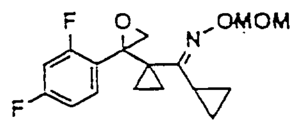
REF. EX. 82

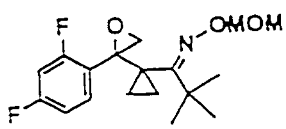
REF. EX. 83

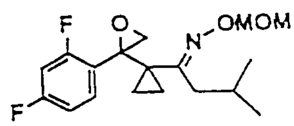
REF. EX. 84

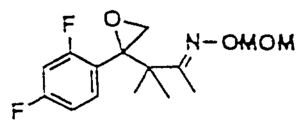
REF. EX. 85

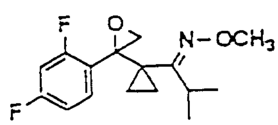
REF. EX. 86

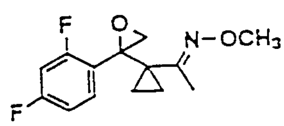
REF. EX. 87

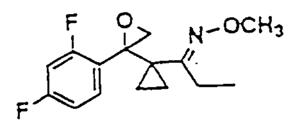
REF. EX. 88

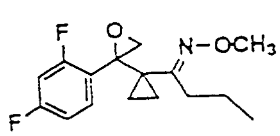
REF. EX. 89

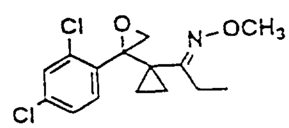
REF. EX. 90

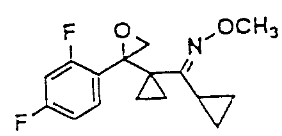
REF. EX. 91

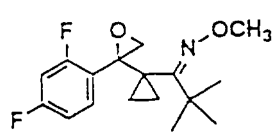
REF. EX. 92

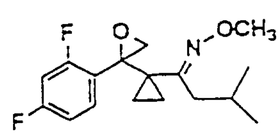
REF. EX. 93

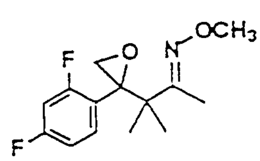
REF. EX. 94

# FIG. 7

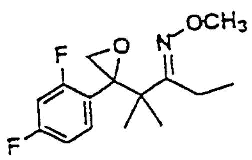

REF. EX. 95

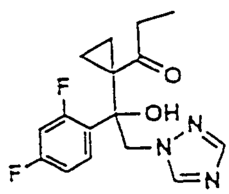

REF. EX. 96

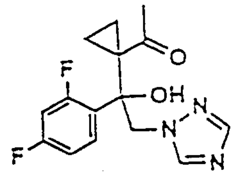

REF. EX. 97

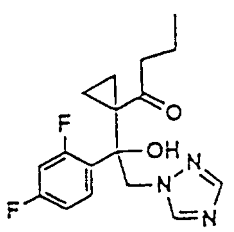

REF. EX. 98

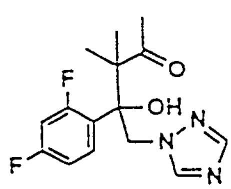

REF. EX. 99

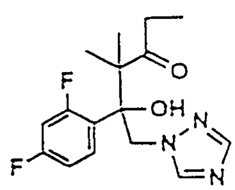

REF. EX. 100

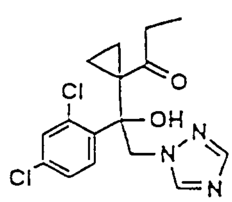

REF. EX. 101

# FIG. 8

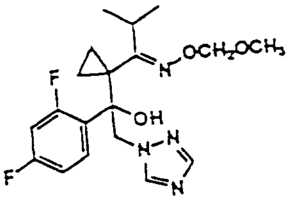

EXAMPLE 1

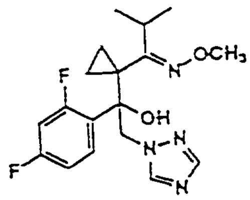

EXAMPLE 2

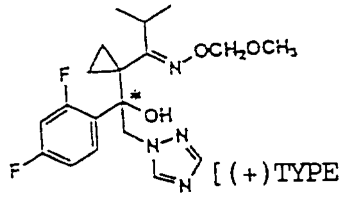

[(+)TYPE]

EXAMPLE 3

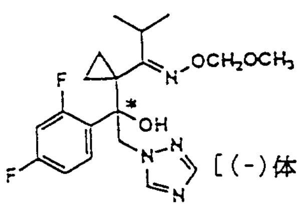

[(-)体]

EXAMPLE 4

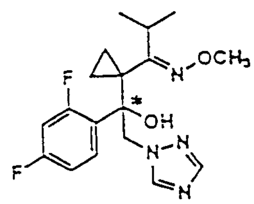

[(+)TYPE]

EXAMPLE 5

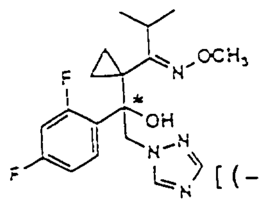

[(-)TYPE]

EXAMPLE 6

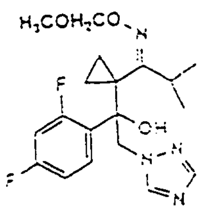

EXAMPLE 7

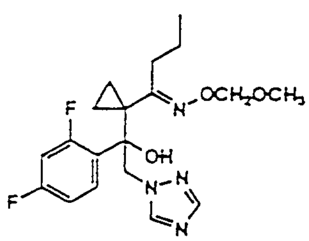

EXAMPLE 8

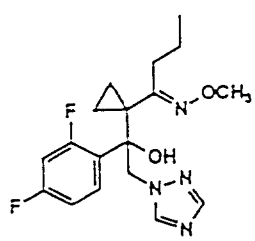

EXAMPLE 9

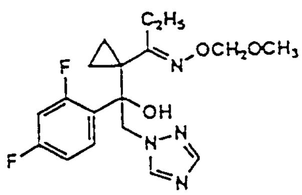

EXAMPLE 10

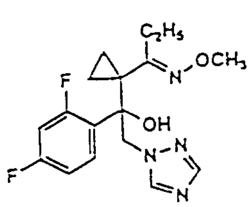

EXAMPLE 11

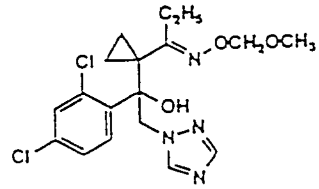

EXAMPLE 12

# FIG. 9

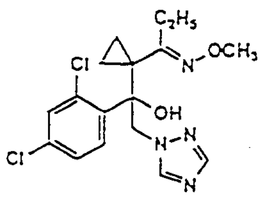

EXAMPLE 13

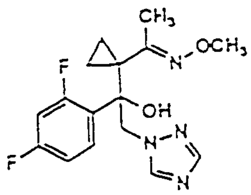

EXAMPLE 14

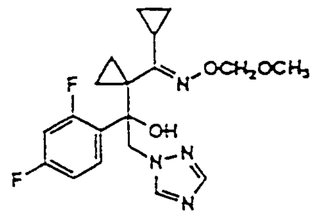

EXAMPLE 15

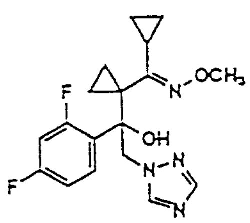

EXAMPLE 16

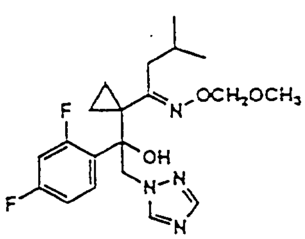

EXAMPLE 17

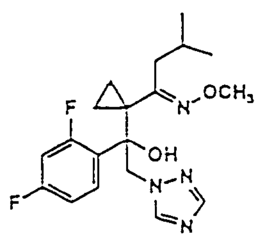

EXAMPLE 18

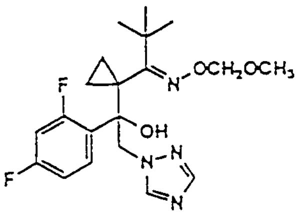

EXAMPLE 19

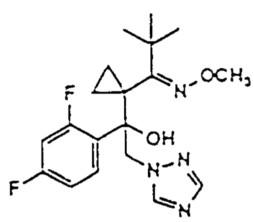

EXAMPLE 20

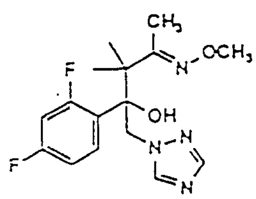

EXAMPLE 21

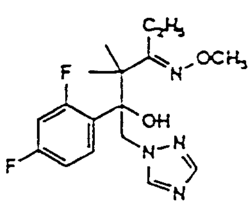

EXAMPLE 22

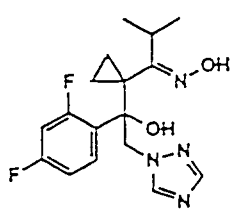

EXAMPLE 23

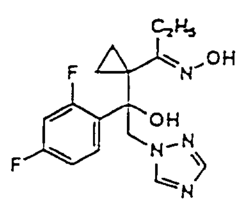

EXAMPLE 24

# FIG. 10

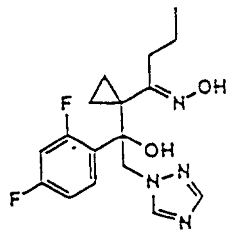

EXAMPLE 25

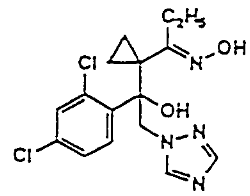

EXAMPLE 26

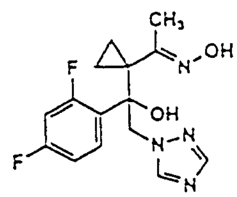

EXAMPLE 27

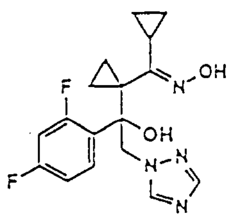

EXAMPLE 28

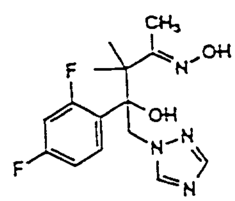

EXAMPLE 29

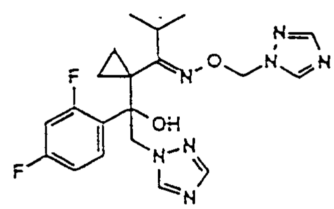

EXAMPLE 30

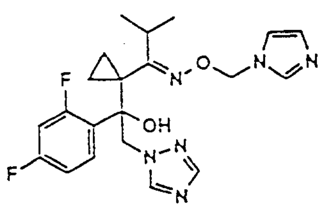

EXAMPLE 31

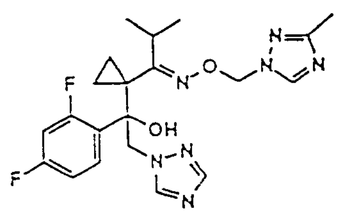

EXAMPLE 32

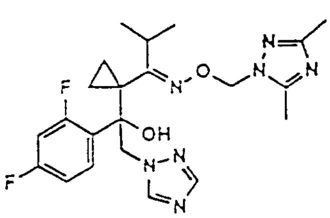

EXAMPLE 33

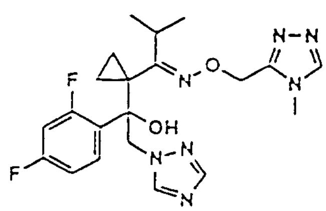

EXAMPLE 34

FIG. 11

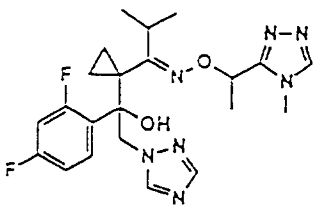

EXAMPLE 35

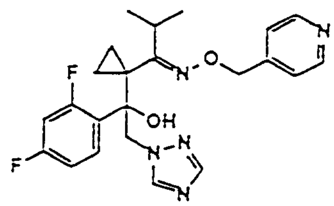

EXAMPLE 36

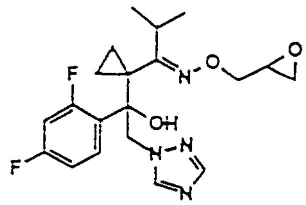

EXAMPLE 37

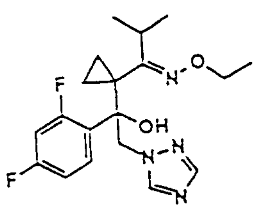

EXAMPLE 38

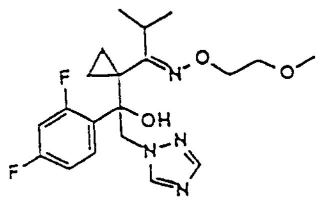

EXAMPLE 39

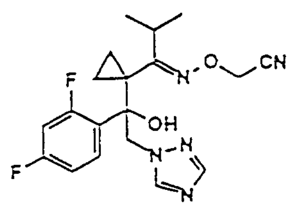

EXAMPLE 40

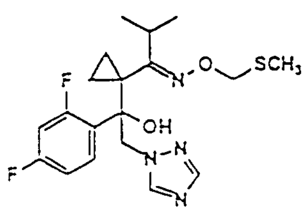

EXAMPLE 41

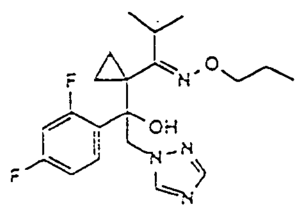

EXAMPLE 42

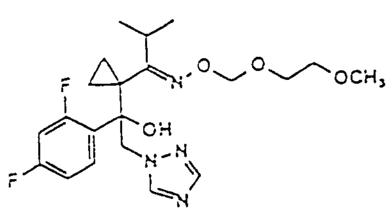

EXAMPLE 43

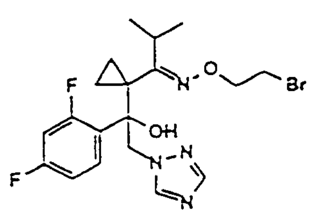

EXAMPLE 44

# FIG. 12

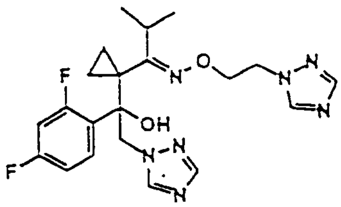

EXAMPLE 45

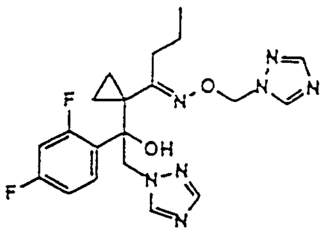

EXAMPLE 46

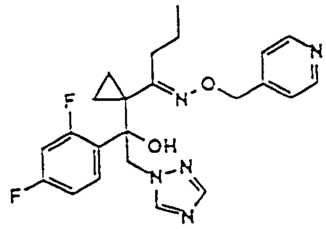

EXAMPLE 47

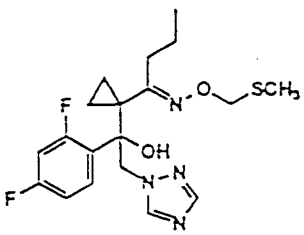

EXAMPLE 48

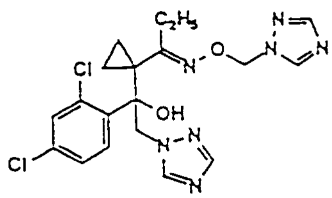

EXAMPLE 49

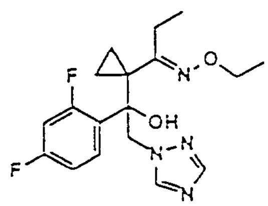

EXAMPLE 50

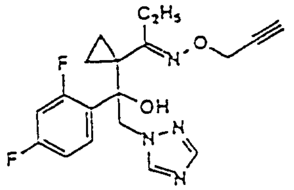

EXAMPLE 51

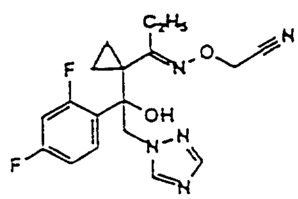

EXAMPLE 52

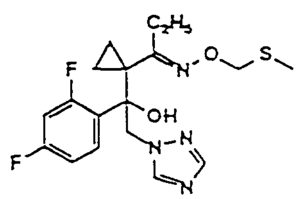

EXAMPLE 53

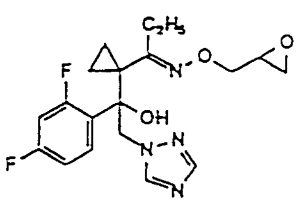

EXAMPLE 54

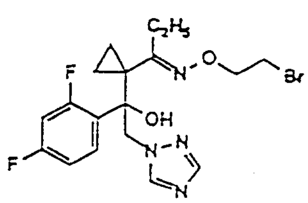

EXAMPLE 55

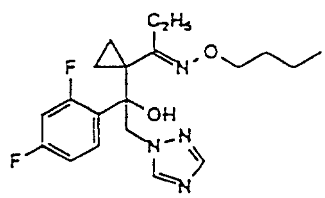

EXAMPLE 56

FIG. 13

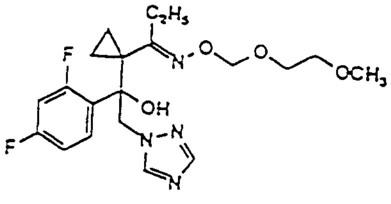

EXAMPLE 57

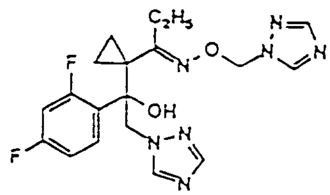

EXAMPLE 58

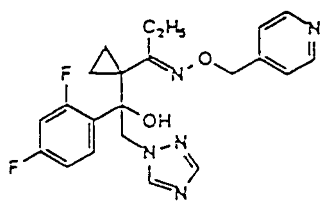

EXAMPLE 59

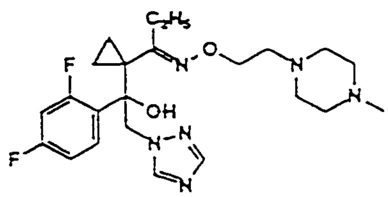

EXAMPLE 60

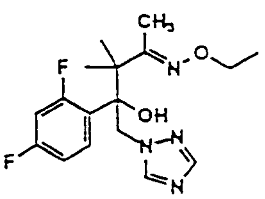

EXAMPLE 61

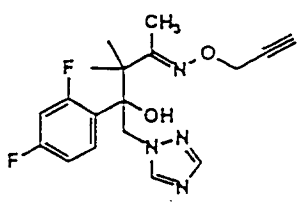

EXAMPLE 62

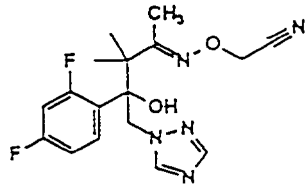

EXAMPLE 63

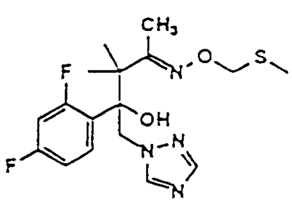

EXAMPLE 64

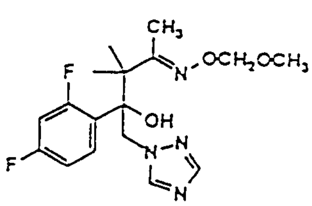

EXAMPLE 65

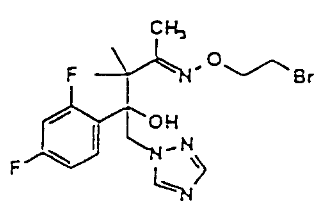

EXAMPLE 66

FIG. 14

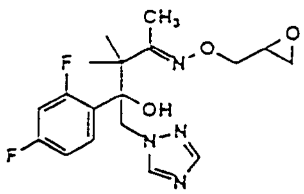

EXAMPLE 67

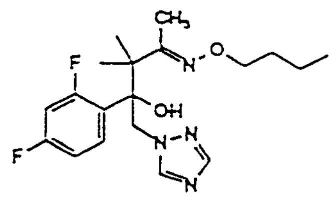

EXAMPLE 68

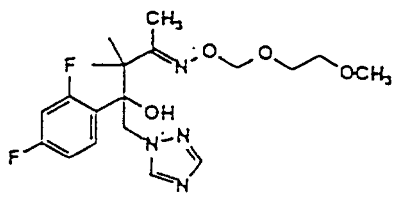

EXAMPLE 69

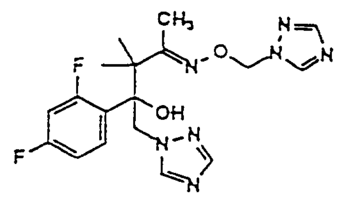

EXAMPLE 70

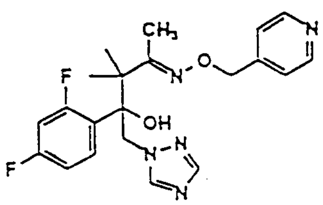

EXAMPLE 71

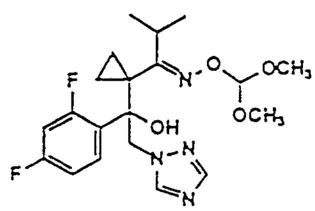

EXAMPLE 72

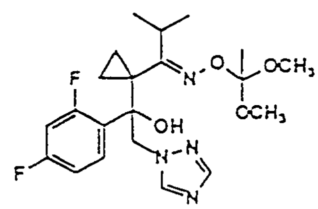

EXAMPLE 73

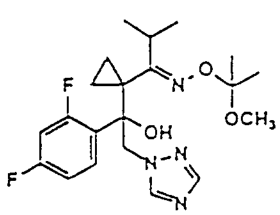

EXAMPLE 74

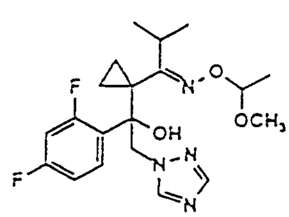

EXAMPLE 75

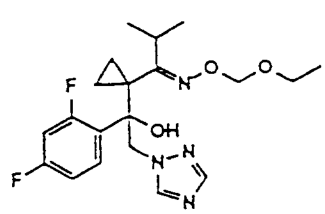

EXAMPLE 76

European Patent Office

# EUROPEAN SEARCH REPORT

Application Number
EP 95 10 1379

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | CHEMICAL ABSTRACTS, vol. 113, no. 3, 16 July 1990 Columbus, Ohio, US; abstract no. 23928x, IMAIZUMI,H. ET AL. 'Triazolylcyclopropylethanols as medical fungicides and their preparation' page 665; * abstract * | 1-14 | C07D249/08 A01N43/653 |
| X | & JP-A-02 009 684 (TOYAMA CHEMICAL CO., LTD.) 12 January 1990 * the whole document * --- | 1-14 | |
| X | EP-A-0 301 349 (BAYER AG) 1 February 1989 * the whole document * --- | 1-14 | |
| Y | EP-A-0 315 861 (BAYER AG) 17 May 1989 * see page 9, formula (I'''') * --- | 1-14 | |
| Y | EP-A-0 141 204 (BAYER AG) 15 May 1985 * the whole document * --- | 1-14 | TECHNICAL FIELDS SEARCHED (Int.Cl.6) |
| X | EP-A-0 176 998 (BAYER AG) 9 April 1986 * the whole document * --- | 1-14 | C07D A01N |
| X | DE-A-37 24 911 (BAYER AG) 9 February 1989 * the whole document * --- | 1-14 | |
| Y | WO-A-92 17474 (BAYER AG ) 15 October 1992 * the whole document * --- | 1-14 | |
| | -/-- | | |

The present search report has been drawn up for all claims

| Place of search | Date of completion of the search | Examiner |
|---|---|---|
| MUNICH | 13 June 1995 | Stellmach, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 95 10 1379

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | CHEMICAL ABSTRACTS, vol. 120, no. 1, 3 January 1994 Columbus, Ohio, US; abstract no. 8596r, IMAIZUMI,H. ET AL. 'Preparation of triazole derivitives as medical fungicides' page 8591; * abstract * | 1-14 | |
| X | & JP-A-05 163 269 (TOYAMAM CHEMICAL CO., LTD.) 13 December 1991 * the whole document * --- | 1-14 | |
| Y | PESTIC.SCI., vol. 15, 1984 pages 188-198, VAN DEN BOSSCHE,H. ET AL. 'Molecular Basis for the Antimycotic and Antibacterial activity of N-substituted Imidazoles and Triazoles: the inhibition of Isoprenoid Biosynthesis' * the whole document * --- | 1-14 | TECHNICAL FIELDS SEARCHED (Int.Cl.6) |
| Y | EUROP.J.MED.CHEM., vol. 24, 1989 PARIS, pages 137-143, OGATA,M. ET AL. 'Synthesis and oral anti-fungal activity of novel 1,3-bis-(azolyl)-2-arylpropan-2-ols ' * see page 137, introduction , first sentence * ----- | 1-14 | |

The present search report has been drawn up for all claims

| Place of search | Date of completion of the search | Examiner |
|---|---|---|
| MUNICH | 13 June 1995 | Stellmach, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document